(19) Europäisches Patentamt
European Patent Office
Office européen des brevets

(11) Veröffentlichungsnummer: **0 398 072 B1**

(12) **EUROPÄISCHE PATENTSCHRIFT**

(45) Veröffentlichungstag der Patentschrift: **01.02.95**

(51) Int. Cl.$^6$: **C07C 271/06**, C07D 213/40, A01N 47/10

(21) Anmeldenummer: **90108285.9**

(22) Anmeldetag: **01.05.90**

Die Akte enthält technische Angaben, die nach dem Eingang der Anmeldung eingereicht wurden und die nicht in dieser Patentschrift enthalten sind.

(54) **Aminosäureamid-Derivate.**

(30) Priorität: **13.05.89 DE 3915755**

(43) Veröffentlichungstag der Anmeldung:
**22.11.90 Patentblatt 90/47**

(45) Bekanntmachung des Hinweises auf die Patenterteilung:
**01.02.95 Patentblatt 95/05**

(84) Benannte Vertragsstaaten:
**BE CH DE ES FR GB GR IT LI NL**

(56) Entgegenhaltungen:
**EP-A- 0 236 874**
**CH-A- 536 819**
**FR-A- 2 513 485**

**DE-A 2625539**

(73) Patentinhaber: **BAYER AG**

**D-51368 Leverkusen (DE)**

(72) Erfinder: **Wollweber, Detlef, Dr.**
**Paul-Ehrlich-Strasse 17**
**D-5600 Wuppertal 1 (DE)**
Erfinder: **Seitz, Thomas, Dr.**
**Mozartstrasse 32**
**D-4019 Monheim (DE)**
Erfinder: **Brandes, Wilhelm, Dr.**
**Eichendorffstrasse 3**
**D-5653 Leichlingen 1 (DE)**

**EP 0 398 072 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung von teilweise bekannten substituierten Aminosäureamid-Derivaten in Schädlingsbekämpfungsmitteln sowie neue substituierte Aminosäureamid-Derivate und ein Verfahren zu deren Herstellung.

Die erfindungsgemäßen sowie die erfindungsgemäß zu verwendenden Substanzen besitzen eine ausgezeichnete Wirkung als Fungizide im Pflanzenschutz.

Bestimmte Aminosäureamide sind bereits bekannt, wie beispielsweise N-tert.-Butoxycarbonyl-L-leucylbenzylamid (EP-A-236 874).

Eine Verwendung dieser Verbindungen in Schädlingsbekämpfungsmitteln wird jedoch nicht beschrieben.

Gegenstand der vorliegenden Anmeldung ist somit die Verwendung von substituierten Aminosäureamid-Derivaten der allgemeinen Formel (I)

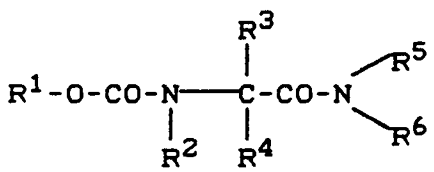

(I)

in welcher

| | |
|---|---|
| $R^1$ | für Alkyl, Alkenyl, Alkinyl, Halogenalkyl, Halogenalkenyl, Halogenalkinyl, Cycloalkyl oder Cycloalkenyl steht, |
| $R^2$, $R^3$, $R^4$ und $R^5$ | gleich oder verschieden sind und für Wasserstoff, Cycloalkyl oder Alkyl stehen oder |
| $R^3$ und $R^4$ | zusammen mit dem Kohlenstoffatom, an das sie gebunden sind, einen Cycloalkylring bilden und |
| $R^6$ | für Wasserstoff, Alkyl, Alkenyl, Alkinyl, Cyanoalkyl, unsubstituiertes oder substituiertes Phenylalkyl, unsubstituiertes oder substituiertes Phenyl, unsubstituiertes oder substituiertes Cycloalkyl oder für unsubstituiertes oder substituiertes Heterocyclyl oder Heterocyclylalkyl steht, |

oder

| | |
|---|---|
| $R^5$ und $R^6$ | gemeinsam mit dem Stickstoff-Atom, an das sie gebunden sind, für einen Heterocyclyl-Rest stehen, der weitere Heteroatome enthalten kann, |

zur Bekämpfung von phytopathogenen Pilzen.

Die Verbindungen der Formel (I) und der folgenden Verbindungen (Ia) können außerdem ein oder mehrere Chiralitätszentren enthalten und können somit in verschiedenen Enantiomeren- und Diastereomerengemischen vorliegen, die gegebenenfalls in üblicher Art und Weise getrennt werden können. Die Verwendung sowohl der reinen Enantiomeren und Diastereomeren, als auch die der Gemische werden ebenfalls erfindungsgemäß beansprucht.

Im folgenden wird der Einfachheit halber stets von der Verwendung von Verbindungen der Formel (I) bzw. (Ia) gesprochen, obwohl sowohl die reinen Verbindungen, als auch die Gemische mit unterschiedlichen Anteilen an isomeren, enantiomeren und diastereomeren Verbindungen gemeint sind.

Die erfindungsgemäß zu verwendenden substituierten Aminosäureamid-Derivate sind durch die Formel (I) allgemein definiert.

Vorzugsweise bedeutet in den allgemeinen Formeln im folgenden, falls nicht anders definiert:

Alkyl, einzeln oder in zusammengesetzten Resten

- geradkettiges oder verzweigtes Alkyl mit 1 bis 6, insbesondere 1 bis 4 Kohlenstoffatomen. Beispielhaft und vorzugsweise seien Methyl, Ethyl, n.- und i.-Propyl, n-, i-, s- und t-Butyl, genannt.

Alkenyl

- geradkettiges oder verzweigtes Alkenyl mit 2 bis 6, insbesondere 2 bis 4 Kohlenstoffatomen. Beispielhaft und vorzugsweise seien gegebenenfalls substituiertes Ethenyl, Propenyl-(1), Propenyl-(2) und Butenyl-(3) genannt.

Alkinyl

- geradkettiges oder verzweigtes Alkinyl mit 2 bis 6, insbesondere 2 bis 4 Kohlenstoffatomen. Beispielhaft und vorzugsweise seien gegebenenfalls substituiertes Ethinyl, Propinyl-(1), Propinyl-(2) und Butinyl-(3) genannte.

Cycloalkyl

- substituiertes oder unsubstituiertes Cycloalkyl mit 3 bis 7, insbesondere 3 bis 6 Kohlenstoffatomen Beispielhaft und vorzugsweise seien Cyclopropyl, Cyclopentyl und Cyclohexyl genannt.

Cycloalkenyl

- substituiertes oder unsubstituiertes Cycloalkenyl mit 3 bis 7, insbesondere 3 bis 6 Kohlenstoffatomen und 1 oder 2 Doppelbindungen.

Heterocyclyl

- substituierte oder unsubstituierte, heteroparaffinische, heteroolefinische und heteroaromatische Ringe mit 2 bis 6, insbesondere 4 oder 5 Kohlenstoffatomen und einem oder zwei, gegebenenfalls weiteren Heteroatomen, wie Stickstoff und Sauerstoff.

Halogenalkyl

- geradkettiges oder verzweigtes Halogenalkyl mit 1 bis 6, insbesondere 1 bis 4 Kohlenstoffatomen und 1 bis 9, insbesondere 1 bis 5 und vor allem 1 bis 3, gleichen oder verschiedenen Halogenatomen wie Fluor, Chlor, Brom, Iod, insbesondere Fluor und Chlor. Beispielhaft und vorzugsweise seien Fluormethyl, Fluorethyl, Fluorpropyl, Chlorpropyl, Fluorbutyl, Chlorbutyl, Difluormethyl, Difluorethyl, Dichlorethyl, Difluorpropyl, Dichlorpropyl, Difluorbutyl, Dichlorbutyl, Trifluormethyl, Trichlormethyl, Trifluorethyl, Trichlorethyl, Trifluorpropyl, Trichlorpropyl, Trifluorbutyl und Trichlorbutyl genannt.

Halogenalkenyl und Halogenalkinyl

- geradkettiges oder verzweigtes gegebenenfalls substituiertes Halogenalkenyl bzw. Halogenalkinyl mit 2 bis 6, insbesondere 2 bis 4 Kohlenstoffatomen und 1 bis 9, insbesondere 1 bis 5 und vor allem 1 bis 3, gleichen oder verschiedenen Halogenatomen wie Fluor, Chlor, Brom, Iod, insbesondere Fluor und Chlor. Beispielhaft und vorzugsweise seien Fluorallyl, Chlorallyl, Fluorbutenyl, Chlorbutenyl, Fluorpropargyl, Chlorpropargyl, Fluorbutinyl und Chlorbutinyl genannt.

Die gegebenenfalls substituierten Reste der allgemeinen Formeln können einen oder mehrere, vorzugsweise 1 bis 3, insbesondere 1 oder 2 gleiche oder verschiedene Substituenten tragen. Als Substituenten seien beispielhaft und vorzugsweise aufgeführt:
Alkyl mit vorzugsweise 1 bis 4, insbesondere 1 oder 2 Kohlenstoffatomen, wie Methyl, Ethyl, n- und i.-Propyl und n.-, i.- und t.-Butyl; Alkoxy mit vorzugsweise 1 bis 4, insbesondere 1 oder 2 Kohlenstoffatomen, wie Methoxy, Ethoxy, n.- und i.-Propyloxy und n.-, i.-, sec.- und t.-Butyloxy; Alkylthio mit vorzugsweise 1 bis 4, insbesondere 1 oder 2 Kohlenstoffatomen, wie Methylthio, Ethylthio, n.- und i.-Propylthio und n.-, i.-, sec.- und t.-Butylthio; Halogenalkyl, Halogenalkoxy und Halogenalkylthio mit vorzugsweise 1 bis 4, insbesondere 1 oder 2 Kohlenstoffatomen und vorzugsweise 1 bis 9, insbesondere 1 bis 5 Halogenatomen, wobei die Halogenatome gleich oder verschieden sind und als Halogenatome, vorzugsweise Fluor, Chlor oder Brom, insbesondere Fluor stehen, wie Trifluormethyl, Trifluormethoxy und Trifluormethylthio; Hydroxy; Halogen, vorzugsweise Fluor, Chlor, Brom und Jod, insbesondere Fluor, Chlor und Brom; Cyano; Nitro; Amino; Alkylamino und Dialkylamino mit vorzugsweise 1 bis 4, insbesondere 1 oder 2 Kohlenstoffatomen je Alkylgruppe, wie Methylamino, Ethylamino, Dimethylamino und Diethylamino; Carboxyl.

Bevorzugt werden die Verbindungen der Formel (I) verwendet, in welcher

$R^1$ für geradkettiges oder verzweigtes Alkyl mit 1 bis 6 Kohlenstoffatomen, geradkettiges oder verzweigtes Alkenyl oder Alkinyl mit 2 bis 6 Kohlenstoffatomen, geradkettiges oder verzweigtes Halogenalkyl mit 1 bis 6 Kohlenstoffatomen und 1 bis 9 gleichen oder verschiedenen Halogenatomen, geradkettiges oder verzweigtes Halogenalkenyl oder Halogenalkinyl mit 2 bis 6 Kohlenstoffatomen und 1 bis 9 gleichen oder verschiedenen Halogenatomen oder substituiertes oder unsubstituiertes Cycloalkyl oder Cycloalkenyl mit 3 bis 7 Kohlenstoffatomen steht und

$R^2$, $R^3$, $R^4$ und $R^5$ gleich oder verschieden sind und für Wasserstoff, unsubstituiertes oder substituiertes Cycloalkyl mit 3 bis 7 Kohlenstoffatomen oder geradkettiges oder verzweigtes Alkyl mit 1 bis 6 Kohlenstoffatomen stehen oder

$R^3$ und $R^4$ zusammen mit dem Kohlenstoffatom, an das sie gebunden sind, einen Cycloalkylring mit 3 bis 7 Kohlenstoffatomen bilden und

$R^6$ für Wasserstoff, geradkettiges oder verzweigtes Alkyl mit 1 bis 6 Kohlenstoffatomen, für Alkenyl oder Alkinyl mit jeweils 2 bis 6 Kohlenstoffatomen, für Cyanalkyl mit 1 bis 4 Kohlenstoffatomen im Alkylteil, unsubstituiertes oder im Phenylteil einfach bis dreifach, gleich oder verschieden substituiertes Phenylalkyl mit 1 bis 4 Kohlenstoffatomen im Alkylteil oder unsubstituiertes oder einfach bis dreifach, gleich oder verschieden substituiertes Phenyl steht, wobei als Substituenten jeweils in Frage kommen: Halogen; Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 gleichen oder verschiedenen Halogenatomen; Hydroxy; Cyano, Nitro, Amino, Alkylamino und Dialkylamino mit jeweils 1 bis 4 Kohlenstoffatomen und Carboxyl; für unsubstituiertes oder gleich oder verschieden substituiertes Cycloalkyl mit 3 bis 7 Kohlenstoffatomen, wobei als Substituenten Alkyl und Alkoxy mit jeweils 1 bis 4 Kohlenstoffatomen in Frage kommen, oder für unsubstituiertes oder substituiertes Heterocyclyl oder Heterocyclylalkyl mit 2 bis 6 Kohlenstoffatomen im Heterocyclylteil und einem oder zwei Heteroatomen und gegebenenfalls 1 bis 4 Kohlenstoffatomen im Alkylteil steht, wobei als Substituenten die oben genannten Phenylsubstituenten in Frage kommen,

oder

$R^5$ und $R^6$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann.

Besonders bevorzugt werden diejenigen Verbindungen der Formel (I) verwendet, in denen

$R^1$ für geradkettiges oder verzweigtes Alkyl mit 1 bis 4 Kohlenstoffatomen, geradkettiges oder verzweigtes Alkenyl oder Alkinyl mit 2 bis 4 Kohlenstoffatomen, geradkettiges oder verzweigtes Halogenalkyl mit 1 bis 4 Kohlenstoffatomen und 1 bis 5 gleichen oder verschiedenen Halogenatomen, geradkettiges oder verzweigtes Halogenalkenyl oder Halogenalkinyl mit 2 bis 4 Kohlenstoffatomen und 1 bis 5 gleichen oder verschiedenen Halogenatomen oder unsubstituiertes oder substituiertes Cycloalkyl oder Cycloalkenyl mit 3 bis 6 Kohlenstoffatomen steht und

$R^2$, $R^3$, $R^4$ und $R^5$ gleich oder verschieden sind und für Wasserstoff unsubstituiertes oder substituiertes Cycloalkyl mit 3 bis 6 Kohlenstoffatomen oder geradkettiges oder verzweigtes Alkyl mit 1 bis 5 Kohlenstoffatomen stehen oder

$R^3$ und $R^4$ zusammen mit dem Kohlenstoffatom, an das sie gebunden sind, einen Cycloalkylring mit 3 bis 6 Kohlenstoffatomen bilden und

$R^6$ für Wasserstoff, Methyl, Ethyl, Allyl, Propargyl, für Cyanoalkyl mit 1 oder 2 Kohlenstoffatomen im Alkylteil; ferner für unsubstituiertes oder im Phenylteil einfach oder zweifach, gleich oder verschieden substituiertes Phenylalkyl mit 1 bis 4 Kohlenstoffatomen im Alkylteil, für unsubstituiertes oder einfach oder zweifach, gleich oder verschieden substituiertes Phenyl steht, wobei als Substituenten jeweils in Frage kommen: Fluor, Chlor und Brom, Methyl, Ethyl, n- und i-Propyl, n-, i-, s- und t-Butyl, Methoxy, Ethoxy, n- und i-Propyloxy, n-, i-, s- und t-Butyloxy, Methylthio, Ethylthio, n- und i-Propylthio, n-, i-, s- und t-Butylthio, Trifluormethyl, Trifluormethoxy, Trifluormethylthio, Hydroxy, Nitro, Cyano, Amino, Methylamino, Ethylamino, Dimethylamino, Diethylamino und Carboxy; für jeweils unsubstituiertes oder einfach oder zweifach, gleich oder verschieden substituiertes Cycloalkyl mit 3

bis 6 Kohlenstoffatomen, wobei als Substituenten Methyl, Ethyl, n- und i-Propyl, n-, i-, s- und t-Butyl, Methoxy, Ethoxy, n- und i-Propyloxy, n-, i-, s- und t-Butyloxy in Frage kommen, oder für unsubstituiertes oder einfach oder zweifach, gleich oder verschieden substituiertes Heterocyclyl oder Heterocyclylalkyl mit 4 oder 5 Kohlenstoffatomen im Heterocyclylteil und einem oder zwei Stickstoffatomen und gegebenenfalls 1 bis 4 Kohlenstoffatomen im Alkylteil steht, wobei als Substituenten die oben genannten Phenylsubstituenten in Frage kommen; oder

$R^5$ und $R^6$ gemeinsam mit dem Stickstoffatom, an das sie sie gebunden sind, für einen 5- oder 6-gliedrigen Heterocyclus stehen, der gegebenenfalls Sauerstoff oder Stickstoff als weitere Heteroatome enthalten kann.

Ganz besonders bevorzugt werden diejenigen Verbindungen der Formel (I) verwendet, in denen

$R^1$ für Methyl, Ethyl, n- und i-Propyl, n-, i-, s- und t-Butyl, Fluormethyl, Fluorethyl, Fluorpropyl, Chlorpropyl, Fluorbutyl, Chlorbutyl, Difluormethyl, Difluorpropyl, Dichlorpropyl, Difluorbutyl, Dichlorbutyl, Trifluormethyl, Trichlormethyl, Trifluorethyl, Trichlorethyl, Trifluorpropyl, Trichlorpropyl, Trifluorbutyl, Trichlorbutyl, Allyl, Butenyl, Propargyl, Butinyl, Fluor- oder Chlor-allyl, -butenyl, -propargyl, -butinyl, Cyclopropyl, Cyclopentyl, Cyclohexyl, Cyclopentenyl oder Cyclohexenyl steht und

$R^2$ für Wasserstoff oder Methyl steht und

$R^3$ und $R^4$ gleich oder verschieden sind und für Wasserstoff, Methyl, Ethyl, n- oder i-Propyl, n-, i-, s- oder t-Butyl, 3-Pentyl, Cyclopropyl, Cyclopentyl oder Cyclohexyl stehen oder

$R^3$ und $R^4$ zusammen mit dem Kohlenstoffatom, an das sie gebunden sind, einen Cyclopropyl-, Cyclopentyl- oder Cyclohexylring bilden und

$R^5$ für Wasserstoff oder Methyl steht und

$R^6$ für Wasserstoff, Methyl, Ethyl, Allyl, Propargyl, oder für Cyanalkyl mit 1 oder 2 Kohlenstoffatomen im Alkylteil, für unsubstituiertes oder im Phenylteil einfach oder zweifach gleich oder verschieden substituiertes Benzyl, 1-Phenethyl, 2-Phenethyl oder 1-Phenylpropyl, 2-Phenylpropyl, 3-Phenylpropyl, 1-Phenylisopropyl, 2-Phenylisopropyl oder 1-Phenyl-n-, -i- oder -s-butyl, für unsubstituiertes oder einfach oder zweifach, gleich oder verschieden substituiertes Phenyl steht, wobei als Substituenten jeweils in Frage kommen: Fluor, Chlor, Brom, Methyl, Ethyl, n- und i-Propyl, n-, i-, s- und t-Butyl, Methoxy, Ethoxy, n- und i-Propyloxy, n-, i-, s- und t-Butyloxy, Methylthio, Ethylthio, n- und i-Propylthio, n-, i-, s- und t-Butylthio, Trifluormethyl, Trifluormethoxy, Trifluormethylthio, Hydroxy, Nitro, Cyano, Amino, Methylamino, Ethylamino, Dimethylamino und Diethylamino; für jeweils unsubstituiertes oder einfach oder zweifach durch Methyl substituiertes Cyclopropyl, Cyclopentyl oder Cyclohexyl; oder für unsubstituiertes oder ein-oder zweifach, gleich oder verschieden substituiertes Pyridyl, Pyridylmethyl oder 1- bzw. 2-Pyridylethyl steht, wobei als Substituenten die oben genannten Phenylsubstituenten in Frage kommen; oder

$R^5$ und $R^6$ gemeinsam mit dem Stickstoffatom, an das sie gebunden sind, für einen 5- oder 6-gliedrigen Heterocyclus stehen, der gegebenenfalls Sauerstoff oder weitere Stickstoffatome enthalten kann.

Vor allem werden die neuen substituierten Aminosäureamid-Derivate der allgemeinen Formel (Ia) verwendet.

Ein weiterer Gegenstand der Anmeldung sind somit neue substituierte Aminosäureamid-Derivate der allgemeinen Formel (Ia)

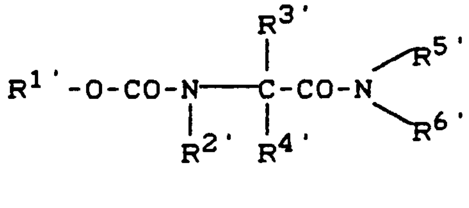

(Ia)

in welcher

$R^{1'}$ für Alkyl, Alkenyl, Alkinyl, Halogenalkyl, Halogenalkenyl, Halogenalkinyl, Cycloalkyl oder Cycloalkenyl steht,

$R^{2'}$ und $R^{5'}$ gleich oder verschieden sind und für Wasserstoff oder Alkyl stehen und

$R^{3'}$ für Wasserstoff steht und
$R^{4'}$ für i-Propyl, i-Butyl, s-Butyl, 3-Pentyl oder Cycloalkyl steht oder
$R^{3'}$ und $R^{4'}$ zusammen mit dem Kohlenstoffatom, an das sie gebunden sind, einen Cycloalkylring bilden und
$R^{6'}$ für Wasserstoff, Alkyl, Alkenyl, Alkinyl, Cyanoalkyl, unsubstituiertes oder substituiertes Phenylalkyl, unsubstituiertes oder substituiertes Phenyl, unsubstituiertes oder substituiertes Cycloalkyl, oder für unsubstituiertes oder substituiertes Heterocyclyl oder Heterocyclylalkyl steht,

oder

$R^{5'}$ und $R^{6'}$ gemeinsam mit dem Stickstoff-Atom, an das sie gebunden sind, für einen Heterocyclyl-Rest stehen, der weitere Heteroatome enthalten kann, ausgenommen Verbindungen, in denen

a)
$R^{4'}$ für i-Butyl steht und
$R^{1'}$ für Methyl oder t-Butyl steht und
$R^{2'}$ für Wasserstoff steht und
$R^{5'}$ für Wasserstoff oder Methyl steht und
$R^{6'}$ für Wasserstoff, Methyl, Ethyl, Phenyl, Benzyl, 2-Phenethyl oder Pyridylmethyl steht oder
$R^{5'}$ und $R^{6'}$ einen Cyclohexylring bilden und

b)
$R^{4'}$ für s-Butyl steht und
$R^{1'}$ für t-Butyl steht und
$R^{2'}$ für Wasserstoff steht und
$R^{5'}$ für Wasserstoff steht und
$R^{6'}$ für Wasserstoff oder Benzyl steht und

c)
$R^{4'}$ für i-Propyl steht und
$R^{1'}$ für Methyl, Ethyl oder t-Butyl steht und
$R^{2'}$ für Wasserstoff steht und
$R^{5'}$ für Wasserstoff steht und
$R^{6'}$ für Wasserstoff, Methyl, Phenyl oder Benzyl steht oder
$R^{5'}$ und $R^{6'}$ für einen Cyclopentylring stehen.

Die Verbindungen der Formel (Ia) können außerdem ein oder mehrere Chiralitätszentren enthalten und können somit in verschiedenen Enantiomeren- und Diastereomerengemischen vorliegen, die gegebenenfalls in üblicher Art und Weise getrennt werden können. Sowohl die reinen Enantiomeren und Diastereomeren, als auch die Gemische werden erfindungsgemäß beansprucht.

Im folgenden wird der Einfachheit halber stets von Verbindungen der Formel (Ia) gesprochen, obwohl sowohl die reinen Verbindungen, als auch die Gemische mit unterschiedlichen Anteilen an isomeren, enantiomeren und diasteromeren Verbindungen gemeint sind.

Bevorzugt sind diejenigen Verbindungen der Formel (Ia), in denen

$R^{1'}$ für geradkettiges oder verzweigtes Alkyl mit 1 bis 6 Kohlenstoffatomen, geradkettiges oder verzweigtes Alkenyl oder Alkinyl mit 2 bis 6 Kohlenstoffatomen, geradkettiges oder verzweigtes Halogenalkyl mit 1 bis 6 Kohlenstoffatomen und 1 bis 9 gleichen oder verschiedenen Halogenatomen, geradkettiges oder verzweites Halogenalkenyl oder Halogenalkinyl mit 2 bis 6 Kohlenstoffatomen und 1 bis 9 gleichen oder verschiedenen Halogenatomen oder unsubstituiertes oder substituiertes Cycloalkyl oder Cycloalkenyl mit 3 bis 7 Kohlenstoffatomen steht und
$R^{2'}$ und $R^{5'}$ gleich oder verschieden sind und für Wasserstoff oder Alkyl mit 1 bis 6 Kohlenstoffatomen stehen und
$R^{3'}$ für Wasserstoff steht und
$R^{4'}$ für i-Propyl, i-Butyl, s-Butyl oder 3-Pentyl steht oder
$R^{3'}$ und $R^{4'}$ zusammen mit dem Kohlenstoffatom, an das sie gebunden sind, einen Cycloalkylring mit 3 bis 7 Kohlenstoffatomen bilden und
$R^{6'}$ für Wasserstoff, geradkettiges oder verzweigtes Alkyl mit 1 bis 6 Kohlenstoffatomen, für Alkenyl oder Alkinyl mit jeweils 2 bis 6 Kohlenstoffatomen, für Cyanalkyl mit 1 bis 4 Kohlenstoffatomen im Alkylteil, unsubstituiertes oder im Phenylteil einfach bis dreifach, gleich oder verschieden substituiertes Phenylalkyl mit 1 bis 4 Kohlenstoffatomen im

Alkylteil oder unsubstituiertes oder einfach bis dreifach, gleich oder verschieden substituiertes Phenyl steht, wobei als Substituenten jeweils in Frage kommen: Halogen; Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 gleichen oder verschiedenen Halogenatomen; Hydroxy; Cyano, Nitro, Amino, Alkylamino, Dialkylamino mit jeweils 1 bis 4 Kohlenstoffatomen und Carboxyl; für unsubstituiertes oder gleich oder verschieden substituiertes Cycloalkyl mit 3 bis 7 Kohlenstoffatomen, wobei als Substituenten Alkyl und Alkoxy mit jeweils 1 bis 4 Kohlenstoffatomen in Frage kommen, oder für unsubstituiertes oder substituiertes Heterocyclyl oder Heterocyclylalkyl mit 2 bis 6 Kohlenstoffatomen im Heterocyclylteil und einem oder zwei Heteroatomen und gegebenenfalls 1 bis 4 Kohlenstoffatomen im Alkylteil steht, wobei als Substituenten die oben genannten Phenylsubstituenten in Frage kommen,

oder

$R^{5'}$ und $R^{6'}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann.

Besonders bevorzugt sind diejenigen Verbindungen der Formel (Ia), in denen

$R^{1'}$ für geradkettiges oder verzweigtes Alkyl mit 1 bis 4 Kohlenstoffatomen, geradkettiges oder verzweigtes Alkenyl oder Alkinyl mit 2 bis 4 Kohlenstoffatomen, geradkettiges oder verzweigtes Halogenalkyl mit 1 bis 4 Kohlenstoffatomen und 1 bis 5 gleichen oder verschiedenen Halogenatomen, geradkettiges oder verzweigtes Halogenalkenyl oder Halogenalkinyl mit 2 bis 4 Kohlenstoffatomen und 1 bis 5 gleichen oder verschiedenen Halogenatomen oder unsubstituiertes oder substituiertes Cycloalkyl oder Cycloalkenyl mit 3 bis 6 Kohlenstoffatomen steht und

$R^{2'}$ und $R^{5'}$ gleich oder verschieden sind und für Wasserstoff, Methyl oder Ethyl stehen und

$R^{3'}$ für Wasserstoff steht und

$R^{4'}$ für i-Propyl, i-Butyl, s-Butyl oder 3-Pentyl steht oder

$R^{3'}$ und $R^{4'}$ zusammen mit dem Kohlenstoffatom, an das sie gebunden sind, einen Cycloalkylring mit 3 bis 6 Kohlenstoffatomen bilden und

$R^{6'}$ für Wasserstoff, Methyl, Ethyl, Allyl, Propargyl, für Cyanoalkyl, mit 1 oder 2 Kohlenstoffatomen im Alkylteil; ferner für unsubstituiertes oder im Phenylteil einfach oder zweifach, gleich oder verschieden substituiertes Phenylalkyl mit 1 bis 4 Kohlenstoffatomen im Alkylteil, für unsubstituiertes oder einfach oder zweifach, gleich oder verschieden substituiertes Phenyl steht, wobei als Substituenten jeweils in Frage kommen: Fluor, Chlor und Brom, Methyl, Ethyl, n- und i-Propyl, n-, i-, s- und t-Butyl, Methoxy, Ethoxy, n- und i-Propyloxy, n-, i-, und t-Butyloxy, Methylthio, Ethylthio, n- und i-Propylthio, n-, i-, s- und t-Butylthio, Trifluormethyl, Trifluormethoxy, Trifluormethylthio, Hydroxy, Nitro, Cyano, Amino, Methylamino, Ethylamino, Dimethylamino, Diethylamino und Carboxy; für jeweils unsubstituiertes oder einfach oder zweifach, gleich oder verschieden substituiertes Cycloalkyl mit 3 bis 6 Kohlenstoffatomen, wobei als Substituenten Methyl, Ethyl, n- und i-Propyl, n-, i-, s- und t-Butyl, Methoxy, Ethoxy, n- und i-Propyloxy, n-, i-, s- und t-Butyloxy in Frage kommen oder für unsubstituiertes oder einfach oder zweifach, gleich oder verschieden substituiertes Heterocyclyl oder Heterocyclylalkyl mit 4 oder 5 Kohlenstoffatomen im Heterocyclylteil und einem oder zwei Stickstoffatomen und gegebenenfalls 1 bis 4 Kohlenstoffatomen im Alkylteil steht, wobei als Substituenten die oben genannten Phenylsubstituenten in Frage kommen; oder

$R^{5'}$ und $R^{6'}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann.

Ganz besonders bevorzugt sind diejenigen Verbindungen der Formel (Ia) in denen

$R^{1'}$ für geradkettiges oder verzweigtes Alkyl mit 1 bis 4 Kohlenstoffatomen, geradkettiges oder verzweigtes Alkenyl oder Alkinyl mit 2 bis 4 Kohlenstoffatomen, geradkettiges oder verzweigtes Halogenalkyl mit 1 bis 4 Kohlenstoffatomen und 1 bis 5 gleichen oder verschiedenen Halogenatomen, geradkettiges oder verzweigtes Halogenalkenyl oder Halogenalkinyl mit 2 bis 4 Kohlenstoffatomen und 1 bis 5 gleichen oder verschiedenen Halogenatomen oder unsubstituiertes oder substituiertes Cycloalkyl oder Cycloalkenyl mit 3 bis 6 Kohlenstoffatomen steht und

$R^{2'}$, $R^{3'}$ und $R^{5'}$ für Wasserstoff stehen und

$R^{4'}$ für i-Propyl, i-Butyl, s-Butyl oder 3-Pentyl steht

$R^{6'}$ für unsubstituiertes oder im Phenylteil einfach oder zweifach, gleich oder verschieden substituiertes Phenylalkyl mit 1 bis 4 Kohlenstoffatomen im Alkylteil, steht, wobei als Substituenten in Frage kommen: Fluor, Chlor und Brom, Methyl, Ethyl, n- und i-Propyl, n-, i-, s- und t-Butyl, Methoxy, Ethoxy, n- und i-Propyloxy, n-, i-, s-und t-Butyloxy, Methylthio, Ethylthio, n- und i-Propylthio, n-, i-, s- und t-Butylthio, Trifluormethyl, Trifluormethoxy, Trifluormethylthio, Cyano, Dimethylamino, Diethylamino und Carboxy; oder für unsubstituiertes oder einfach oder zweifach substituiertes Heterocyclylalkyl mit 4 oder 5 Kohlenstoffatom im Heterocyclylteil und einem oder zwei Stickstoffatomenen und gegebenenfalls 1 bis 4 Kohlenstoffatomen im Alkylteil steht, wobei als Substituenten die obengenannten Phenylsubstituenten in Frage kommen.

Insbesondere bevorzugt sind diejenigen Verbindungen der Formel (Ia), in denen

$R^{1'}$ für Methyl, Ethyl, n- und i-Propyl, n-, i-, s- und t-Butyl, Fluormethyl, Fluorethyl, Fluorpropyl, Chlorpropyl, Fluorbutyl, Chlorbutyl, Difluormethyl, Difluorpropyl, Dichlorpropyl, Difluorbutyl, Dichlorbutyl, Trifluormethyl, Trichlormethyl, Trifluorethyl, Trichlorethyl, Trifluorpropyl, Trichlorpropyl, Trifluorbutyl, Trichlorbutyl, Allyl, Butenyl, Propargyl, Butinyl, Fluor- oder Chlor-allyl, -butenyl, -propargyl, -butinyl, Cyclopropyl, Cyclopentyl, Cyclohexyl, Cyclopentenyl oder Cyclohexenyl steht und

$R^{2'}$, $R^{3'}$ und $R^{5'}$ für Wasserstoff stehen und

$R^{4'}$ für i-Propyl, i-Butyl, s-Butyl oder 3-Pentyl steht

$R^{6'}$ für unsubstituiertes oder im Phenylteil einfach oder zweifach, gleich oder verschieden substituiertes Benzyl, 1-Phenethyl, 2-Phenethyl oder 1-Phenylpropyl, 2-Phenylpropyl, 3-Phenylpropyl, 1-Phenylisopropyl, 2-Phenylisopropyl oder 1-Phenyl-n-, -i-oder -s-butyl steht, wobei als Substituenten jeweils in Frage kommen: Fluor, Chlor, Brom, Methyl, Ethyl, n- und i-Propyl, n-, i-, s- und t-Butyl, Methoxy, Ethoxy, n- und i-Propyloxy, n-, i-, s- und t-Butyloxy, Methylthio, Ethylthio, n-und i-Propylthio, n-, i-, s- und t-Butylthio, Trifluormethyl, Trifluormethoxy, Trifluormethylthio, Cyano, Dimethylamino und Diethylamino; für unsubstituiertes oder ein oder zweifach, gleich oder verschieden substituiertes Pyridylmethyl oder 1- bzw. 2-Pyridylethyl steht, wobei als Substituenten die oben genannten Phenylsubstituenten in Frage kommen.

Man erhält die substituierten Aminosäureamid-Derivate der allgemeinen Formel (Ia)

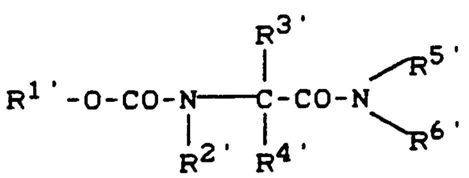

(Ia)

in welcher

$R^{1'}$ für Alkyl, Alkenyl, Alkinyl, Halogenalkyl, Halogenalkenyl, Halogenalkinyl, Cycloalkyl oder Cycloalkenyl steht,

$R^{2'}$ und $R^{5'}$ gleich oder verschieden sind und für Wasserstoff oder Alkyl stehen und

$R^{3'}$ für Wasserstoff steht und

$R^{4'}$ für i-Propyl, i-Butyl, s-Butyl oder 3-Pentyl steht oder

$R^{3'}$ und $R^{4'}$ zusammen mit dem Kohlenstoffatom, an das sie gebunden sind, einen Cycloalkylring bilden und

$R^{6'}$ für Wasserstoff, Alkyl, Alkenyl, Alkinyl, Cyanoalkyl, unsubstituiertes oder substituiertes Phenylalkyl, unsubstituiertes oder substituiertes Phenyl, unsubstituiertes oder substituiertes Cycloalkyl oder für unsubstituiertes oder substituiertes Heterocyclyl oder Heterocyclylalkyl steht,

oder

$R^{5'}$ und $R^{6'}$ gemeinsam mit dem Stickstoff-Atom, an das sie gebunden sind, für einen Heterocyclyl-Rest stehen, der weitere Heteroatome enthalten kann, ausgenommen Verbindungen, in denen

a)

$R^{4'}$ für i-Butyl steht und

$R^{1'}$ für Methyl oder t-Butyl steht und
$R^{2'}$ für Wasserstoff steht und
$R^{5'}$ für Wasserstoff oder Methyl steht und
$R^{6'}$ für Wasserstoff, Methyl, Ethyl, Phenyl, Benzyl, 2-Phenethyl oder Pyridylmethyl steht oder
$R^{5'}$ und $R^{6'}$ einen Cyclohexylring bilden und

b)
$R^{4'}$ für s-Butyl steht und
$R^{1'}$ für t-Butyl steht und
$R^{2'}$ für Wasserstoff steht und
$R^{5'}$ für Wasserstoff steht und
$R^{6'}$ für Wasserstoff oder Benzyl steht und

c)
$R^{4'}$ für i-Propyl steht und
$R^{1'}$ für Methyl, Ethyl oder t-Butyl steht und
$R^{2'}$ für Wasserstoff steht und
$R^{5'}$ für Wasserstoff steht und
$R^{6'}$ für Wasserstoff, Methyl, Phenyl oder Benzyl steht oder
$R^{5'}$ und $R^{6'}$ für einen Cyclopentylring stehen,

wenn man
eine substituierte Aminosäure der Formel (II)

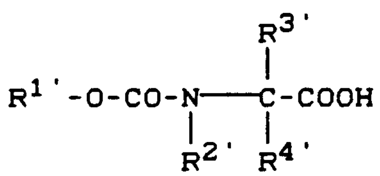 (II)

in welcher
$R^{1'}$, $R^{2'}$, $R^{3'}$ und $R^{4'}$ die oben angegebene Bedeutung haben, bzw. deren carboxy-aktivierte Derivate, gegebenenfalls in Gegenwart eines Katalysators, gegebenenfalls in Gegenwart eines Säurebindemittels und gegebenenfalls in Gegenwart eines Verdünnungsmittels
mit einem Amin der Formel (III)

$HNR^{5'}R^{6'}$ (III)

in welcher
$R^{5'}$ und $R^{6'}$ die oben angegebene Bedeutung haben,
umsetzt.

Die Aminosäure-Derivate der allgemeinen Formel (I), in welcher
$R^1$, $R^2$, $R^3$, $R^4$, $R^5$ und $R^6$ die oben angegebene Bedeutung haben,
können ebenfalls nach diesem Verfahren hergestellt werden.

Verwendet man beispielsweise tert.-Butoxycarbonyl-D/L-Valin und 4-Methoxyphenethylamin als Ausgangsprodukte, so kann der Ablauf des erfindungsgemäßen Verfahrens durch das folgende Formelschema wiedergegeben werden:

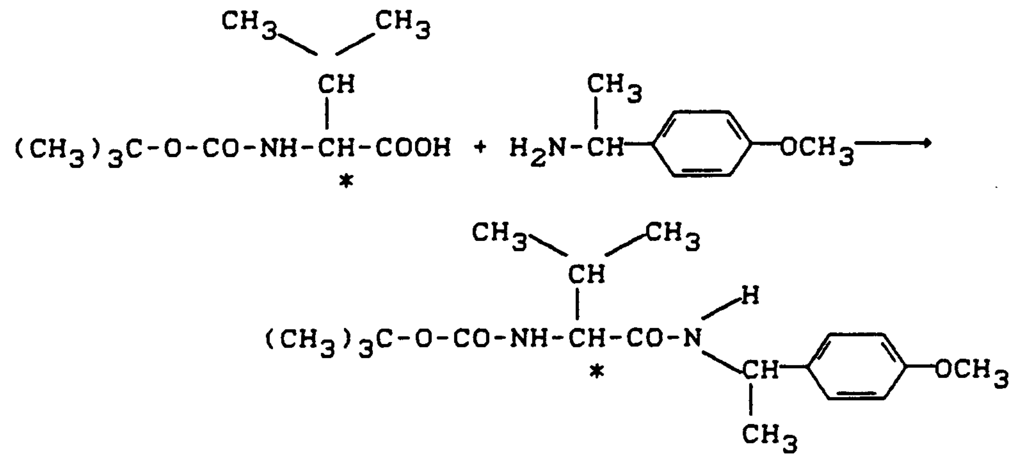

Die für die Durchführung des erfindungsgemäßen Verfahrens als Ausgangsstoffe zu verwendenden Aminosäure-Derivate sind durch die Formel (II) allgemein definiert. In dieser Formel haben $R^{1'}$, $R^{2'}$, $R^{3'}$ und $R^{4'}$ vorzugsweise die Bedeutungen, die bereits im Zusammenhang mit der Beschreibung der erfindungsmäßen Stoffe der Formel (Ia) für diese Substituenten bevorzugt genannt wurden.

Die Aminosäure-Derivate der Formel (II) sind allgemein bekannt (vgl. z.B. Houben-Weyl, Methoden der organischen Chemie, Band XV, Teil 1 und 2, Seiten 46 ff und 112 ff, Georg Thieme Verlag, Stuttgart 1974; D. Keller et.al., Org. Synth. 60, 2145 (1981); bzw. R.C. Sheppard, A Specialist Periodical Report, Aminoacids, Peptids and Proteins, The Royal Society of Chemistry, Burlington House, London 1978, bzw. I.P. Greenstein and M. Winitz, Chemistry of Amino Acids, I. Wiley Sons Inc., New York, London 1961: bzw. E. Schröder and K. Lübke, The Peptides Vol. I, Academic Press, New York, London 1965) oder können nach den dort angegebenen Verfahren erhalten werden.

Die außerdem für die Durchführung des erfindungsgemäßen Verfahrens als Ausgangsstoffe zu verwendenden carboxyaktivierten Derivate der Aminosäure der Formel (II) sind allgemein bekannte.

Als carboxy-aktivierte Derivate der Aminosäuren der Formel (II) kommen alle Carboxy-aktivierten Derivate infrage, wie Säurehalogenide, wie z.B. Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxisuccinimidester sowie mit Kondensationsmitteln, wie z.B. Dicyclohexylcarbodiimid oder Carbonyldiimidazol, in situ erzeugte aktivierte Formen der Aminosäuren.

Vorzugsweise werden die den Aminosäuren der Formel (II) entsprechenden Säurechloride und gemischten Anhydride eingesetzt. Sie können hergestellt werden, indem man die Aminosäuren der Formel (II) oder deren Salze mit einem Halogenierungsmittel oder einem der allgemein bekannten Mittel zur Herstellung von gemischten Anhydriden, wie beispielsweise Phosphorpentachlorid, Thionylchlorid, Oxalylchlorid oder Chlorameisensäureisobutylester, in allgemein bekannter Art und Weise umsetzt. Bevorzugt ist der Einsatz von Chlorameisensäureisobutylester.

Die Umsetzung kann in Gegenwart indifferenter Verdünnungsmittel wie z.B. aromatische, nichtaromatische oder halogenierte Kohlenwasserstoffe wie: Ketone, wie z.B. Aceton; Ester, wie z.B. Ethylacetat; Amide, wie z.B. Dimethylformamid-; Nitrile, wie z.B. Acetonitril; Chlorkohlenwasserstoffe, wie z.B. Methylenchlorid: Kohlenwasserstoffe, wie z.B. Toluol; oder Ether, wie z.B. Tetrahydrofuran bzw. deren Mischungen und/oder in Gegenwart eines Säurebindemittels, wie vorzugsweise eines tertiären Amins, wie z.B. Triethylamin, Pyridin oder N-Methylpiperidin, bei Temperaturen von -78 °C bis 100 °C, vorzugsweise -60 °C bis 25 °C, durchgeführt werden.

Die außerdem für die Durchführung des erfindungsgemäßen Verfahrens als Ausgangsstoffe zu verwendenden Amine sind durch die Formel (III) allgemein definierte. In dieser Formel haben $R^{5'}$ und $R^{6'}$ die oben genannten Bedeutungen.

Die Amine der Formel (III) sind allgemein bekannte Verbindungen der organischen Chemie.

Als Verdünnungsmittel kommen für das erfindungsgemäße Verfahren inerte, organische Lösungsmittel wie: Ketone, wie Aceton oder Ethylmethylketon; Ester wie Ethyl- oder Methylacetat; Amide wie Dimethylformamid; Nitrile, wie Acetonitril; Chlorkohlenwasserstoffe, wie Methylenchlorid oder Tetrachlorkohlenstoff; Kohlenwasserstoffe, wie Toluol oder Ether, wie Tetrahydrofuran sowie gegebenenfalls Wasser und deren Mischungen in Frage.

Als Säurebindemittel kommen für das erfindungsgemäße Verfahren übliche anorganische und organische Säurebinder in Frage. Hierzu gehören vorzugsweise tertiäre Amine, wie Triethylamin, Pyridin oder N-Methylpiperidin, sowie anorganische Basen, z.B. Metallhydroxide wie Natrium- und Kaliumhydroxid oder Metallcarbonate wie Natriumcarbonat oder Calciumcarbonat.

Das erfindungsgemäße Verfahren wird gegebenenfalls in Gegenwart eines Katalysators durchgeführt. Beispielsweise genannt seien 4-Dimethylaminopyridin, 1-Hydroxy-benzotriazol oder Dimethylformamid.

Die Temperaturen können bei der Durchführung des Verfahrens in einem größeren Bereich variiert werden. Im allgemeinen arbeitet man zwischen -78 bis +120 °C, vorzugsweise bei -60 bis +40 °C.

Bei der Durchführung des erfindungsgemäßen Verfahrens arbeitet man vorzugsweise in äquimolaren Mengen.

Dabei werden die Aminosäurederivate der Formel (II) als reine optische Isomere (D bzw. L-Form) oder als Racemate eingesetzt.

Die Erfindung umfaßt sowohl die reinen Isomeren als auch die Gemische. Diese Gemische können nach gebräuchlichen Methoden, z.B. selektive Kristallisation aus geeigneten Lösungsmitteln oder Chromatographie an Kieselgel oder Aluminiumoxid in die Komponenten aufgetrennt werden. Racemate können nach üblichen Methoden in die einzelnen Enantiomeren aufgetrennt werden, so z.B. durch Salzbildung mit optisch aktiven Säuren wie Camphersulfonsäure oder Dibenzoylweinsäure und selektive Kristallisation oder durch Derivatisierung mit geeigneten, optisch aktiven Reagenzien, Trennung der diastereomeren Derivate und Rückspaltung oder Trennung an optisch aktivem Säulenmaterial.

Die erfindungsgemäßen und bekannten Wirkstoffe der Formeln (Ia) bzw. (I) weisen eine starke Wirkung gegen Schädlinge auf und können zur Bekämpfung von unerwünschten Schadorganismen praktisch eingesetzt werden. Die Wirkstoffe sind für den Gebrauch als Fungizide in Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg protektiv und systemisch zur Bekämpfung von Phytophthora-Arten an Tomaten oder Plasmopara-Arten an Reben sowie

zur Bekämpfung von Reiskrankheiten, wie beispielsweise gegen den Erreger der Reisfleckenkrankheit (Pyricularia oryzae) oder gegen den Erreger der Reisstengelkrankheit (Pellicularia sasakii) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; al feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wi Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut von Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1

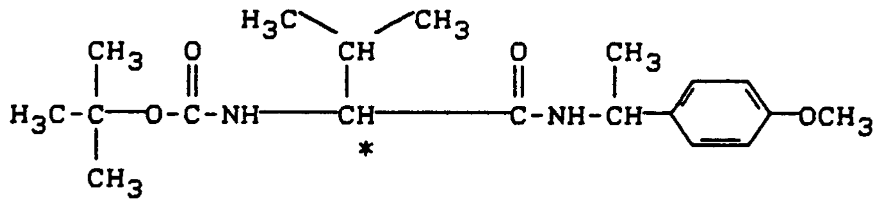

Zu 5,0 g t-Butoxycarbonyl-L-Valin (0,023 Mol), gelöst in 50 ml $CH_2Cl_2$, werden bei -20 °C 2,3 g (0,023 Mol) N-Methylpiperidin zugegeben. Anschließend tropft man bei -20 °C schnell 3,2 g (0,023 Mol) Chlorameisensäureisobutylester zu, rührt bei gleicher Temperatur 10 Minuten nach, kühlt auf -60 °C ab und läßt 3,5 g (0,023 Mol) 4-Methoxy-1-phenylethylamin zulaufen, wobei die Temperatur unter -15 °C gehalten wird. Nach 2 Stunden bei -15 °C läßt man 15 Stunden bei Raumtemperatur nachrühren, filtriert vom Feststoff ab, wäscht mit $CH_2Cl_2$ nach, engt ein, gibt den Rückstand auf Wasser, extrahiert 2mal mit Essigester, wäscht die vereinigten Essigester-Phasen mit $NaHCO_3$-Lösung und Wasser, trocknet und engt ein. Man erhält 17,5 g (85 % d. Th.) N-(t-Butyloxycarbonyl)-L-valin-4-methoxyphenylethylamid mit einem Schmelzpunkt von 126-127 °C.

Analog Beispiel 1 werden die folgenden Verbindungen der Formel (Ia) erhalten

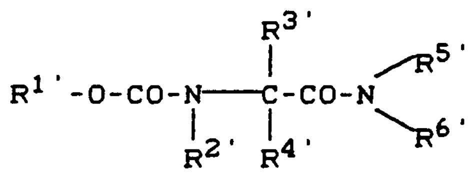

(Ia)

<u>Tabelle 1</u>

| Nr. | $R^{1'}$ | $R^{2'}$ | $R^{3'}$ | $R^{4'}$ | N | | phys. Konstante | eingesetzte Aminosäure |
|---|---|---|---|---|---|---|---|---|
| | | | | | $R^{5'}$ | $R^{6'}$ | | |
| 2 | $(CH_3)_3C-$ | H | H | $-CH(CH_3)_2$ | $CH_3$ | $-CH(CH_3)-$ 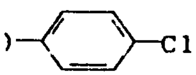 | NMR: δ 0,9-1,0/ 1,4-1,5/ 7,1-7,4 | Boc-L-Valin |
| 3 | $(CH_3)_3C-$ | H | H | $-CH(CH_3)_2$ | H | $-CH(CH_3)-$ 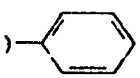 | mp: 119-120° C | Boc-L-Valin |
| 4 | $(CH_3)_3C-$ | H | H | $-CH(CH_3)_2$ | H | $-CH(CH_3)-$ 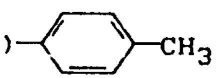 | mp: 139-140° C | Boc-L-Valin |
| 5 | $(CH_3)_3C-$ | H | H | $-CH(CH_3)_2$ | H | $-CH(CH_3)-$ 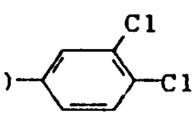 | mp: 95-100° C | Boc-L-Valin |
| 6 | $(CH_3)_3C-$ | H | H | $-CH(CH_3)_2$ | H | $-CH(CH_3)-$ 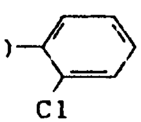 | mp: 146-147° C | Boc-L-Valin |
| 7 | $(CH_3)_3C-$ | H | H | $-CH(CH_3)_2$ | H | $-CH(C_2H_5)-$ 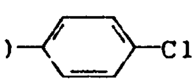 | mp: 150-151° C | Boc-L-Valin |

Tabelle 1 Fortsetzung

| Nr. | $R^{1'}$ | $R^{2'}$ | $R^{3'}$ | $R^{4'}$ | 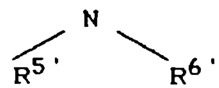 | | phys. Konstante | eingesetzte Aminosäure |
|---|---|---|---|---|---|---|---|---|
| 8 | $(CH_3)_3C-$ | H | H | $-CH(CH_3)_2$ | H | $-CH(CH_3)-$ 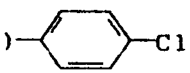 * | mp: 137-138° C *: R-(+)- | Boc-L-Valin |
| 9 | $(CH_3)_3C-$ | H | H | $-CH(CH_3)_2$ | H | $-CH_2-CH_2-$ 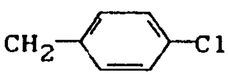 | mp: 172-174° C | Boc-L-Valin |
| 10 | $(CH_3)_3C-$ | H | H | $-CH(CH_3)_2$ | H | $-CH_2-CH_2-$ 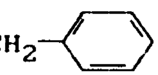 | mp: 166-167° C | Boc-L-Valin |
| 11 | $(CH_3)_3C-$ | H | H | $-CH(CH_3)_2$ | $CH_3$ | $-CH_2-CH_2-$ 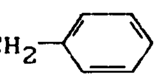 | NMR: δ 0,8-1,0/ 1,4/ 3,4-3,6 | Boc-L-Valin |
| 12 | $(CH_3)_3C-$ | H | H | $-CH(CH_3)_2$ | H | H | mp: 154-156° C | Boc-L-Valin |
| 13 | $(CH_3)_3C-$ | H | H | $-CH(CH_3)_3$ | $CH_3$ | $CH_3$ | NMR: δ 0,8-1,0/ 1,4/ 2,95-3,1 | Boc-L-Valin |

Tabelle 1 Fortsetzung

| Nr. | $R^{1'}$ | $R^{2'}$ | $R^{3'}$ | $R^{4'}$ | 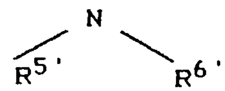 | | phys. Konstante | eingesetzte Aminosäure |
|---|---|---|---|---|---|---|---|---|
| 14 | $(CH_3)_3C-$ | H | H | $-CH(CH_3)_2$ | $C_2H_5$ | $C_2H_5$ | NMR: δ 0,9-1,0/ 1,1/1,2/ 1,4 | Boc-L-Valin |
| 15 | $(CH_3)_3C-$ | H | H | $-CH(CH_3)_2$ | H | $CH_3$ | mp: 130-131° C | Boc-L-Valin |
| 16 | $(CH_3)_3C-$ | H | H | $-CH(CH_3)_2$ | 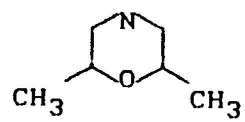 | | NMR: δ 0,8-1,0/ 1,2-1,3/ 1,4 | Boc-L-Valin |
| 17 | $(CH_3)_3C-$ | H | H | $-CH(CH_3)_2$ | H | $C_2H_5$ | mp: 121-122° C | Boc-L-Valin |
| 18 | $(CH_3)_3C-$ | H | H | $-CH(CH_3)_2$ | 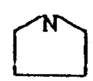 | | NMR: δ 0,9-1,0/ 1,4/ 1,8-2,0 | Boc-L-Valin |
| 19 | $(CH_3)_3C-$ | H | H | $-CH(CH_3)_2$ | H | $-CH_2-$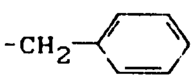 | mp: 123-124° C | Boc-L-Valin |

<u>Tabelle 1</u> Fortsetzung

| Nr. | $R^{1'}$ | $R^{2'}$ | $R^{3'}$ | $R^{4'}$ | 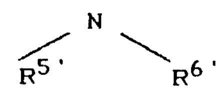 | phys. Konstante | eingesetzte Aminosäure |
|---|---|---|---|---|---|---|---|
| 20 | $(CH_3)_3C-$ | H | H | $-CH(CH_3)_2$ | H 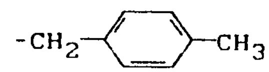 | mp: 143-144° C | Boc-L-Valin |
| 21 | $(CH_3)_3C-$ | H | H | $-CH(CH_3)_2$ | H 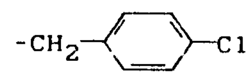 | mp: 124-125° C | Boc-L-Valin |
| 22 | $(CH_3)_3C-$ | H | H | $-CH(CH_3)_2$ | H 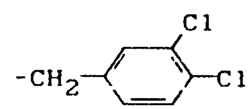 | mp: 112-113° C | Boc-L-Valin |
| 23 | $(CH_3)_3C-$ | H | H | $-CH(CH_3)_2$ | H 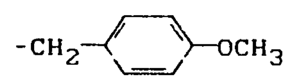 | mp: 114-115° C | Boc-L-Valin |
| 24 | $(CH_3)_3C-$ | H | H | $-CH(CH_3)_2$ | H 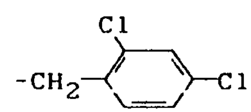 | mp: 121-122° C | Boc-L-Valin |
| 25 | $(CH_3)_3C-$ | H | H | $-CH(CH_3)_2$ | H 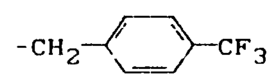 | mp: 120-121° C | Boc-L-Valin |

<u>Tabelle 1</u> Fortsetzung

| Nr. | $R^{1'}$ | $R^{2'}$ | $R^{3'}$ | $R^{4'}$ | 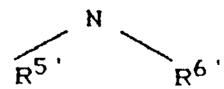 | | phys. Konstante | eingesetzte Aminosäure |
|---|---|---|---|---|---|---|---|---|
| 26 | $(CH_3)_3C-$ | H | H | $-CH(CH_3)_2$ | H | 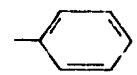 | mp: 160-161° C | Boc-L-Valin |
| 27 | $(CH_3)_3C-$ | H | H | $-CH(CH_3)_2$ | H | 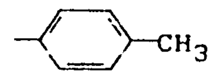 | mp: 133-134° C | Boc-L-Valin |
| 28 | $(CH_3)_3C-$ | H | H | $-CH(CH_3)_2$ | H | 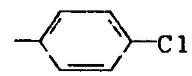 | mp: 142-144° C | Boc-L-Valin |
| 29 | $(CH_3)_3C-$ | H | H | $-CH(CH_3)_2$ | H | 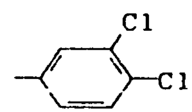 | mp: 143-144° C | Boc-L-Valin |
| 30 | $(CH_3)_3C-$ | H | H | $-CH(CH_3)_2$ | H | 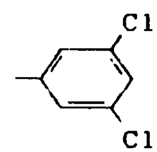 | mp: 133-135° C | Boc-L-Valin |
| 31 | $(CH_3)_3C-$ | H | H | $-CH(CH_3)_2$ | H | $-CH_2-$ 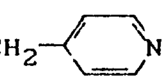 | mp: 120-122° C | Boc-L-Valin |

Tabelle 1 Fortsetzung

| Nr. | $R^{1'}$ | $R^{2'}$ | $R^{3'}$ | $R^{4'}$ | N $R^{5'}$ | N $R^{6'}$ | phys. Konstante | eingesetzte Aminosäure |
|---|---|---|---|---|---|---|---|---|
| 32 | $(CH_3)_3C-$ | H | H | $-CH(CH_3)_2$ | H | $-CH_2-$ 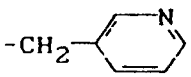 | mp: 85-87° C | Boc-L-Valin |
| 33 | $(CH_3)_3C-$ | H | H | $-CH(CH_3)_2$ | H | $-CH_2-$ 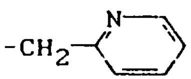 | mp: 102-104° C | Boc-L-Valin |
| 34 | $(CH_3)_3C-$ | H | H | $-CH(CH_3)_2$ | H | 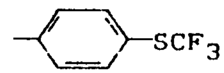 | mp: 79-81° C | Boc-L-Valin |
| 35 | $(CH_3)_3C-$ | H | H | $-CH(CH_3)_2$ | H | 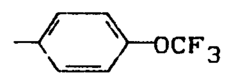 | NMR: δ 0,9-1,0/ 1,4/ 7,0-7,1/ 7,4-7,5 | Boc-L-Valin |
| 36 | $(CH_3)_3C-$ | H | H | $-CH(CH_3)_2$ | H | 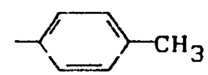 | mp: 127-129° C | Boc-L-Valin |
| 37 | $(CH_3)_3C-$ | H | H | $-CH_2-CH(CH_3)_2$ | H | $-CH(CH_3)-$ * 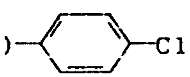 | mp: 141-143° C *: R-(+)- | Boc-L-Leucin |

<u>Tabelle 1</u> Fortsetzung

| | | | | | N | | | |
|---|---|---|---|---|---|---|---|---|
| Nr. | $R^{1'}$ | $R^{2'}$ | $R^{3'}$ | $R^{4'}$ | $R^{5'}$ | $R^{6'}$ | phys. Konstante | eingesetzte Aminosäure |
| 38 | $(CH_3)_3C-$ | H | H | $-CH_2-CH(CH_3)_2$ | H | $-CH(CH_3)-$ 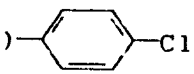 | mp: 119-121° C | Boc-L-Leucin |
| 39 | $(CH_3)_3C-$ | H | H | $-CH(CH_3)-CH_2-CH_3$ | H | $-CH(CH_3)-$ 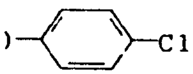 * | mp: 178-180° C *: R-(+)- | Boc-L-Isoleucin |
| 40 | $(CH_3)_3C-$ | H | H | $-CH(CH_3)-CH_2-CH_3$ | H | $-CH(CH_3)-$ 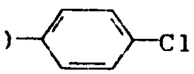 | mp: 112-114° C | Boc-L-Isoleucin |
| 41 | $(CH_3)_3C-$ | H | H | $-CH(CH_3)_2$ | H | $-CH(CH_3)-$ 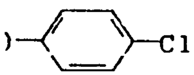 * | mp: 161-163° C *: R-(+)- | Boc-D/L-Valin |
| 42 | $(CH_3)_3C-$ | H | H | $-CH(CH_3)_2$ | H | $-CH(CH_3)-$ 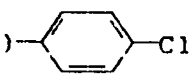 | mp: 159-161° C | Boc-D/L-Valin |
| 43 | $(CH_3)_3C-$ | H | H | $-CH(CH_3)_2$ | H | 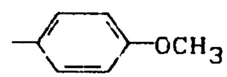 | mp: 173-175° C | Boç-D/L-Valin |
| 44 | $(CH_3)_3C-$ | H | H | $-CH(CH_3)_2$ | H | $-CH_2-CN$ | mp: 96-98° C | Boc-D/L-Valin |

Tabelle 1 Fortsetzung

| Nr. | $R^{1'}$ | $R^{2'}$ | $R^{3'}$ | $R^{4'}$ | N<br>$R^{5'}$ | N<br>$R^{6'}$ | phys. Konstante | eingesetzte Aminosäure |
|---|---|---|---|---|---|---|---|---|
| 45 | 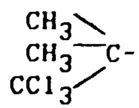 | H | H | $-CH(CH_3)_2$ | H | $-CH(CH_3)-$ 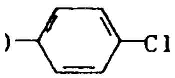 | mp: 168-169° C | Trichlorbut-oxycarbonyl-L-Valin |
| 46 | 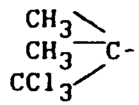 | H | H | $-CH(CH_3)_2$ | H | $-CH(CH_3)-$ 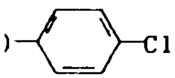 | mp: 154-155° C | Trichlorbut-oxycarbonyl-D/L-Valin |
| 47 | 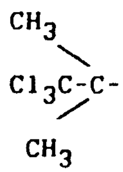 | H | H | $-CH(CH_3)_2$ | H | 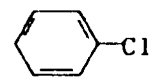 | mp: 181-182° C | Trichlorbut-oxycarbonyl-L-Valin |
| 48 | 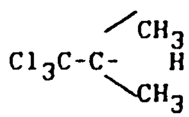 | H | H | $-CH(CH_3)_2$ | H | $-CH_2-$ 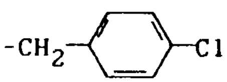 | mp: 150-151° C | Trichlorbut-oxycarbonyl-L-Valin |
| 49 | $CH_3-$ | H | H | $-CH(CH_3)_2$ | H | $-CH(CH_3)-$ 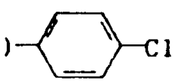 | mp: 160-161° C | Methoxy-carbonyl-L-Valin |
| 50 | $CH_3CH_2-$ | H | H | $-CH(CH_3)_2$ | H | $-CH(CH_3)-$ 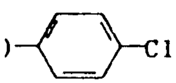 | mp: 164-165° C | Ethoxy-carbonyl-L-Valin |

**<u>Tabelle 1</u> Fortsetzung**

| Nr. | $R^{1'}$ | $R^{2'}$ | $R^{3'}$ | $R^{4'}$ | 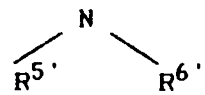 | | phys. Konstante | eingesetzte Aminosäure |
|---|---|---|---|---|---|---|---|---|
| 51 | $CH_3$-$CH_2$- | H | H | -$CH(CH_3)_2$ | H | -$CH(CH_3)$- 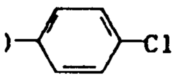 | mp: 136-137° C | Ethoxy-carbonyl-D/L-Valin |
| 52 | $CH_2$=$CHCH_2$- | H | H | -$CH(CH_3)_2$ | H | -$CH(CH_3)$- 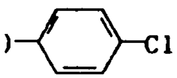 | mp: 146-147° C | Allyloxy-carbonyl-L-Valin |
| 53 | $CH_2$=$CHCH_2$- | H | H | -$CH(CH_3)_2$ | H | 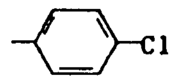 | mp: 167-168° C | Allyloxy-carbonyl-L-Valin |
| 54 | $CH_2$=$CHCH_2$- | H | H | -$CH(CH_3)_2$ | H | -$CH_2$- 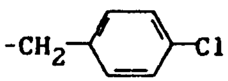 | mp: 170-171° C | Allyloxy-carbonyl-L-Valin |
| 55 | $CH_3CH_2CH_2CH_2$- | H | H | -$CH(CH_3)_2$ | H | -$CH(CH_3)$- 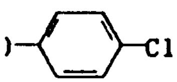 | mp: 172-173° C | n-Butyloxy-carbonyl-L-Valin |
| 56 | $CH_3CH_2CH_2CH_2$- | H | H | -$CH(CH_3)_2$ | H | 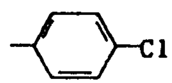 | mp: 146-147° C | n-Butyloxy-carbonyl-L-Valin |
| 57 | $CH_3CH_2CH_2CH_2$- | H | H | -$CH(CH_3)_2$ | H | -$CH_2$- 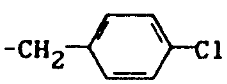 | mp: 148-149° C | n-Butyloxy-carbonyl-L-Valin |

<u>Tabelle 1</u> Fortsetzung

| Nr. | $R^{1'}$ | $R^{2'}$ | $R^{3'}$ | $R^{4'}$ | N: $R^{5'}$ | N: $R^{6'}$ | phys. Konstante | eingesetzte Aminosäure |
|---|---|---|---|---|---|---|---|---|
| 58 | $(CH_3)_2CHCH_2$- | H | H | $-CH(CH_3)_2$ | H | $-CH(CH_3)$ 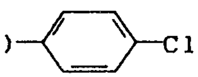 | mp: 161-162° C | i-Butyloxy-carbonyl-L-Valin |
| 59 | $(CH_3)_2CHCH_2$- | H | H | $-CH(CH_3)_2$ | H | 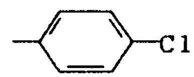 | mp: 160-162° C | i-Butyloxy-carbonyl-L-Valin |
| 60 | $(CH_3)_2CHCH_2$- | H | H | $-CH(CH_3)_2$ | H | $-CH_2$ 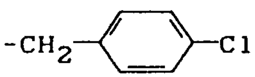 | mp: 157-158° C | i-Butyloxy-carbonyl-L-Valin |
| 61 | 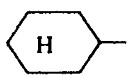 | H | H | $-CH(CH_3)_2$ | H | $-CH(CH_3)$ 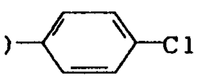 | mp: 160-161° C | Cyclohexyl-oxycarbonyl-L-Valin |
| 62 | 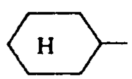 | H | H | $-CH(CH_3)_2$ | H | 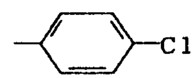 | mp: 195-197° C | Cyclohexyl-oxycarbonyl-L-Valin |
| 63 | 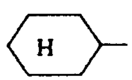 | H | H | $-CH(CH_3)_2$ | H | $-CH_2$ 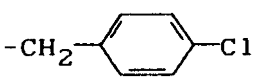 | mp: 180-181° C | Cyclohexyl-oxycarbonyl-L-Valin |

<u>Tabelle 1</u> Fortsetzung

| Nr. | $R^{1'}$ | $R^{2'}$ | $R^{3'}$ | $R^{4'}$ | $R^{5'}$ (N) | $R^{6'}$ | phys. Konstante | eingesetzte Aminosäure |
|---|---|---|---|---|---|---|---|---|
| 64 | $CH_2=CH-CH_2-$ | H | H | $-CH(CH_3)_2$ | H | 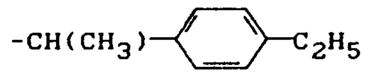 | mp: 147-152° C | Alkoxycarbonyl-L-Valin |
| 65 | $CH_2=CH-CH_2-$ | H | H | $-CH(CH_3)_2$ | H | 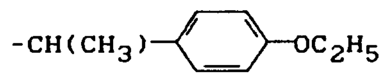 | mp: 163-167° C | Alkoxycarbonyl-L-Valin |
| 66 | $(CH_3)_3C$ | H | H | $-CH_2-CH(CH_3)_2$ | H | 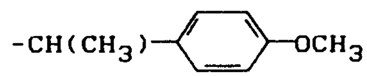 | | BOC-L-Leucin |
| 67 | 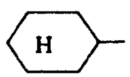 | H | H | $-CH(CH_3)_2$ | H | 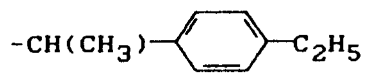 | mp: 178-183° C | Cyclohexyloxycarbonyl-L-Valin |
| 68 | 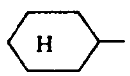 | H | H | $-CH(CH_3)_2$ | H | 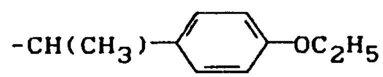 | mp: 177-183° C | Cyclohexylcarbonyl-L-Valin |
| 69 | 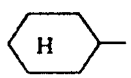 | H | H | $-CH(CH_3)_2$ | H | 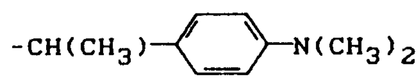 | mp: 124-128° C | Cyclohexyloxycarbonyl-L-Valin |
| 70 | $CH_2=CH-CH_2-$ | H | H | $-CH(CH_3)_2$ | H | 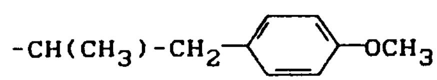 | mp: 159-160° C | Allyloxycarbonyl-L-Valin |
| 71 | $CH_2=CH-CH_2-$ | H | H | $-CH(CH_3)_2$ | H | 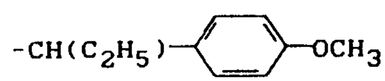 | mp: 168-170° C | Allyloxycarbonyl-L-Valin |

**Tabelle 1** Fortsetzung

| Nr. | $R^{1'}$ | $R^{2'}$ | $R^{3'}$ | $R^{4'}$ | N<br>$R^{5'}$ | $R^{6'}$ | phys. Konstante | eingesetzte Aminosäure |
|---|---|---|---|---|---|---|---|---|
| 72 | $CH_2=CH-CH_2-$ | H | H | $-CH(CH_3)_2$ | H | $-CH(C_2H_5)-$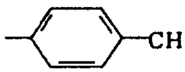$-CH_3$ | mp: 170-171° C | Allyloxy-carbonyl-L-Valin |
| 73 | 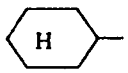 | H | H | $-CH(CH_3)_2$ | H | $-CH(CH_3)-CH_2-$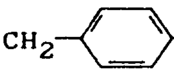$-OCH_3$ | mp: 153-154° C | Cyclohexyl-oxycarbonyl-L-Valin |
| 74 | 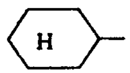 | H | H | $-CH(CH_3)_2$ | H | $-CH(C_2H_5)-$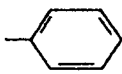$-OCH_3$ | mp: 164-168° C | Cyclohexyl-oxycarbonyl-L-Valin |
| 75 | 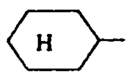 | H | H | $-CH(CH_3)_2$ | H | $-CH(C_2H_5)-$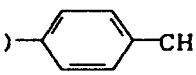$-CH_3$ | mp: 151-152° C | Cyclohexyl-oxycarbonyl-L-Valin |
| 76 | $CH_2=CH-CH_2-$ | H | H | $-CH(CH_3)_2$ | H | $-CH(CH_3)-CH_2-CH_2-$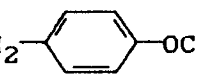$-OCH_3$ | mp: 164-168° C | Allyloxy-carbonyl-L-Valin |
| 77 | $CH_2=CH-CH_2-$ | H | H | $-CH(CH_3)_2$ | H | $-CH(CH_3)-$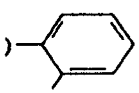 $OCH_3$ | mp: 152-153° C | Allyloxy-carbonyl-L-Valin |
| 78 | $CH_2=CH-CH_2-$ | H | H | $-CH(CH_3)_2$ | H | $-CH(CH_3)-$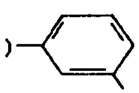 $OCH_3$ | mp: 77-80° C | Allyloxy-carbonyl-L-Valin |

Tabelle 1 Fortsetzung

| Nr. | $R^{1'}$ | $R^{2'}$ | $R^{3'}$ | $R^{4'}$ | N: $R^{5'}$ | N: $R^{6'}$ | phys. Konstante | eingesetzte Aminosäure |
|---|---|---|---|---|---|---|---|---|
| 79 | 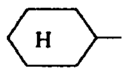 | H | H | $-CH(CH_3)_2$ | H | $-CH(CH_3)-CH_2-CH_2-$ 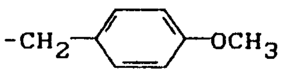 | mp: 167-171° C | Cyclohexyl-oxycarbonyl-L-Valin |
| 80 | 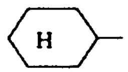 | H | H | $-CH(CH_3)_2$ | H | $-CH(CH_3)-$ 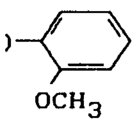 | mp: 169-172° C | Cyclohexyl-oxycarbonyl-L-Valin |
| 81 | 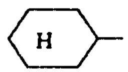 | H | H | $-CH(CH_3)_2$ | H | $-CH(CH_3)-$ 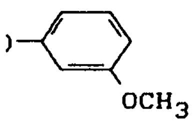 | mp: 158-163° C | Cyclohexyl-oxycarbonyl-L-Valin |
| 82 | $CH_2=CH-CH_2-$ | H | H | $-CH(CH_3)_2$ | H | $-CH(C_2H_5)-$ 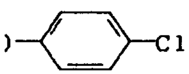 | mp: 153-157° C | Allyloxy-carbonyl-L-Valin |
| 83 | $CH_2=CH-CH_2-$ | H | H | $-CH(CH_3)_2$ | H | $-CH(CH_3)-$ 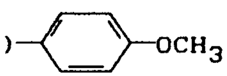 | mp: 152-153° C | Allyloxy-carbonyl-L-Valin |
| 84 | $CH_2=CH-CH_2-$ | H | H | $-CH(CH_3)_2$ | H | $-CH(CH_3)-$ 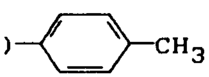 | mp: 139-145° C | Allyloxy-carbonyl-L-Valin |
| 85 | 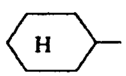 | H | H | $-CH(CH_3)_2$ | H | $-CH(C_2H_5)-$ 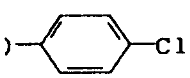 | mp: 172-173° C | Cyclohexyl-oxycarbonyl-L-Valin |

<u>Tabelle 1</u> Fortsetzung

| Nr. | $R^{1'}$ | $R^{2'}$ | $R^{3'}$ | $R^{4'}$ | N $R^{5'}$ | $R^{6'}$ | phys. Konstante | eingesetzte Aminosäure |
|---|---|---|---|---|---|---|---|---|
| 86 | 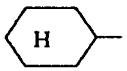 | H | H | $-CH(CH_3)_2$ | H | $-CH(CH_3)-$ 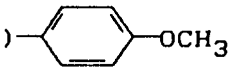 | mp: 166-167° C | Cyclohexyl-oxycarbonyl-L-Valin |
| 87 | 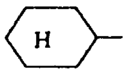 | H | H | $-CH(CH_3)_2$ | H | $-CH(CH_3)-$ 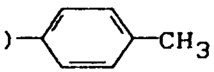 | mp: 167-180° C | Cyclohexyl-oxycarbonyl-L-Valin |
| 88 | 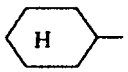 | H | H | $-CH(CH_3)_2$ | H | $-CH(CH_3)-$ 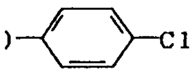 | mp: 169-172° C | Cyclohexyl-oxycarbonyl-L-Valin |
| 89 | 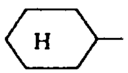 | H | H | $-CH(CH_3)_2$ | H | $-CH(CH_3)-$ 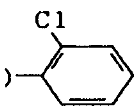 | mp: 177-180° C | Cyclohexyl-oxycarbonyl-L-Valin |
| 90 | 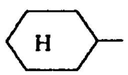 | H | H | $-CH(CH_3)_2$ | H | $-CH(CH_3)-$ 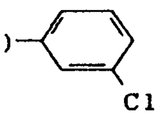 | mp: 167-170° C | Cyclohexyl-oxycarbonyl-L-Valin |
| 91 | 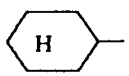 | H | H | $-CH(CH_3)_2$ | H | $-CH(CH_3)-$ 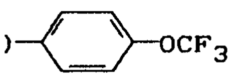 | mp: 183-187° C | Cyclohexyl-carbonyl-L-Valin |
| 92 | 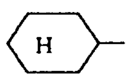 | H | H | $-CH(CH_3)_2$ | H | $-CH(CH_3)-$ 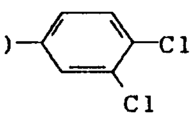 | mp: 176-180° C | Cyclohexyl-carbonyl-L-Valin |

Tabelle 1 Fortsetzung

| Nr. | $R^{1'}$ | $R^{2'}$ | $R^{3'}$ | $R^{4'}$ | N $R^{5'}$ | N $R^{6'}$ | phys. Konstante | eingesetzte Aminosäure |
|---|---|---|---|---|---|---|---|---|
| 93 | $(CH_3)_2CH-CH_2-$ | H | H | $-CH(CH_3)_2$ | H | $-CH(CH_3)-$ 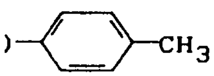 | mp: 160-166° C | i-Butyloxy-carbonyl-L-Valin |
| 94 | $(CH_3)_2CH-CH_2-$ | H | H | $-CH(CH_3)_2$ | H | $-CH(CH_3)-$ 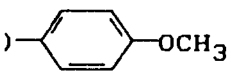 | mp: 154-158° C | i-Butyloxy-carbonyl-L-Valin |
| 95 | $(CH_3)_2CH-CH_2-$ | H | H | $-CH(CH_3)_2$ | H | $-CH(C_2H_5)-$ 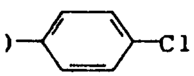 | mp: 150-151° C | i-Butyloxy-carbonyl-L-Valin |
| 96 | $(CH_3)_2CH-CH_2$ | H | H | $-CH(CH_3)_2$ | H | $-CH(CH_3)-$ 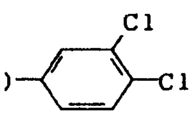 | mp: 172-176° C | i-Butyloxy-carbonyl-L-Valin |
| 97 | $(CH_3)_2CH-CH_2-$ | H | H | $-CH(CH_3)_2$ | H | $-CH(C_2H_5)-$ 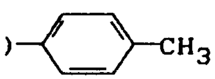 | mp: 154-159° C | i-Butyloxy-carbonyl-L-Valin |
| 98 | $(CH_3)_2CH-CH_2-$ | H | H | $-CH(CH_3)_2$ | H | $-CH(C_2H_5)-$ 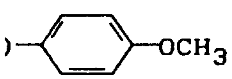 | mp: 164-170° C | i-Butyloxy-carbonyl-L-Valin |
| 99 | 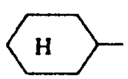 | H | H | $-CH(CH_3)_2$ | H | $-CH(CH_3)-$ 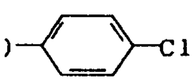 | mp: | Cyclohexyl-oxycarbonyl-L-Valin |

<u>Tabelle 1</u> Fortsetzung

| Nr. | $R^{1'}$ | $R^{2'}$ | $R^{3'}$ | $R^{4'}$ | $R^{5'}$ (N) | $R^{6'}$ | phys. Konstante | eingesetzte Aminosäure |
|---|---|---|---|---|---|---|---|---|
| 100 | $CH_2=CH-CH_2-$ | H | H | $-CH-C_2H_5$<br>$CH_3$ | H | $-CH(CH_3)-$ 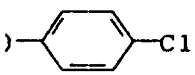 | mp: 178-180° C | Allyloxycarbonyl-L-Isoleucin |
| 101 | $(CH_3)_2CH-CH_2-$ | H | H | $-CH-C_2H_5$<br>$CH_3$ | H | $-CH(CH_3)-$ 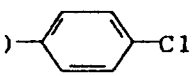 | mp: 142-144° C | i-Butyloxycarbonyl-L-Isoleucin |
| 102 | $CH_3-(CH_2)_3-$ | H | H | $-CH-C_2H_5$<br>$CH_3$ | H | $-CH(CH_3)-$ 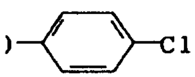 | mp: 158-161° C | n-Butyloxycarbonyl-L-Isoleucin |
| 103 | $CH_3-(CH_2)_2-$ | H | H | $-CH-C_2H_5$<br>$CH_3$ | H | $-CH(CH_3)-$ 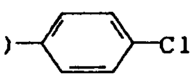 | mp: 167-171° C | n-Propyloxycarbonyl-L-Isoleucin |
| 104 | $CH_3-(CH_2)_5-$ | H | H | $-CH-C_2H_5$<br>$CH_3$ | H | $-CH(CH_3)-$ 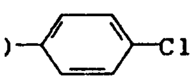 | mp: 152-154° C | n-Hexyloxycarbonyl-L-Isoleucin |
| 105 | $(CH_3)_3C-$ | H | $-CH_2-CH_2-CH_2-CH_2-CH_2-$ | | H | $-CH(CH_3)-$ 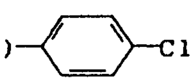 | mp: 198-199° C | BOC-(1-Aminocyclohexancarbonsäure) |

**Tabelle 1** Fortsetzung

| | | | | | N | | | |
|---|---|---|---|---|---|---|---|---|
| Nr. | $R^{1'}$ | $R^{2'}$ | $R^{3'}$ | $R^{4'}$ | $R^{5'}$ | $R^{6'}$ | phys. Konstante | eingesetzte Aminosäure |
| 106 | $(CH_3)_3C-$ | H | $-CH_2-CH_2-CH_2-CH_2-CH_2-$ | | H | $-CH(CH_3)-$ 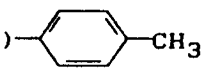 | mp: 185-186° C | BOC-(1-Amino-cyclohexan-carbonsäure) |
| 107 | $(CH_3)_3C-$ | H | $-CH_2-CH_2-CH_2-CH_2-CH_2-$ | | H | $-CH(CH_3)-$ 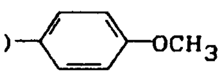 | mp: 176-177° C | BOC-(1-Amino-cyclohexan-carbonsäure) |
| 108 | $(CH_3)_3C-$ | H | $-CH_2-CH_2-CH_2-CH_2-$ | | H | $-CH(CH_3)-$ 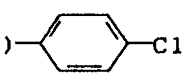 | mp: 193-196° C | BOC-(1-Amino-cyclopentancar-bonsäure) |
| 109 | $(CH_3)_3C-$ | H | $-CH_2-CH_2-CH_2-CH_2-$ | | H | $-CH(CH_3)-$ 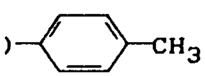 | mp: 172-175° C | BOC-(1-Amino-cyclopentancar-bonsäure) |
| 110 | $(CH_3)_3C-$ | H | $-CH_2-CH_2-CH_2-CH_2-$ | | H | $-CH(CH_3)-$ 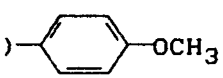 | mp: 180-182° C | BOC-(1-Amino-cyclopentancar-bonsäure) |
| 111 | $(CH_3)_3C-$ | H | H | $-CH(CH_3)-C_2H_5$ | H | $-CH(CH_3)-$ 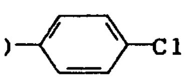 | mp: 145-147° C | BOC-D,L-Isoleucin |
| 112 | $(CH_3)_3C-$ | H | H | $-CH(CH_3)-C_2H_5$ | H | $-CH(CH_3)-$ 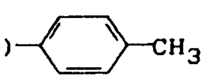 | mp: 145-147° C | BOC-D,L-Isoleucin |

Tabelle 1 Fortsetzung

| Nr. | $R^{1'}$ | $R^{2'}$ | $R^{3'}$ | $R^{4'}$ | N: $R^{5'}$ | N: $R^{6'}$ | phys. Konstante | eingesetzte Aminosäure |
|---|---|---|---|---|---|---|---|---|
| 113 | $(CH_3)_3C-$ | H | H | $-CH-C_2H_5$<br>$CH_3$ | H | $-CH(CH_3)-$ 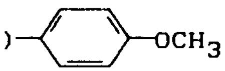 | mp: 140-142° C | BOC-D,L-Isoleucin |
| 114 | $(CH_3)_3C-$ | H | H | 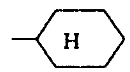 | H | $-CH(CH_3)-$ 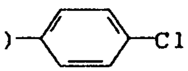 | mp: > 250° C | BOC-(α,(L)-Amino-cyclo-hexanessig-säure) |
| 115 | $(CH_3)_3C-$ | H | H | 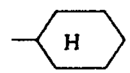 | H | $-CH(CH_3)-$ 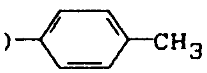 | mp: 135-138° C | BOC-(α,(L)-Amino-cyclo-hexanessig-säure) |
| 116 | $(CH_3)_3C-$ | H | H | 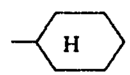 | H | $-CH(CH_3)-$ 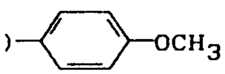 | mp: > 250° C | BOC-(α-(L)-Amino-cyclo-hexanessig-säure) |
| 117 | $(CH_3)_3C-$ | H | H | 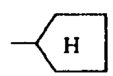 | H | $-CH(CH_3)-$ 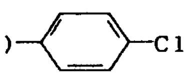 | mp: 169-172° C | BOC-(α-(L)-Amino-cyclo-pentanessig-säure) |
| 118 | $(CH_3)_3C-$ | H | H | 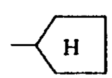 | H | $-CH(CH_3)-$ 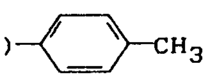 | mp: 162-169° C | BOC-(α-(L)-Amino-cyclo-pentanessig-säure) |
| 119 | $(CH_3)_3C-$ | H | H | 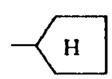 | H | $-CH(CH_3)-$ 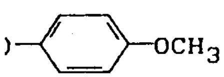 | mp: 150-160° C | BOC-(α-(L)-Amino-cyclo-pentanessig-säure) |

**Tabelle 1** Fortsetzung

| Nr. | $R^{1'}$ | $R^{2'}$ | $R^{3'}$ | $R^{4'}$ | 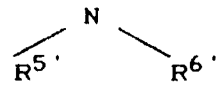 | phys. Konstante | eingesetzte Aminosäure |
|---|---|---|---|---|---|---|---|
| 120 | $(CH_3)_3C-$ | H | H | 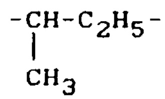 | H -CH(CH$_3$)- 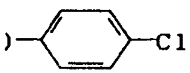 | mp: 136-137° C | BOC-L-Isoleucin |
| 121 | 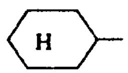 | H | H | $-CH(CH_3)_2$ | H -CH(CH$_3$)- 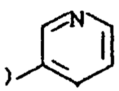 | mp: 108-111° C | Cyclohexyl-oxycarbonyl-L-Valin |
| 122 | 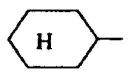 | H | H | $-CH(CH_3)_2$ | H -CH(CH$_3$)- 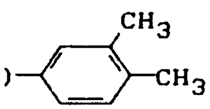 | mp: 168-174° C | Cyclohexyl-oxycarbonyl-L-Valin |
| 123 | 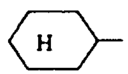 | H | H | $-CH(CH_3)_2$ | H -CH(CH$_3$)- 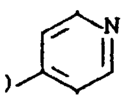 | mp: 124-129° C | Cyclohexyl-oxycarbonyl-L-Valin |
| 124 | 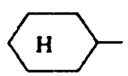 | H | H | $-CH(CH_3)_2$ | H -CH(CH$_3$)- 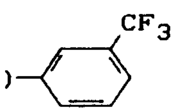 | mp: 120-128° C | Cyclohexyl-oxycarbonyl-L-Valin |
| 125 | 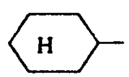 | H | H | $-CH(CH_3)_2$ | H -CH(CH$_3$)- 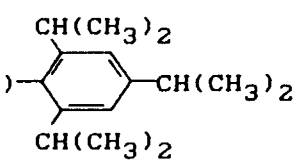 | mp: 140-142° C | Cyclohexyl-oxycarbonyl-L-Valin |

Tabelle 1 Fortsetzung

| Nr. | $R^{1'}$ | $R^{2'}$ | $R^{3'}$ | $R^{4'}$ | N $R^{5'}$ | N $R^{6'}$ | phys. Konstante | eingesetzte Aminosäure |
|---|---|---|---|---|---|---|---|---|
| 126 | $(CH_3)_3C-$ | H | H | $CH_2-CH(CH_3)_2$ | H | $-CH(CH_3)-$ 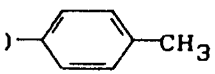 | mp: 110-111° C | BOC-L-Leucin |
| 127 | $(CH_3)_3C-$ | H | H | $-CH(CH_3)_2$ | H | $-CH(CH_3)-$ 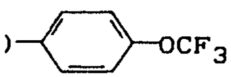 | mp: 79° C | BOC-L-Valin |
| 128 | $(CH_3)_3C-$ | H | H | $-CH(CH_3)_2$ | H | $-CH(CH_3)-$ 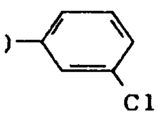 | mp: 114° C | BOC-L-Valin |
| 129 | $(CH_3)_3C-$ | H | H | $-CH(CH_3)_2$ | H | $-CH(CH_3)-$ 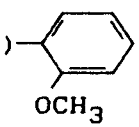 | mp: 114° C | BOC-L-Valin |
| 130 | $(CH_3)_3C-$ | H | H | $-CH(CH_3)_2$ | H | $-CH(CH_3)-$ 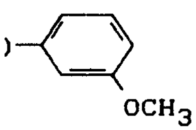 | mp: 93° C | BOC-L-Valin |
| 131 | $(CH_3)_3C-$ | H | H | $-CH(CH_3)_2$ | H | $-CH(CH_3)-$ 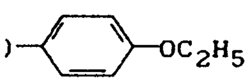 | mp: 138° C | BOC-L-Valin |
| 132 | $(CH_3)_3C-$ | H | H | $-CH(CH_3)_2$ | H | $-CH(CH_3)-$ 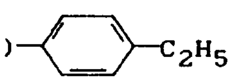 | mp: 112° C | BOC-L-Valin |

<u>Tabelle 1</u> Fortsetzung

| Nr. | $R^{1'}$ | $R^{2'}$ | $R^{3'}$ | $R^{4'}$ | $R^{5'}$ (N) | $R^{6'}$ | phys. Konstante | eingesetzte Aminosäure |
|---|---|---|---|---|---|---|---|---|
| 133 | $(CH_3)_3C-$ | H | H | $-CH(CH_3)_2$ | H | $-CH(CH_3)-$ 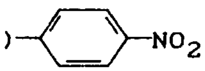 | mp: 130° C | BOC-L-Valin |
| 134 | $(CH_3)_3C-$ | H | H | $-CH(CH_3)_2$ | H | $-CH(CH_3)-$ 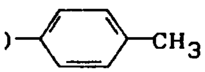 | mp: 124° C | BOC-L-Valin |
| 135 | $(CH_3)_3C-$ | H | H | $-CH(CH_3)_2$ | H | $-CH(C_2H_5)-$ 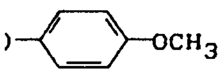 | mp: 138° C | BOC-L-Valin |
| 136 | $(CH_3)_3C-$ | H | H | $-CH(CH_3)_2$ | H | $-CH(CH_3)-CH_2-$ 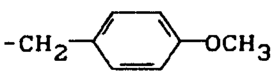 | mp: 63° C | BOC-L-Valin |
| 137 | $(CH_3)_3C-$ | H | H | $-CH(CH_3)_2$ | H | $-C(CH_3)-$<br>$CH_3$ 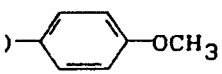 | mp: 94° C | BOC-L-Valin |
| 138 | $(CH_3)_3C-$ | H | H | $-CH-C_2H_5$ 2<br>$CH_3$ | H | $-CH(CH_3)-$ 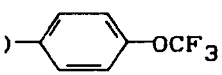 | | BOC-L-Isoleucin |

Tabelle 1 Fortsetzung

| Nr. | $R^{1'}$ | $R^{2'}$ | $R^{3'}$ | $R^{4'}$ | N<br>$R^{5'}$ | <br>$R^{6'}$ | phys. Konstante | eingesetzte Aminosäure |
|---|---|---|---|---|---|---|---|---|
| 139 | $(CH_3)_3C-$ | H | H | $-CH-C_2H_5$<br>$CH_3$ | H | $-CH(CH_3)-$ 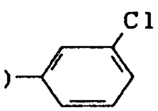 | mp: 96° C | BOC-L-Iso-leucin |
| 140 | $(CH_3)_3C-$ | H | H | $-CH-C_2H_5$<br>$CH_3$ | H | $-CH(CH_3)-$ 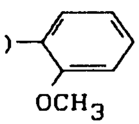 | mp: 123° C | BOC-L-Iso-leucin |
| 141 | $(CH_3)_3C-$ | H | H | $-CH-C_2H_5$<br>$CH_3$ | H | $-CH(CH_3)-$ 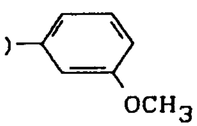 | mp: 105° C | BOC-L-Iso-leucin |
| 142 | $(CH_3)_3C-$ | H | H | $-CH-C_2H_5$<br>$CH_3$ | H | $-CH(CH_3)-$ 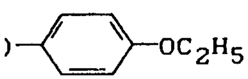 | mp: 130° C | BOC-L-Iso-leucin |
| 143 | $(CH_3)_3C-$ | H | H | $-CH-C_2H_5$<br>$CH_3$ | H | $-CH(CH_3)-$ 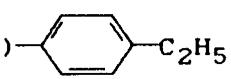 | mp: 82° C | BOC-L-Iso-leucin |
| 144 | $(CH_3)_3C-$ | H | H | $-CH-C_2H_5$<br>$CH_3$ | H | $-CH(C_2H_5)-$ 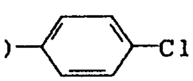 | mp: 134° C | BOC-L-Iso-leucin |

<u>Tabelle 1</u> Fortsetzung

| Nr. | R$^{1'}$ | R$^{2'}$ | R$^{3'}$ | R$^{4'}$ | N<br>R$^{5'}$ | R$^{6'}$ | phys. Konstante | eingesetzte Aminosäure |
|---|---|---|---|---|---|---|---|---|
| 145 | $(CH_3)_3C-$ | H | H | $-CH-C_2H_5$<br>$CH_3$ | H | 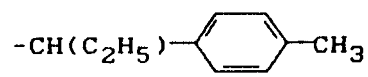 | mp: 121° C | BOC-L-Iso-leucin |
| 146 | $(CH_3)_3C-$ | H | H | $-CH-C_2H_5$<br>$CH_3$ | H | 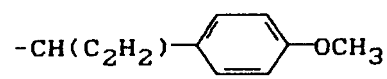 | mp: 135° C | BOC-L-Iso-leucin |
| 147 | $(CH_3)_3C-$ | H | H | $-CH-C_2H_5$<br>$CH_3$ | H | 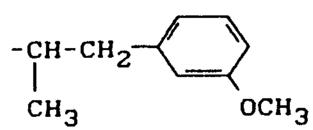 | mp: 116° C | BOC-L-Iso-leucin |
| 148 | $(CH_3)_3C-$ | H | H | $-CH-C_2H_5$<br>$CH_3$ | H | 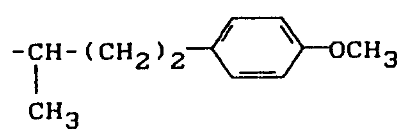 | mp: 111° C | BOC-L-Iso-leucin |

Beispiel A

Plasmopara-Test (Reben) / protektiv

Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Die Wirksamkeit zeigen in diesem Test z.B. die Verbindungen gemäß folgender Herstellungsbeispiele:

Tabelle A

Plasmopara-Test (Reben) / protektiv

| Wirkstoff | Wirkungsgrad in % der unbehandelten Kontrolle bei einer Wirkstoffkonzentration von 5 ppm |
|---|---|
| 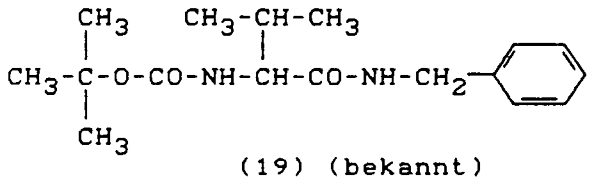 (19) (bekannt) | 29 |
| 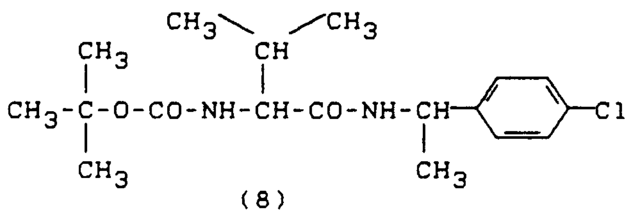 (8) | 100 |
| 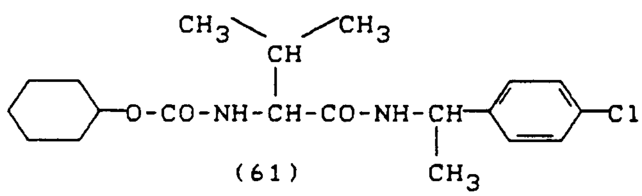 (61) | 94 |
| 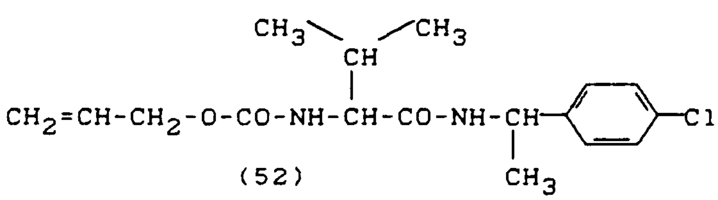 (52) | 94 |

Tabelle A

Plasmopara-Test (Reben) / protektiv

| Wirkstoff | Wirkungsgrad in % der unbehandelten Kontrolle bei einer Wirkstoffkonzentration von 5 ppm |
|---|---|
| 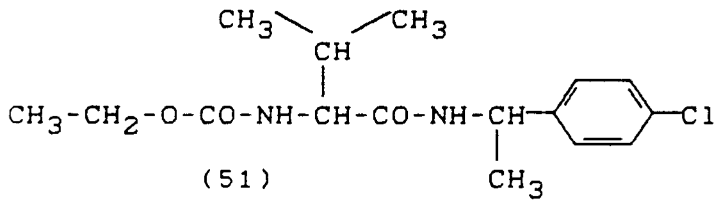 (51) | 94 |
| 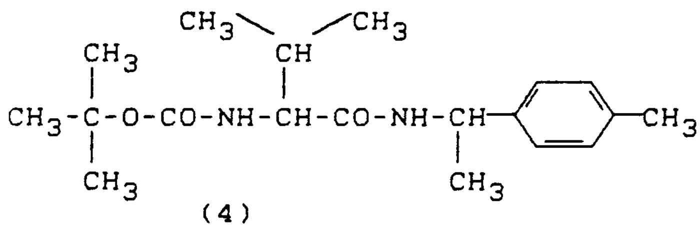 (4) | 100 |
| 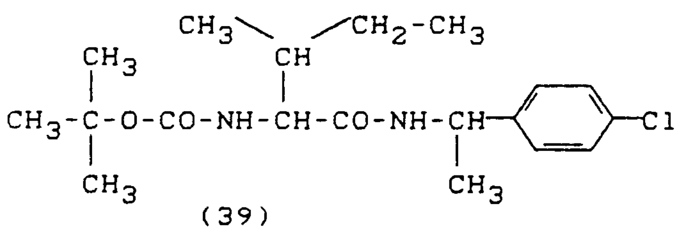 (39) | 91 |

Beispiel B

Phytophthora-Test (Tomate) /protektiv

Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20 °C aufgestellt.

3 Tage nach der Inokulation erfolgt die Auswertung.

Z. B. zeigen die Verbindungen gemäß der Herstellungsbeispiele (7), (40) und (41) bei einer beispielhaften Wirkstoffkonzentration von 5 ppm einen ausgezeichneten Wirkungsgrad.

Tabelle B

Phytophthora-Test (Tomate) /protektiv

| Wirkstoff | Wirkungsgrad in % der unbehandelten Kontrolle bei einer Wirkstoffkonzentration von 5 ppm |
|---|---|
| Bekannt: | |
| 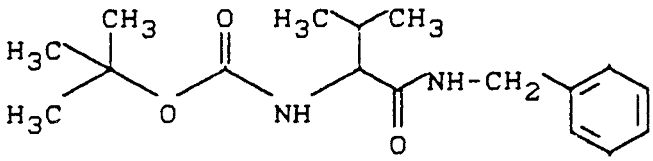 | 0 |
| Erfindungsgemäß: | |
| 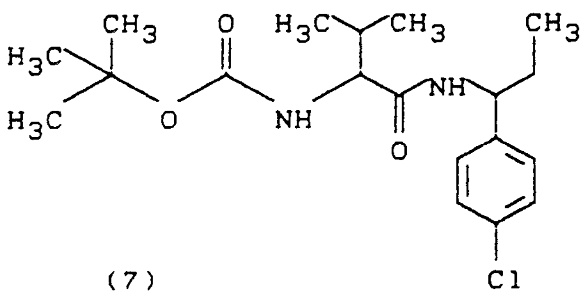 (7) | 85 |
| 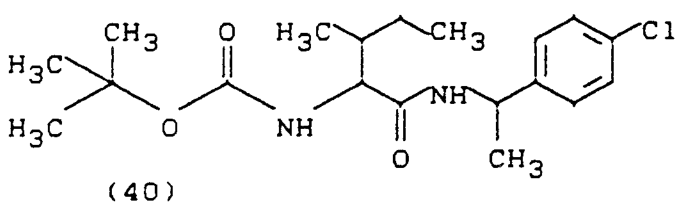 (40) | 80 |
| 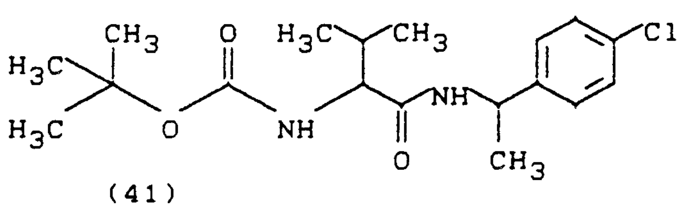 (41) | 80 |

## Patentansprüche

## Patentansprüche für folgende Vertragsstaaten : BE, CH, DE, FR, GB, GR, IT, LI, NL

**1.** Verwendung von substituierten Aminosäureamid-Derivaten der allgemeinen Formel (I)

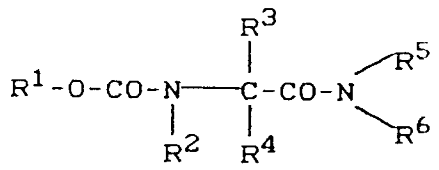

(I)

in welcher

- $R^1$ für Alkyl, Alkenyl, Alkinyl, Halogenalkyl, Halogenalkenyl, Halogenalkinyl, Cycloalkyl oder Cycloalkenyl steht,
- $R^2$, $R^3$, $R^4$ und $R^5$ gleich oder verschieden sind und für Wasserstoff, Cycloalkyl oder Alkyl stehen oder
- $R^3$ und $R^4$ zusammen mit dem Kohlenstoffatom, an das sie gebunden sind, einen Cycloalkylring bilden und
- $R^6$ für Wasserstoff, Alkyl, Alkenyl, Alkinyl, Cyanoalkyl, unsubstituiertes oder substituiertes Phenylalkyl, unsubstituiertes oder substituiertes Phenyl, unsubstituiertes oder substituiertes Cycloalkyl oder für unsubstituiertes oder substituiertes Heterocyclyl oder Heterocyclylalkyl steht,

oder

- $R^5$ und $R^6$ gemeinsam mit dem Stickstoff-Atom, an das sie gebunden sind, für einen Heterocyclyl-Rest stehen, der weitere Heteroatome enthalten kann,

zur Bekämpfung von phytophatogenen Pilzen.

**2.** Substituierte Aminosäureamid-Derivate der allgemeinen Formel (Ia)

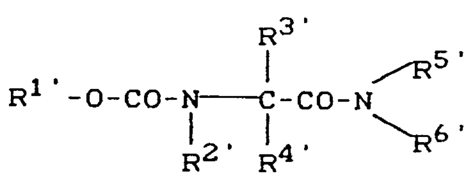

(Ia)

in welcher

- $R^{1'}$ für Alkyl, Alkenyl, Alkinyl, Halogenalkyl, Halogenalkenyl, Halogenalkinyl, Cycloalkyl oder Cycloalkenyl steht,
- $R^{2'}$ und $R^{5'}$ gleich oder verschieden sind und für Wasserstoff oder Alkyl stehen und
- $R^{3'}$ für Wasserstoff steht und
- $R^{4'}$ für i-Propyl, i-Butyl, s-Butyl, 3-Pentyl oder Cycloalkyl steht oder
- $R^{3'}$ und $R^{4'}$ zusammen mit dem Kohlenstoffatom, an das sie gebunden sind, einen Cycloalkylring bilden und
- $R^{6'}$ für Wasserstoff, Alkyl, Alkenyl, Alkinyl, Cyanoalkyl, unsubstituiertes oder substituiertes Phenylalkyl, unsubstituiertes oder substituiertes Phenyl, unsubstituiertes oder substituiertes Cycloalkyl, oder für unsubstituiertes oder substituiertes Heterocyclyl oder Heterocyclylalkyl steht,

oder

- $R^{5'}$ und $R^{6'}$ gemeinsam mit dem Stickstoff-Atom, an das sie gebunden sind, für einen Heterocyclyl-Rest stehen, der weitere Heteroatome enthalten kann, ausgenommen Verbindungen, in denen

a)

- $R^{4'}$ für i-Butyl steht und
- $R^{1'}$ für Methyl oder t-Butyl steht und

$R^{2'}$ für Wasserstoff steht und
$R^{5'}$ für Wasserstoff oder Methyl steht und
$R^{6'}$ für Wasserstoff, Methyl, Ethyl, Phenyl, Benzyl, 2-Phenethyl oder Pyridylmethyl steht oder
$R^{5'}$ und $R^{6'}$ zusammen mit dem Stickstoffatom, an das sie gebunden sind einen Piperidinring bilden und

b)
$R^{4'}$ für s-Butyl steht und
$R^{1'}$ für t-Butyl steht und
$R^{2'}$ für Wasserstoff steht und
$R^{5'}$ für Wasserstoff steht und
$R^{6'}$ für Wasserstoff oder Benzyl steht und

c)
$R^{4'}$ für i-Propyl steht und
$R^{1'}$ für Methyl, Ethyl oder t-Butyl steht und
$R^{2'}$ für Wasserstoff steht und
$R^{5'}$ für Wasserstoff steht und
$R^{6'}$ für Wasserstoff, Methyl, Phenyl oder Benzyl steht oder
$R^{5'}$ und $R^{6'}$ zusammen mit dem Stickstoffatom, an das sie gebunden sind einen Pyrrolidinring bilden.

**3.** Substituierte Aminosäureamid-Derivate der Formel (Ia) gemäß Anspruch 2, in welcher
$R^{1'}$ für geradkettiges oder verzweigtes Alkyl mit 1 bis 6 Kohlenstoffatomen, geradkettiges oder verzweigtes Alkenyl oder Alkinyl mit 2 bis 6 Kohlenstoffatomen, geradkettiges oder verzweigtes Halogenalkyl mit 1 bis 6 Kohlenstoffatomen und 1 bis 9 gleichen oder verschiedenen Halogenatomen, geradkettiges oder verzweites Halogenalkenyl oder Halogenalkinyl mit 2 bis 6 Kohlenstoffatomen und 1 bis 9 gleichen oder verschiedenen Halogenatomen oder unsubstituiertes oder substituiertes Cycloalkyl oder Cycloalkenyl mit 3 bis 7 Kohlenstoffatomen steht und
$R^{2'}$ und $R^{5'}$ gleich oder verschieden sind und für Wasserstoff oder Alkyl mit 1 bis 6 Kohlenstoffatomen stehen und
$R^{3'}$ für Wasserstoff steht und
$R^{4'}$ für i-Propyl, i-Butyl, s-Butyl oder 3-Pentyl steht oder
$R^{3'}$ und $R^{4'}$ zusammen mit dem Kohlenstoffatom, an das sie gebunden sind, einen Cycloalkylring mit 3 bis 7 Kohlenstoffatomen bilden und
$R^{6'}$ für Wasserstoff, geradkettiges oder verzweigtes Alkyl mit 1 bis 6 Kohlenstoffatomen, für Alkenyl oder Alkinyl mit jeweils 2 bis 6 Kohlenstoffatomen, für Cyanalkyl mit 1 bis 4 Kohlenstoffatomen im Alkylteil, unsubstituiertes oder im Phenylteil einfach bis dreifach, gleich oder verschieden substituiertes Phenylalkyl mit 1 bis 4 Kohlenstoffatomen im Alkylteil oder unsubstituiertes oder einfach bis dreifach, gleich oder verschieden substituiertes Phenyl steht, wobei als Substituenten jeweils in Frage kommen: Halogen; Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 gleichen oder verschiedenen Halogenatomen; Hydroxy; Cyano, Nitro, Amino, Alkylamino, Dialkylamino mit jeweils 1 bis 4 Kohlenstoffatomen und Carboxyl; für unsubstituiertes oder gleich oder verschieden substituiertes Cycloalkyl mit 3 bis 7 Kohlenstoffatomen, wobei als Substituenten Alkyl und Alkoxy mit jeweils 1 bis 4 Kohlenstoffatomen in Frage kommen, oder für unsubstituiertes oder substituiertes Heterocyclyl oder Heterocyclylalkyl mit 2 bis 6 Kohlenstoffatomen im Heterocyclylteil und einem oder zwei Heteroatomen und gegebenenfalls 1 bis 4 Kohlenstoffatomen im Alkylteil steht, wobei als Substituenten die oben genannten Phenylsubstituenten in Frage kommen,

oder
$R^{5'}$ und $R^{6'}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann, ausgenommen Verbindungen, in denen

a)
$R^{4'}$ für i-Butyl steht und

$R^{1'}$ für Methyl oder t-Butyl steht und
$R^{2'}$ für Wasserstoff steht und
$R^{5'}$ für Wasserstoff oder Methyl steht und
$R^{6'}$ für Wasserstoff, Methyl, Ethyl, Phenyl, Benzyl, 2-Phenethyl oder Pyridylmethyl steht oder
$R^{5'}$ und $R^{6'}$ zusammen mit dem Stickstoffatom, an das sie gebunden sind einen Piperidinring bilden und

b)
$R^{4'}$ für s-Butyl steht und
$R^{1'}$ für t-Butyl steht und
$R^{2'}$ für Wasserstoff steht und
$R^{5'}$ für Wasserstoff steht und
$R^{6'}$ für Wasserstoff oder Benzyl steht und

c)
$R^{4'}$ für i-Propyl steht und
$R^{1'}$ für Methyl, Ethyl oder t-Butyl steht und
$R^{2'}$ für Wasserstoff steht und
$R^{5'}$ für Wasserstoff steht und
$R^{6'}$ für Wasserstoff, Methyl, Phenyl oder Benzyl steht oder
$R^{5'}$ und $R^{6'}$ zusammen mit dem Stickstoffatom, an das sie gebunden sind einen Pyrrolidinring bilden.

**4.** Substituierte Aminosäureamid-Derivate der Formel (Ia) gemäß Anspruch 2, in welcher
$R^{1'}$ für geradkettiges oder verzweigtes Alkyl mit 1 bis 4 Kohlenstoffatomen, geradkettiges oder verzweigtes Alkenyl oder Alkinyl mit 2 bis 4 Kohlenstoffatomen, geradkettiges oder verzweigtes Halogenalkyl mit 1 bis 4 Kohlenstoffatomen und 1 bis 5 gleichen oder verschiedenen Halogenatomen, geradkettiges oder verzweigtes Halogenalkenyl oder Halogenalkinyl mit 2 bis 4 Kohlenstoffatomen und 1 bis 5 gleichen oder verschiedenen Halogenatomen oder unsubstituiertes oder substituiertes Cycloalkyl oder Cycloalkenyl mit 3 bis 6 Kohlenstoffatomen steht und
$R^{2'}$ und $R^{5'}$ gleich oder verschieden sind und für Wasserstoff, Methyl oder Ethyl stehen und
$R^{3'}$ für Wasserstoff steht und
$R^{4'}$ für i-Propyl, i-Butyl, s-Butyl oder 3-Pentyl steht oder
$R^{3'}$ und $R^{4'}$ zusammen mit dem Kohlenstoffatom, an das sie gebunden sind, einen Cycloalkylring mit 3 bis 6 Kohlenstoffatomen bilden und
$R^{6'}$ für Wasserstoff, Methyl, Ethyl, Allyl, Propargyl, für Cyanoalkyl, mit 1 oder 2 Kohlenstoffatomen im Alkylteil; ferner für unsubstituiertes oder im Phenylteil einfach oder zweifach, gleich oder verschieden substituiertes Phenylalkyl mit 1 bis 4 Kohlenstoffatomen im Alkylteil, für unsubstituiertes oder einfach oder zweifach, gleich oder verschieden substituiertes Phenyl steht, wobei als Substituenten jeweils in Frage kommen: Fluor, Chlor und Brom, Methyl, Ethyl, n- und i-Propyl, n-, i-, s- und t-Butyl, Methoxy, Ethoxy, n- und i-Propyloxy, n-, i-, s- und t-Butyloxy, Methylthio, Ethylthio, n- und i-Propylthio, n-, i-, s- und t-Butylthio, Trifluormethyl, Trifluormethoxy, Trifluormethylthio, Hydroxy, Nitro, Cyano, Amino, Methylamino, Ethylamino, Dimethylamino, Diethylamino und Carboxy; für jeweils unsubstituiertes oder einfach oder zweifach, gleich oder verschieden substituiertes Cycloalkyl mit 3 bis 6 Kohlenstoffatomen, wobei als Substituenten Methyl, Ethyl, n- und i-Propyl, n-, i-, s- und t-Butyl, Methoxy, Ethoxy, n- und i-Propyloxy, n-, i-, s- und t-Butyloxy in Frage kommen oder für unsubstituiertes oder einfach oder zweifach, gleich oder verschieden substituiertes Heterocyclyl oder Heterocyclylalkyl mit 4 oder 5 Kohlenstoffatom im Heterocyclylteil und einem oder zwei Stickstoffatomenen und gegebenenfalls 1 bis 4 Kohlenstoffatomen im Alkylteil steht, wobei als Substituenten die obengenannten Phenylsubstituenten in Frage kommen; oder
$R^{5'}$ und $R^{6'}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann, ausgenommen Verbindungen, in denen

a)
$R^{4'}$ für i-Butyl steht und

$R^{1'}$ für Methyl oder t-Butyl steht und
$R^{2'}$ für Wasserstoff steht und
$R^{5'}$ für Wasserstoff oder Methyl steht und
$R^{6'}$ für Wasserstoff, Methyl, Ethyl, Phenyl, Benzyl, 2-Phenethyl oder Pyridylmethyl steht oder
$R^{5'}$ und $R^{6'}$ zusammen mit dem Stickstoffatom, an das sie gebunden sind einen Piperidinring bilden und

b)
$R^{4'}$ für s-Butyl steht und
$R^{1'}$ für t-Butyl steht und
$R^{2'}$ für Wasserstoff steht und
$R^{5'}$ für Wasserstoff steht und
$R^{6'}$ für Wasserstoff oder Benzyl steht und

c)
$R^{4'}$ für i-Propyl steht und
$R^{1'}$ für Methyl, Ethyl oder t-Butyl steht und
$R^{2'}$ für Wasserstoff steht und
$R^{5'}$ für Wasserstoff steht und
$R^{6'}$ für Wasserstoff, Methyl, Phenyl oder Benzyl steht oder
$R^{5'}$ und $R^{6'}$ zusammen mit dem Stickstoffatom, an das sie gebunden sind einen Pyrrolidinring bilden.

**5.** Substituierte Aminosäureamid-Derivate der Formel (Ia) gemäß Anspruch 2, in welcher
$R^{1'}$ für geradkettiges oder verzweigtes Alkyl mit 1 bis 4 Kohlenstoffatomen, geradkettiges oder verzweigtes Alkenyl oder Alkinyl mit 2 bis 4 Kohlenstoffatomen, geradkettiges oder verzweigtes Halogenalkyl mit 1 bis 4 Kohlenstoffatomen und 1 bis 5 gleichen oder verschiedenen Halogenatomen, geradkettiges oder verzweigtes Halogenalkenyl oder Halogenalkinyl mit 2 bis 4 Kohlenstoffatomen und 1 bis 5 gleichen oder verschiedenen Halogenatomen oder unsubstituiertes oder substituiertes Cycloalkyl oder Cycloalkenyl mit 3 bis 6 Kohlenstoffatomen steht und
$R^{2'}$, $R^{3'}$ und $R^{5'}$ gleich oder verschieden sind und für Wasserstoff stehen und
$R^{4'}$ für i-Propyl, i-Butyl, s-Butyl oder 3-Pentyl steht und
$R^{6'}$ für unsubstituiertes oder im Phenylteil einfach oder zweifach, gleich oder verschieden substituiertes Phenylalkyl mit 1 bis 4 Kohlenstoffatomen im Alkylteil steht, wobei als Substituenten jeweils in Frage kommen: Fluor, Chlor und Brom, Methyl, Ethyl, n- und i-Propyl, n-, i-, s- und t-Butyl, Methoxy, Ethoxy, n- und i-Propyloxy, n-, i-, s- und t-Butyloxy, Methylthio, Ethylthio, n-und i-Propylthio, n-, i-, s- und t-Butylthio, Trifluormethyl, Trifluormethoxy, Trifluormethylthio, Cyano, Dimethylamino, Diethylamino und Carboxy; oder für unsubstituiertes oder einfach oder zweifach substituiertes Heterocyclylalkyl mit 4 oder 5 Kohlenstoffatom im Heterocyclylteil und einem oder zwei Stickstoffatomenen und gegebenenfalls 1 bis 4 Kohlenstoffatomen im Alkylteil steht, wobei als Substituenten die obengenannten Phenylsubstituenten in Frage kommen, ausgenommen Verbindungen, in denen

a)
$R^{4'}$ für i-Butyl steht und
$R^{1'}$ für Methyl oder t-Butyl steht und
$R^{2'}$ für Wasserstoff steht und
$R^{5'}$ für Wasserstoff steht und
$R^{6'}$ für Benzyl, 2-Phenethyl oder Pyridylmethyl steht und

b)
$R^{4'}$ für s-Butyl steht und
$R^{1'}$ für t-Butyl steht und
$R^{2'}$ für Wasserstoff steht und
$R^{5'}$ für Wasserstoff steht und
$R^{6'}$ für Benzyl steht und

c)
$R^{4'}$ für i-Propyl steht und

$R^{1'}$ für Methyl, Ethyl oder t-Butyl steht und
$R^{2'}$ für Wasserstoff steht und
$R^{5'}$ für Wasserstoff steht und
$R^{6'}$ für Benzyl steht.

**6.** Substituierte Aminosäureamid-Derivate der Formel (Ia) gemäß Anspruch 2, in welcher

$R^{1'}$ für Methyl, Ethyl, n- und i-Propyl, n-, i-, s- und t-Butyl, Fluormethyl, Fluorethyl, Fluorpropyl, Chlorpropyl, Fluorbutyl, Chlorbutyl, Difluormethyl, Difluorpropyl, Dichlorpropyl, Difluorbutyl, Dichlorbutyl, Trifluormethyl, Trichlormethyl, Trifluorethyl, Trichlorethyl, Trifluorpropyl, Trichlorpropyl, Trifluorbutyl, Trichlorbutyl, Allyl, Butenyl, Propargyl, Butinyl, Fluor- oder Chlor-allyl, butenyl, -propargyl, -butinyl, Cyclopropyl, Cyclopentyl, Cyclohexyl, Cyclopentenyl oder Cyclohexenyl steht und
$R^{2'}$, $R^{3'}$ und $R^{5'}$ für Wasserstoff stehen und
$R^{4'}$ für i-Propyl, i-Butyl, s-Butyl oder 3-Pentyl steht und
$R^{6'}$ für unsubstituiertes oder im Phenylteil einfach oder zweifach, gleich oder verschieden substituiertes Benzyl, 1-Phenethyl, 2-Phenethyl oder 1-Phenylpropyl, 2-Phenylpropyl, 3-Phenylpropyl, 1-Phenylisopropyl, 2-Phenylisopropyl oder 1-Phenyl-n-, -i- oder für steht, wobei als Substituenten jeweils in Frage kommen: Fluor, Chlor, Brom, Methyl, Ethyl, n- und i-Propyl, n-, i-, s- und t-Butyl, Methoxy, Ethoxy, n-und i-Propyloxy, n-, i-, s- und t-Butyloxy, Methylthio, Ethylthio, n-und i-Propylthio, n-, i-, s- und t-Butylthio, Trifluormethyl, Trifluormethoxy, Trifluormethylthio, Cyano, Dimethylamino und Diethylamino; für unsubstituiertes oder ein oder zweifach, gleich oder verschieden substituiertes Pyridylmethyl oder 1-bzw. 2-Pyridylethyl steht, wobei als Substituenten die oben genannten Phenylsubstituenten in Frage kommen, ausgenommen Verbindungen, in denen

a)
$R^{4'}$ für i-Butyl steht und
$R^{1'}$ für Methyl oder t-Butyl steht und
$R^{2'}$ für Wasserstoff steht und
$R^{5'}$ für Wasserstoff steht und
$R^{6'}$ für Benzyl, 2-Phenethyl oder Pyridylmethyl steht und

b)
$R^{4'}$ für s-Butyl steht und
$R^{1'}$ für t-Butyl steht und
$R^{2'}$ für Wasserstoff steht und
$R^{5'}$ für Wasserstoff steht und
$R^{6'}$ für Benzyl steht und

c)
$R^{4'}$ für i-Propyl steht und
$R^{1'}$ für Methyl, Ethyl oder t-Butyl steht und
$R^{2'}$ für Wasserstoff steht und
$R^{5'}$ für Wasserstoff steht und
$R^{6'}$ für Benzyl steht.

**7.** Verfahren zur Herstellung von substituierten Aminosäureamid-Derivaten der allgemeinen Formel (Ia)

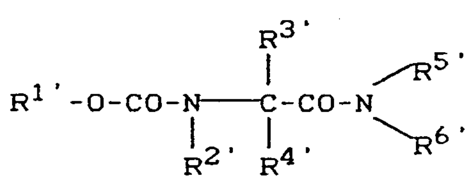

(Ia)

in welcher

$R^{1'}$ für Alkyl, Alkenyl, Alkinyl, Halogenalkyl, Halogenalkenyl, Halogenalkinyl, Cycloalkyl

oder Cycloalkenyl steht,

$R^{2'}$ und $R^{5'}$ gleich oder verschieden sind und für Wasserstoff oder Alkyl stehen und

$R^{3'}$ für Wasserstoff steht und

$R^{4'}$ für i-Propyl, i-Butyl, s-Butyl, 3-Pentyl oder Cycloalkyl steht oder

$R^{3'}$ und $R^{4'}$ zusammen mit dem Kohlenstoffatom, an das sie gebunden sind, einen Cycloalkylring bilden und

$R^{6'}$ für Wasserstoff, Alkyl, Alkenyl, Alkinyl, Cyanoalkyl, unsubstituiertes oder substituiertes Phenylalkyl, unsubstituiertes oder substituiertes Phenyl, unsubstituiertes oder substituiertes Cycloalkyl oder für unsubstituiertes oder substituiertes Heterocyclyl oder Heterocyclylalkyl steht,

oder

$R^{5'}$ und $R^{6'}$ gemeinsam mit dem Stickstoff-Atom, an das sie gebunden sind, für einen Heterocyclyl-Rest stehen, der weitere Heteroatome enthalten kann, ausgenommen Verbindungen, in denen

a)

$R^{4'}$ für i-Butyl steht und

$R^{1'}$ für Methyl oder t-Butyl steht und

$R^{2'}$ für Wasserstoff steht und

$R^{5'}$ für Wasserstoff oder Methyl steht und

$R^{6'}$ für Wasserstoff, Methyl, Ethyl, Phenyl, Benzyl, 2-Phenethyl oder Pyridylmethyl steht oder

$R^{5'}$ und $R^{6'}$ zusammen mit dem Stickstoffatom, an das sie gebunden sind einen Piperidinring bilden und

b)

$R^{4'}$ für s-Butyl steht und

$R^{1'}$ für t-Butyl steht und

$R^{2'}$ für Wasserstoff steht und

$R^{5'}$ für Wasserstoff steht und

$R^{6'}$ für Wasserstoff oder Benzyl steht und

c)

$R^{4'}$ für i-Propyl steht und

$R^{1'}$ für Methyl, Ethyl oder t-Butyl steht und

$R^{2'}$ für Wasserstoff steht und

$R^{5'}$ für Wasserstoff steht und

$R^{6'}$ für Wasserstoff, Methyl, Phenyl oder Benzyl steht oder

$R^{5'}$ und $R^{6'}$ zusammen mit dem Stickstoffatom, an das sie gebunden sind einen Pyrrolidinring bilden,

dadurch gekennzeichnet, daß man
eine substituierte Aminosäure der Formel (II)

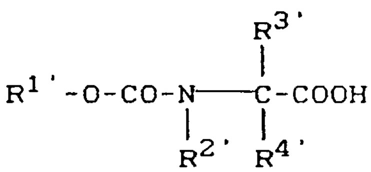

(II)

in welcher

$R^{1'}$, $R^{2'}$, $R^{3'}$ und $R^{4'}$ die oben angegebene Bedeutung haben, bzw. deren carboxy-aktivierte Derivate,

gegebenenfalls in Gegenwart eines Katalysators, gegebenenfalls in Gegenwart eines Säurebindemittels und gegebenenfalls in Gegenwart eines Verdünnungsmittels
mit einem Amin der Formel (III)

$HNR^{5'}R^{6'}$ (III)

in welcher

$R^{5'}$ und $R^{6'}$ die oben angegebene Bedeutung haben,

umsetzt.

**8.** Schädlingsbekämpfungsmittel, gekennzeichnet durch einen Gehalt an mindestens einem Aminosäureamid-Derivat der Formel (I) oder (Ia) nach den Ansprüchen 1 bis 7.

**9.** Verfahren zur Bekämpfung von phytophatogenen Pilzen, dadurch gekennzeichnet, daß man Aminosäureamid-Derivate der Formel (I) oder (Ia) nach den Ansprüchen 1 bis 7 auf Pilze und/oder ihren Lebensraum einwirken läßt.

**10.** Verfahren zur Herstellung von Schädlingsbekämpfungsmitteln, dadurch gekennzeichnet, daß man Aminosäureamid-Derivate der Formel (I) oder (Ia) nach den Ansprüchen 1 bis 7 mit Streckmitteln und/oder oberflächenaktiven Mitteln vermischt.

**Patentansprüche für folgenden Vertragsstaat : ES**

**1.** Verwendung von substituierten Aminosäureamid-Derivaten der allgemeinen Formel (I)

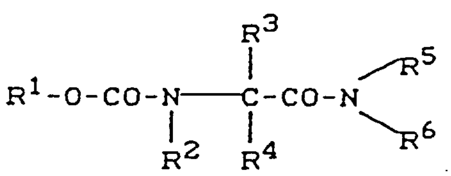

(I)

in welcher

$R^1$ für Alkyl, Alkenyl, Alkinyl, Halogenalkyl, Halogenalkenyl, Halogenalkinyl, Cycloalkyl oder Cycloalkenyl steht,

$R^2$, $R^3$, $R^4$ und $R^5$ gleich oder verschieden sind und für Wasserstoff, Cycloalkyl oder Alkyl stehen oder

$R^3$ und $R^4$ zusammen mit dem Kohlenstoffatom, an das sie gebunden sind, einen Cycloalkylring bilden und

$R^6$ für Wasserstoff, Alkyl, Alkenyl, Alkinyl, Cyanoalkyl, unsubstituiertes oder substituiertes Phenylalkyl, unsubstituiertes oder substituiertes Phenyl, unsubstituiertes oder substituiertes Cycloalkyl oder für unsubstituiertes oder substituiertes Heterocyclyl oder Heterocyclylalkyl steht,

oder

$R^5$ und $R^6$ gemeinsam mit dem Stickstoff-Atom, an das sie gebunden sind, für einen Heterocyclyl-Rest stehen, der weitere Heteroatome enthalten kann,

zur Bekämpfung von phytophatogenen Pilzen.

**2.** Verfahren zur Herstellung von substituierten Aminosäureamid-Derivaten der allgemeinen Formel (Ia)

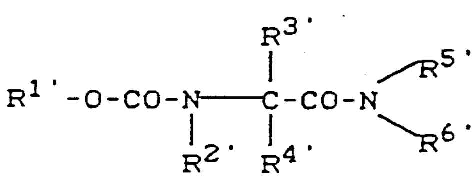

(Ia)

in welcher

$R^{1'}$ für Alkyl, Alkenyl, Alkinyl, Halogenalkyl, Halogenalkenyl, Halogenalkinyl, Cycloalkyl oder Cycloalkenyl steht,

$R^{2'}$ und $R^{5'}$ gleich oder verschieden sind und für Wasserstoff oder Alkyl stehen und

$R^{3'}$ für Wasserstoff steht und
$R^{4'}$ für i-Propyl, i-Butyl, s-Butyl, 3-Pentyl oder Cycloalkyl steht oder
$R^{3'}$ und $R^{4'}$ zusammen mit dem Kohlenstoffatom, an das sie gebunden sind, einen Cycloalkylring bilden und
$R^{6'}$ für Wasserstoff, Alkyl, Alkenyl, Alkinyl, Cyanoalkyl, unsubstituiertes oder substituiertes Phenylalkyl, unsubstituiertes oder substituiertes Phenyl, unsubstituiertes oder substituiertes Cycloalkyl oder für unsubstituiertes oder substituiertes Heterocyclyl oder Heterocyclylalkyl steht,

oder
$R^{5'}$ und $R^{6'}$ gemeinsam mit dem Stickstoff-Atom, an das sie gebunden sind, für einen Heterocyclyl-Rest stehen, der weitere Heteroatome enthalten kann, ausgenommen Verbindungen, in denen

a)
$R^{4'}$ für i-Butyl steht und
$R^{1'}$ für Methyl oder t-Butyl steht und
$R^{2'}$ für Wasserstoff steht und
$R^{5'}$ für Wasserstoff oder Methyl steht und
$R^{6'}$ für Wasserstoff, Methyl, Ethyl, Phenyl, Benzyl, 2-Phenethyl oder Pyridylmethyl steht oder
$R^{5'}$ und $R^{6'}$ zusammen mit dem Stickstoffatom, an das sie gebunden sind einen Piperidinring bilden und

b)
$R^{4'}$ für s-Butyl steht und
$R^{1'}$ für t-Butyl steht und
$R^{2'}$ für Wasserstoff steht und
$R^{5'}$ für Wasserstoff steht und
$R^{6'}$ für Wasserstoff oder Benzyl steht und

c)
$R^{4'}$ für i-Propyl steht und
$R^{1'}$ für Methyl, Ethyl oder t-Butyl steht und
$R^{2'}$ für Wasserstoff steht und
$R^{5'}$ für Wasserstoff steht und
$R^{6'}$ für Wasserstoff, Methyl, Phenyl oder Benzyl steht oder
$R^{5'}$ und $R^{6'}$ zusammen mit dem Stickstoffatom, an das sie gebunden sind einen Pyrrolidinring bilden,

dadurch gekennzeichnet, daß man
eine substituierte Aminosäure der Formel (II)

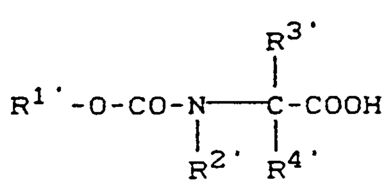

(II)

in welcher
$R^{1'}$, $R^{2'}$, $R^{3'}$ und $R^{4'}$ die oben angegebene Bedeutung haben, bzw. deren carboxy-aktivierte Derivate,

gegebenenfalls in Gegenwart eines Katalysators, gegebenenfalls in Gegenwart eines Säurebindemittels und gegebenenfalls in Gegenwart eines Verdünnungsmittels
mit einem Amin der Formel (III)

$HNR^{5'}R^{6'}$ (III)

in welcher
$R^{5'}$ und $R^{6'}$ die oben angegebene Bedeutung haben,
umsetzt.

**3.** Verfahren gemäß Anspruch 2, in welcher

$R^{1'}$ für geradkettiges oder verzweigtes Alkyl mit 1 bis 6 Kohlenstoffatomen, geradkettiges oder verzweigtes Alkenyl oder Alkinyl mit 2 bis 6 Kohlenstoffatomen, geradkettiges oder verzweigtes Halogenalkyl mit 1 bis 6 Kohlenstoffatomen und 1 bis 9 gleichen oder verschiedenen Halogenatomen, geradkettiges oder verzweites Halogenalkenyl oder Halogenalkinyl mit 2 bis 6 Kohlenstoffatomen und 1 bis 9 gleichen oder verschiedenen Halogenatomen oder unsubstituiertes oder substituiertes Cycloalkyl oder Cycloalkenyl mit 3 bis 7 Kohlenstoffatomen steht und

$R^{2'}$ und $R^{5'}$ gleich oder verschieden sind und für Wasserstoff oder Alkyl mit 1 bis 6 Kohlenstoffatomen stehen und

$R^{3'}$ für Wasserstoff steht und

$R^{4'}$ für i-Propyl, i-Butyl, s-Butyl oder 3-Pentyl steht oder

$R^{3'}$ und $R^{4'}$ zusammen mit dem Kohlenstoffatom, an das sie gebunden sind, einen Cycloalkylring mit 3 bis 7 Kohlenstoffatomen bilden und

$R^{6'}$ für Wasserstoff, geradkettiges oder verzweigtes Alkyl mit 1 bis 6 Kohlenstoffatomen, für Alkenyl oder Alkinyl mit jeweils 2 bis 6 Kohlenstoffatomen, für Cyanalkyl mit 1 bis 4 Kohlenstoffatomen im Alkylteil, unsubstituiertes oder im Phenylteil einfach bis dreifach, gleich oder verschieden substituiertes Phenylalkyl mit 1 bis 4 Kohlenstoffatomen im Alkylteil oder unsubstituiertes oder einfach bis dreifach, gleich oder verschieden substituiertes Phenyl steht, wobei als Substituenten jeweils in Frage kommen: Halogen; Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 gleichen oder verschiedenen Halogenatomen; Hydroxy; Cyano, Nitro, Amino, Alkylamino, Dialkylamino mit jeweils 1 bis 4 Kohlenstoffatomen und Carboxyl; für unsubstituiertes oder gleich oder verschieden substituiertes Cycloalkyl mit 3 bis 7 Kohlenstoffatomen, wobei als Substituenten Alkyl und Alkoxy mit jeweils 1 bis 4 Kohlenstoffatomen in Frage kommen, oder für unsubstituiertes oder substituiertes Heterocyclyl oder Heterocyclylalkyl mit 2 bis 6 Kohlenstoffatomen im Heterocyclylteil und einem oder zwei Heteroatomen und gegebenenfalls 1 bis 4 Kohlenstoffatomen im Alkylteil steht, wobei als Substituenten die oben genannten Phenylsubstituenten in Frage kommen,

oder

$R^{5'}$ und $R^{6'}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann, ausgenommen Verbindungen, in denen

a)

$R^{4'}$ für i-Butyl steht und

$R^{1'}$ für Methyl oder t-Butyl steht und

$R^{2'}$ für Wasserstoff steht und

$R^{5'}$ für Wasserstoff oder Methyl steht und

$R^{6'}$ für Wasserstoff, Methyl, Ethyl, Phenyl, Benzyl, 2-Phenethyl oder Pyridylmethyl steht oder

$R^{5'}$ und $R^{6'}$ zusammen mit dem Stickstoffatom, an das sie gebunden sind einen Piperidinring bilden und

b)

$R^{4'}$ für s-Butyl steht und

$R^{1'}$ für t-Butyl steht und

$R^{2'}$ für Wasserstoff steht und

$R^{5'}$ für Wasserstoff steht und

$R^{6'}$ für Wasserstoff oder Benzyl steht und

c)

$R^{4'}$ für i-Propyl steht und

$R^{1'}$ für Methyl, Ethyl oder t-Butyl steht und

$R^{2'}$ für Wasserstoff steht und

$R^{5'}$ für Wasserstoff steht und

$R^{6'}$ für Wasserstoff, Methyl, Phenyl oder Benzyl steht oder

$R^{5'}$ und $R^{6'}$ zusammen mit dem Stickstoffatom an das sie gebunden sind einen Pyrrolidinring bilden.

**4.** Verfahren gemäß Anspruch 2, in welcher

$R^{1'}$ für geradkettiges oder verzweigtes Alkyl mit 1 bis 4 Kohlenstoffatomen, geradkettiges oder verzweigtes Alkenyl oder Alkinyl mit 2 bis 4 Kohlenstoffatomen, geradkettiges oder verzweigtes Halogenalkyl mit 1 bis 4 Kohlenstoffatomen und 1 bis 5 gleichen oder verschiedenen Halogenatomen, geradkettiges oder verzweigtes Halogenalkenyl oder Halogenalkinyl mit 2 bis 4 Kohlenstoffatomen und 1 bis 5 gleichen oder verschiedenen Halogenatomen oder unsubstituiertes oder substituiertes Cycloalkyl oder Cycloalkenyl mit 3 bis 6 Kohlenstoffatomen steht und

$R^{2'}$ und $R^{5'}$ gleich oder verschieden sind und für Wasserstoff, Methyl oder Ethyl stehen und

$R^{3'}$ für Wasserstoff steht und

$R^{4'}$ für i-Propyl, i-Butyl, s-Butyl oder 3-Pentyl steht oder

$R^{3'}$ und $R^{4'}$ zusammen mit dem Kohlenstoffatom, an das sie gebunden sind, einen Cycloalkylring mit 3 bis 6 Kohlenstoffatomen bilden und

$R^{6'}$ für Wasserstoff, Methyl, Ethyl, Allyl, Propargyl, für Cyanoalkyl, mit 1 oder 2 Kohlenstoffatomen im Alkylteil; ferner für unsubstituiertes oder im Phenylteil einfach oder zweifach, gleich oder verschieden substituiertes Phenylalkyl mit 1 bis 4 Kohlenstoffatomen im Alkylteil, für unsubstituiertes oder einfach oder zweifach, gleich oder verschieden substituiertes Phenyl steht, wobei als Substituenten jeweils in Frage kommen: Fluor, Chlor und Brom, Methyl, Ethyl, n- und i-Propyl, n-, i-, s- und t-Butyl, Methoxy, Ethoxy, n- und i-Propyloxy, n-, i-, s- und t-Butyloxy, Methylthio, Ethylthio, n- und i-Propylthio, n-, i-, s- und t-Butylthio, Trifluormethyl, Trifluormethoxy, Trifluormethylthio, Hydroxy, Nitro, Cyano, Amino, Methylamino, Ethylamino, Dimethylamino, Diethylamino und Carboxy; für jeweils unsubstituiertes oder einfach oder zweifach, gleich oder verschieden substituiertes Cycloalkyl mit 3 bis 6 Kohlenstoffatomen, wobei als Substituenten Methyl, Ethyl, n- und i-Propyl, n-, i-, s- und t-Butyl, Methoxy, Ethoxy, n- und i-Propyloxy, n-, i-, s- und t-Butyloxy in Frage kommen oder für unsubstituiertes oder einfach oder zweifach, gleich oder verschieden substituiertes Heterocyclyl oder Heterocyclylalkyl mit 4 oder 5 Kohlenstoffatom im Heterocyclylteil und einem oder zwei Stickstoffatomenen und gegebenenfalls 1 bis 4 Kohlenstoffatomen im Alkylteil steht, wobei als Substituenten die obengenannten Phenylsubstituenten in Frage kommen; oder

$R^{5'}$ und $R^{6'}$ gemeinsam mit dem Stickstoffatom, an das sie gebunden sind, für einen 5- oder 6-gliedrigen Heterocyclus stehen, der gegebenenfalls Sauerstoff oder Stickstoff als weitere Heteroatome enthalten kann, ausgenommen Verbindungen, in denen

a)

$R^{4'}$ für i-Butyl steht und

$R^{1'}$ für Methyl oder t-Butyl steht und

$R^{2'}$ für Wasserstoff steht und

$R^{5'}$ für Wasserstoff oder Methyl steht und

$R^{6'}$ für Wasserstoff, Methyl, Ethyl, Phenyl, Benzyl, 2-Phenethyl oder Pyridylmethyl steht oder

$R^{5'}$ und $R^{6'}$ zusammen mit dem Stickstoffatom, an das sie gebunden sind einen Piperidinring bilden und

b)

$R^{4'}$ für s-Butyl steht und

$R^{1'}$ für t-Butyl steht und

$R^{2'}$ für Wasserstoff steht und

$R^{5'}$ für Wasserstoff steht und

$R^{6'}$ für Wasserstoff oder Benzyl steht und

c)

$R^{4'}$ für i-Propyl steht und

$R^{1'}$ für Methyl, Ethyl oder t-Butyl steht und

$R^{2'}$ für Wasserstoff steht und

$R^{5'}$ für Wasserstoff steht und

$R^{6'}$ für Wasserstoff, Methyl, Phenyl oder Benzyl steht oder

$R^{5'}$ und $R^{6'}$ zusammen mit dem Stickstoffatom, an das sie gebunden sind einen Pyrrolidinring bilden.

**5.** Verfahren gemäß Anspruch 2, in welcher

$R^{1'}$ für geradkettiges oder verzweigtes Alkyl mit 1 bis 4 Kohlenstoffatomen, geradkettiges oder verzweigtes Alkenyl oder Alkinyl mit 2 bis 4 Kohlenstoffatomen, geradkettiges oder verzweigtes Halogenalkyl mit 1 bis 4 Kohlenstoffatomen und 1 bis 5 gleichen oder verschiedenen Halogenatomen, geradkettiges oder verzweigtes Halogenalkenyl oder Halogenalkinyl mit 2 bis 4 Kohlenstoffatomen und 1 bis 5 gleichen oder verschiedenen Halogenatomen oder unsubstituiertes oder substituiertes Cycloalkyl oder Cycloalkenyl mit 3 bis 6 Kohlenstoffatomen steht und

$R^{2'}$, $R^{3'}$ und $R^{5'}$ gleich oder verschieden sind und für Wasserstoff stehen und

$R^{4'}$ für i-Propyl, i-Butyl, s-Butyl oder 3-Pentyl steht und

$R^{6'}$ für unsubstituiertes oder im Phenylteil einfach oder zweifach, gleich oder verschieden substituiertes Phenylalkyl mit 1 bis 4 Kohlenstoffatomen im Alkylteil steht, wobei als Substituenten jeweils in Frage kommen: Fluor, Chlor und Brom, Methyl, Ethyl, n- und i-Propyl, n-, i-, s- und t-Butyl, Methoxy, Ethoxy, n- und i-Propyloxy, n-, i-, s- und t-Butyloxy, Methylthio, Ethylthio, n-und i-Propylthio, n-, i-, s- und t-Butylthio, Trifluormethyl, Trifluormethoxy, Trifluormethylthio, Cyano, Dimethylamino, Diethylamino und Carboxy; oder für unsubstituiertes oder einfach oder zweifach substituiertes Heterocyclylalkyl mit 4 oder 5 Kohlenstoffatom im Heterocyclylteil und einem oder zwei Stickstoffatomenen und gegebenenfalls 1 bis 4 Kohlenstoffatomen im Alkylteil steht, wobei als Substituenten die obengenannten Phenylsubstituenten in Frage kommen, ausgenommen Verbindungen, in denen

a)

$R^{4'}$ für i-Butyl steht und
$R^{1'}$ für Methyl oder t-Butyl steht und
$R^{2'}$ für Wasserstoff steht und
$R^{5'}$ für Wasserstoff steht und
$R^{6'}$ für Benzyl, 2-Phenethyl oder Pyridylmethyl steht und

b)

$R^{4'}$ für s-Butyl steht und
$R^{1'}$ für t-Butyl steht und
$R^{2'}$ für Wasserstoff steht und
$R^{5'}$ für Wasserstoff steht und
$R^{6'}$ für Benzyl steht und

c)

$R^{4'}$ für i-Propyl steht und
$R^{1'}$ für Methyl, Ethyl oder t-Butyl steht und
$R^{2'}$ für Wasserstoff steht und
$R^{5'}$ für Wasserstoff steht und
$R^{6'}$ für Benzyl steht.

**6.** Verfahren gemäß Anspruch 2, in welcher

$R^{1'}$ für Methyl, Ethyl, n- und i-Propyl, n-, i-, s- und t-Butyl, Fluormethyl, Fluorethyl, Fluorpropyl, Chlorpropyl, Fluorbutyl, Chlorbutyl, Difluormethyl, Difluorpropyl, Dichlorpropyl, Difluorbutyl, Dichlorbutyl, Trifluormethyl, Trichlormethyl, Trifluorethyl, Trichlorethyl, Trifluorpropyl, Trichlorpropyl, Trifluorbutyl, Trichlorbutyl, Allyl, Butenyl, Propargyl, Butinyl, Fluor- oder Chlor-allyl, butenyl, -propargyl, -butinyl, Cyclopropyl, Cyclopentyl, Cyclohexyl, Cyclopentenyl oder Cyclohexenyl steht und

$R^{2'}$, $R^{3'}$ und $R^{5'}$ für Wasserstoff stehen und

$R^{4'}$ für i-Propyl, i-Butyl, s-Butyl oder 3-Pentyl steht und

$R^{6'}$ für unsubstituiertes oder im Phenylteil einfach oder zweifach, gleich oder verschieden substituiertes Benzyl, 1-Phenethyl, 2-Phenethyl oder 1-Phenylpropyl, 2-Phenylpropyl, 3-Phenylpropyl, 1-Phenylisopropyl, 2-Phenylisopropyl oder 1-Phenyl-n-, -i- oder für steht, wobei als Substituenten jeweils in Frage kommen: Fluor, Chlor, Brom, Methyl, Ethyl, n- und i-Propyl, n-, i-, s- und t-Butyl, Methoxy, Ethoxy, n-und i-Propyloxy, n-, i-, s- und t-Butyloxy, Methylthio, Ethylthio, n-und i-Propylthio, n-, i-, s- und t-Butylthio, Trifluormethyl, Trifluormethoxy, Trifluormethylthio, Cyano, Dimethylamino und Diethylamino; für unsubstituiertes oder ein oder zweifach, gleich oder verschieden substituiertes Pyridylmethyl oder 1-bzw, 2-Pyridylethyl steht, wobei als Substituenten die oben genannten Phenylsubstituenten in Frage kommen, ausgenommen Verbindungen, in denen

a)
$R^{4'}$ für i-Butyl steht und
$R^{1'}$ für Methyl oder t-Butyl steht und
$R^{2'}$ für Wasserstoff steht und
$R^{5'}$ für Wasserstoff steht und
$R^{6'}$ für Benzyl, 2-Phenethyl oder Pyridylmethyl steht und

b)
$R^{4'}$ für s-Butyl steht und
$R^{1'}$ für t-Butyl steht und
$R^{2'}$ für Wasserstoff steht und
$R^{5'}$ für Wasserstoff steht und
$R^{6'}$ für Benzyl steht und

c)
$R^{4'}$ für i-Propyl steht und
$R^{1'}$ für Methyl, Ethyl oder t-Butyl steht und
$R^{2'}$ für Wasserstoff steht und
$R^{5'}$ für Wasserstoff steht und
$R^{6'}$ für Benzyl steht.

**7.** Schädlingsbekämpfungsmittel, gekennzeichnet durch einen Gehalt an mindestens einem Aminosäureamid-Derivat der Formel (I) oder (Ia) nach den Ansprüchen 1 bis 6.

**8.** Verfahren zur Bekämpfung von phytophatogenen Pilzen, dadurch gekennzeichnet, daß man Aminosäureamid-Derivate der Formel (I) oder (Ia) nach den Ansprüchen 1 bis 6 auf Pilze und/oder ihren Lebensraum einwirken läßt.

**9.** Verfahren zur Herstellung von Schädlingsbekämpfungsmitteln, dadurch gekennzeichnet, daß man Aminosäureamid-Derivate der Formel (I) oder (Ia) nach den Ansprüchen 1 bis 6 mit Streckmitteln und/oder oberflächenaktiven Mitteln vermischt.

## Claims

**Claims for the following Contracting States : BE, CH, DE, FR, GB, GR, IT, LI, NL**

**1.** Use of substituted amino acid amide derivatives of the general formula (I)

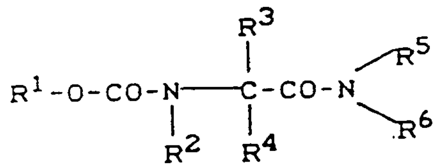

(I)

in which

$R^1$ represents alkyl, alkenyl, alkinyl, halogenoalkyl, halogenoalkenyl, halogenoalkinyl, cycloalkyl or cycloalkenyl,

$R^2$, $R^3$, $R^4$ and $R^5$ are identical or different and represent hydrogen, cycloalkyl or alkyl, or

$R^3$ and $R^4$, together with the carbon atom to which they are bonded, form a cycloalkyl ring, and

$R^6$ represents hydrogen, alkyl, alkenyl, alkinyl, cyanoalkyl, unsubstituted or substituted phenylalkyl, unsubstituted or substituted phenyl or unsubstituted or substituted cycloalkyl, or represents unsubstituted or substituted heterocyclyl or heterocyclylalkyl,

or

$R^5$ and $R^6$, together with the nitrogen atom to which they are bonded, represent a heterocyclyl radical, which can contain further hetero atoms,
for combating phytopathogenic fungi.

**2.** Substituted amino acid amide derivatives of the general formula (Ia)

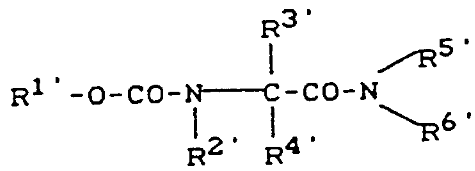

(Ia)

in which

$R^{1'}$ represents alkyl, alkenyl, alkinyl, halogenoalkyl, halogenoalkenyl, halogenoalkinyl, cycloalkyl or cycloalkenyl,
$R^{2'}$ and $R^{5'}$ are identical or different and represent hydrogen or alkyl,
$R^{3'}$ represents hydrogen and
$R^{4'}$ represents i-propyl, i-butyl, s-butyl, 3-pentyl or cycloalkyl or
$R^{3'}$ and $R^{4'}$, together with the carbon atom to which they are bonded, form a cycloalkyl ring, and
$R^{6'}$ represents hydrogen, alkyl, alkenyl, alkinyl, cyanoalkyl, unsubstituted or substituted phenylalkyl, unsubstituted or substituted phenyl or unsubstituted or substituted cycloalkyl, or represents unsubstituted or substituted heterocyclyl or heterocyclylalkyl,

or

$R^{5'}$ and $R^{6'}$, together with the nitrogen atom to which they are bonded, represent a heterocyclyl radical which can contain further hetero atoms, excluding compounds in which

a)
$R^{4'}$ represents i-butyl and
$R^{1'}$ represents methyl or t-butyl and
$R^{2'}$ represents hydrogen and
$R^{5'}$ represents hydrogen or methyl and
$R^{6'}$ represents hydrogen, methyl, ethyl, phenyl, benzyl, 2-phenethyl or pyridylmethyl or
$R^{5'}$ and $R^{6'}$, together with the nitrogen atom to which they are bonded, form a piperidine ring and

b)
$R^{4'}$ represents s-butyl and
$R^{1'}$ represents t-butyl and
$R^{2'}$ represents hydrogen and
$R^{5'}$ represents hydrogen and
$R^{6'}$ represents hydrogen or benzyl and

c)
$R^{4'}$ represents i-propyl and
$R^{1'}$ represents methyl, ethyl or t-butyl and
$R^{2'}$ represents hydrogen and
$R^{5'}$ represents hydrogen and
$R^{6'}$ represents hydrogen, methyl, phenyl or benzyl or
$R^{5'}$ and $R^{6'}$, together with the nitrogen atom to which they are bonded, form a pyrrolidine ring.

**3.** Substituted amino acid amide derivatives of the formula (Ia) according to Claim 2, in which

R1' represents straight-chain or branched alkyl having 1 to 6 carbon atoms, straight-chain or branched alkenyl or alkinyl having 2 to 6 carbon atoms, straight-chain or branched halogenoalkyl having 1 to 6 carbon atoms and 1 to 9 identical or different halogen atoms, straight-chain or branched halogenoalkenyl or halogenoalkinyl having 2 to 6 carbon atoms and 1 to 9 identical or different halogen atoms or unsubstituted or substituted cycloalkyl or cycloalkenyl having 3 to 7 carbon atoms,

$R^{2'}$ and $R^{5'}$ are identical or different and represent hydrogen or alkyl having 1 to 6 carbon atoms,
$R^{3'}$ represents hydrogen and
$R^{4'}$ represents i-propyl, i-butyl, s-butyl or 3-pentyl, or
$R^{3'}$ and $R^{4'}$, together with the carbon atom to which they are bonded, form a cycloalkyl ring having 3 to 7 carbon atoms and
$R^{6'}$ represents hydrogen or straight-chain or branched alkyl having 1 to 6 carbon atoms, or represents alkenyl or alkinyl having in each case 2 to 6 carbon atoms, or represents cyanoalkyl having 1 to 4 carbon atoms in the alkyl part, phenylalkyl having 1 to 4 carbon atoms in the alkyl part, which is unsubstituted or substituted in the phenyl part by one to three identical or different substituents, or phenyl which is unsubstituted or substituted by one to three identical or different substituents, possible substituents in each case being: halogen; alkyl, alkoxy and alkylthio having in each case 1 to 4 carbon atoms; halogenoalkyl, halogenoalkoxy and halogenoalkylthio having in each case 1 to 4 carbon atoms and 1 to 9 identical or different halogen atoms; hydroxyl; cyano, nitro, amino, alkylamino and dialkylamino having in each case 1 to 4 carbon atoms and carboxyl; or represents cycloalkyl having 3 to 7 carbon atoms which is unsubstituted or substituted by identical or different substituents, possible substituents being alkyl and alkoxy having in each case 1 to 4 carbon atoms, or represents unsubstituted or substituted heterocyclyl or heterocyclylalkyl having 2 to 6 carbon atoms in the heterocyclyl part and one or two hetero atoms and if appropriate 1 to 4 carbon atoms in the alkyl part, possible substituents being the abovementioned substituents for phenyl,

or

$R^{5'}$ and $R^{6'}$, together with the nitrogen atom to which they are bonded, represent a 5- or 6-membered heterocyclic radical, which can optionally contain oxygen or nitrogen as further hetero atoms, excluding compounds in which

a)
$R^{4'}$ represents i-butyl and
$R^{1'}$ represents methyl or t-butyl and
$R^{2'}$ represents hydrogen and
$R^{5'}$ represents hydrogen or methyl and
$R^{6'}$ represents hydrogen, methyl, ethyl, phenyl, benzyl, 2-phenethyl or pyridylmethyl or
$R^{5'}$ and $R^{6'}$, together with the nitrogen atom to which they are bonded, form a piperidine ring and

b)
$R^{4'}$ represents s-butyl and
$R^{1'}$ represents t-butyl and
$R^{2'}$ represents hydrogen and
$R^{5'}$ represents hydrogen and
$R^{6'}$ represents hydrogen or benzyl and

c)
$R^{4'}$ represents i-propyl and
$R^{1'}$ represents methyl, ethyl or t-butyl and
$R^{2'}$ represents hydrogen and
$R^{5'}$ represents hydrogen and
$R^{6'}$ represents hydrogen, methyl, phenyl or benzyl or
$R^{5'}$ and $R^{6'}$, together with the nitrogen atom to which they are bonded, form a pyrrolidine ring.

**4.** Substituted amino acid amide derivatives of the formula (Ia) according to Claim 2, in which
$R^{1'}$ represents straight-chain or branched alkyl having 1 to 4 carbon atoms, straight-chain or branched alkenyl or alkinyl having 2 to 4 carbon atoms, straight-chain or branched halogenoalkyl having 1 to 4 carbon atoms and 1 to 5 identical or different halogen atoms, straight-chain or branched halogenoalkenyl or halogenoalkinyl having 2 to 4 carbon atoms and 1 to 5 identical or different halogen atoms or unsubstituted or substituted cycloalkyl or cycloalkenyl having 3 to 6 carbon atoms,
$R^{2'}$ and $R^{5'}$ are identical or different and represent hydrogen, methyl or ethyl,
$R^{3'}$ represents hydrogen and

$R^{4'}$ represents i-propyl, i-butyl, s-butyl or 3-pentyl, or

$R^{3'}$ and $R^{4'}$, together with the carbon atom to which they are bonded, form a cycloalkyl ring having 3 to 6 carbon atoms, and

$R^{6'}$ represents hydrogen, methyl, ethyl, allyl or propargyl, or represents cyanoalkyl having 1 or 2 carbon atoms in the alkyl part; or furthermore represents phenylalkyl having 1 to 4 carbon atoms in the alkyl part, which is unsubstituted or substituted in the phenyl part by one or two identical or different substituents, or represents phenyl which is unsubstituted or substituted by one or two identical or different substituents, possible substituents in each case being: fluorine, chlorine and bromine, methyl, ethyl, n- and i-propyl, n-, i-, s- and t-butyl, methoxy, ethoxy, n- and i-propoxy, n-, i-, s-and t-butoxy, methylthio, ethylthio, n- and i-propylthio, n-, i-, s- and t-butylthio, trifluoromethyl, trifluoromethoxy, trifluoromethylthio, hydroxyl, nitro, cyano, amino, methylamino, ethylamino, dimethylamino, diethylamino and carboxyl; or represents cycloalkyl having 3 to 6 carbon atoms, in each case unsubstituted or substituted by one or two identical or different substituents, possible substituents being methyl, ethyl, n- and i-propyl, n-, i-, s- and t-butyl, methoxy, ethoxy, n- and i-propoxy and n-, i-, s- and t-butoxy, or represents heterocyclyl or heterocyclylalkyl having 4 or 5 carbon atoms in the heterocyclyl part and one or two nitrogen atoms and if appropriate 1 to 4 carbon atoms in the alkyl part and being unsubstituted or substituted by one or two identical or different substituents, possible substituents being the abovementioned substituents for phenyl; or

$R^{5'}$ and $R^{6'}$, together with the nitrogen atom to which they are bonded, represent a 5- or 6-membered heterocyclic radical, which can optionally contain oxygen or nitrogen as further hetero atoms, excluding compounds in which

a)

R4' represents i-butyl and

$R^{1'}$ represents methyl or t-butyl and

$R^{2'}$ represents hydrogen and

$R^{5'}$ represents hydrogen or methyl and

$R^{6'}$ represents hydrogen, methyl, ethyl, phenyl, benzyl, 2-phenethyl or pyridylmethyl or

$R^{5'}$ and $R^{6'}$, together with the nitrogen atom to which they are bonded, form a piperidine ring and

b)

$R^{4'}$ represents s-butyl and

$R^{1'}$ represents t-butyl and

$R^{2'}$ represents hydrogen and

$R^{5'}$ represents hydrogen and

$R^{6'}$ represents hydrogen or benzyl and

c)

$R^{4'}$ represents i-propyl and

$R^{1'}$ represents methyl, ethyl or t-butyl and

$R^{2'}$ represents hydrogen and

$R^{5'}$ represents hydrogen and

$R^{6'}$ represents hydrogen, methyl, phenyl or benzyl or

$R^{5'}$ and $R^{6'}$, together with the nitrogen atom to which they are bonded, form a pyrrolidine ring.

**5.** Substituted amino acid amide derivatives of the formula (Ia) according to Claim 2, in which

$R^{1'}$ represents straight-chain or branched alkyl having 1 to 4 carbon atoms, straight-chain or branched alkenyl or alkinyl having 2 to 4 carbon atoms, straight-chain or branched halogenoalkyl having 1 to 4 carbon atoms and 1 to 5 identical or different halogen atoms, straight-chain or branched halogenoalkenyl or halogenoalkinyl having 2 to 4 carbon atoms and 1 to 5 identical or different halogen atoms or unsubstituted or substituted cycloalkyl or cycloalkenyl having 3 to 6 carbon atoms,

$R^{2'}$, $R^{3'}$ and $R^{5'}$ are identical or different and represent hydrogen and

$R^{4'}$ represents i-propyl, i-butyl, s-butyl or 3-pentyl, and

$R^{6'}$ represents phenylalkyl having 1 to 4 carbon atoms in the alkyl part, which is

unsubstituted or substituted in the phenyl part by one or two identical or different substituents, possible substituents in each case being: fluorine, chlorine and bromine, methyl, ethyl, n- and i-propyl, n-, i-, s- and t-butyl, methoxy, ethoxy, n- and i-propoxy, n-, i-, s- and t-butoxy, methylthio, ethylthio, n- and i-propylthio, n-, i-, s- and t-butylthio, trifluoromethyl, trifluoromethoxy, trifluoromethylthio, cyano, dimethylamino, diethylamino and carboxyl; or represents heterocyclylalkyl having 4 or 5 carbon atoms in the heterocyclyl part and one or two nitrogen atoms and if appropriate 1 to 4 carbon atoms in the alkyl part and being unsubstituted or substituted by one or two substituents, possible substituents being the abovementioned substituents for phenyl, excluding compounds in which

a)
$R^{4'}$ represents i-butyl and
$R^{1'}$ represents methyl or t-butyl and
$R^{2'}$ represents hydrogen and
$R^{5'}$ represents hydrogen and
$R^{6'}$ represents benzyl, 2-phenethyl or pyridylmethyl
and
b)
$R^{4'}$ represents s-butyl and
$R^{1'}$ represents t-butyl and
$R^{2'}$ represents hydrogen and
$R^{5'}$ represents hydrogen and
$R^{6'}$ represents benzyl and
c)
$R^{4'}$ represents i-propyl and
$R^{1'}$ represents methyl, ethyl or t-butyl and
$R^{2'}$ represents hydrogen and
$R^{5'}$ represents hydrogen and
$R^{6'}$ represents benzyl.

**6.** Substituted amino acid amide derivatives of the formula (Ia) according to Claim 2, in which
$R^{1'}$ represents methyl, ethyl, n- or i-propyl, n-, i-, s- or t-butyl, fluoromethyl, fluoroethyl, fluoropropyl, chloropropyl, fluorobutyl, chlorobutyl, difluoromethyl, difluoropropyl, dichloropropyl, difluorobutyl, dichlorobutyl, trifluoromethyl, trichloromethyl, trifluoroethyl, trichloroethyl, trifluoropropyl, trichloropropyl, trifluorobutyl, trichlorobutyl, allyl, butenyl, propargyl, butinyl, fluoro- or chloro-allyl, -butenyl, -propargyl, -butinyl, cyclopropyl, cyclopentyl, cyclohexyl, cyclopentenyl or cyclohexenyl and
$R^{2'}$, $R^{3'}$ and $R^{5'}$ represent hydrogen and
$R^{4'}$ represents i-propyl, i-butyl, s-butyl or 3-pentyl and
$R^{6'}$ represents benzyl, 1-phenethyl, 2-phenethyl or 1-phenylpropyl, 2-phenylpropyl, 3-phenylpropyl, 1-phenylisopropyl, 2-phenylisopropyl or 1-phenyl-n-, -i- or -s-butyl, which is unsubstituted or substituted in the phenyl part by one or two identical or different substituents, possible substituents in each case being: fluorine, chlorine, bromine, methyl, ethyl, n- and i-propyl, n-, i-, s- and t-butyl, methoxy, ethoxy, n- and i-propoxy, n-, i-, s- and t-butoxy, methylthio, ethylthio, n- and i-propylthio, n-, i-, s- and t-butylthio, trifluoromethyl, trifluoromethoxy, trifluoromethylthio, cyano, dimethylamino and diethylamino; or represents pyridylmethyl or 1- or 2-pyridylethyl which is unsubstituted or substituted by one or two identical or different substituents, possible substituents being the abovementioned substituents for phenyl, excluding compounds in which

a)
$R^{4'}$ represents i-butyl and
$R^{1'}$ represents methyl or t-butyl and
$R^{2'}$ represents hydrogen and
$R^{5'}$ represents hydrogen and
$R^{6'}$ represents benzyl, 2-phenethyl or pyridylmethyl
and

b)

$R^{4'}$ represents s-butyl and
$R^{1'}$ represents t-butyl and
$R^{2'}$ represents hydrogen and
$R^{5'}$ represents hydrogen and
$R^{6'}$ represents benzyl and

c)

$R^{4'}$ represents i-propyl and
$R^{1'}$ represents methyl, ethyl or t-butyl and
$R^{2'}$ represents hydrogen and
$R^{5'}$ represents hydrogen and
$R^{6'}$ represents benzyl.

**7.** Process for the preparation of substituted amino acid amide derivatives of the general formula (Ia)

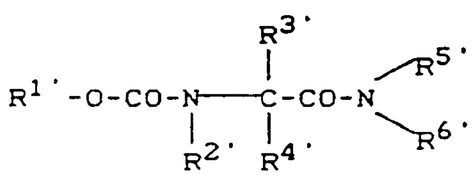

(Ia)

in which

$R^{1'}$ represents alkyl, alkenyl, alkinyl, halogenoalkyl, halogenoalkenyl, halogenoalkinyl, cycloalkyl or cycloalkenyl,
$R^{2'}$ and $R^{5'}$ are identical or different and represent hydrogen or alkyl,
$R^{3'}$ represents hydrogen and
$R^{4'}$ represents i-propyl, i-butyl, s-butyl, 3-pentyl or cycloalkyl or
$R^{3'}$ and $R^{4'}$, together with the carbon atom to which they are bonded, form a cycloalkyl ring, and
$R^{6'}$ represents hydrogen, alkyl, alkenyl, alkinyl, cyanoalkyl, unsubstituted or substituted phenylalkyl, unsubstituted or substituted phenyl or unsubstituted or substituted cycloalkyl, or represents unsubstituted or substituted heterocyclyl or heterocyclylalkyl,

or

$R^{5'}$ and $R^{6'}$, together with the nitrogen atom to which they are bonded, represent a heterocyclyl radical which can contain further hetero atoms, excluding compounds in which

a)

$R^{4'}$ represents i-butyl and
$R^{1'}$ represents methyl or t-butyl and
$R^{2'}$ represents hydrogen and
$R^{5'}$ represents hydrogen or methyl and
$R^{6'}$ represents hydrogen, methyl, ethyl, phenyl, benzyl, 2-phenethyl or pyridylmethyl or
$R^{5'}$ and $R^{6'}$, together with the nitrogen atom to which they are bonded, form a piperidine ring and

b)

$R^{4'}$ represents s-butyl and
$R^{1'}$ represents t-butyl and
$R^{2'}$ represents hydrogen and
$R^{5'}$ represents hydrogen and
$R^{6'}$ represents hydrogen or benzyl and

c)

$R^{4'}$ represents i-propyl and
$R^{1'}$ represents methyl, ethyl or t-butyl and
$R^{2'}$ represents hydrogen and
$R^{5'}$ represents hydrogen and
$R^{6'}$ represents hydrogen, methyl, phenyl or benzyl or
$R^{5'}$ and $R^{6'}$, together with the nitrogen atom to which they are bonded, form a pyrrolidine ring,

characterized in that
a substituted amino acid of the formula (II)

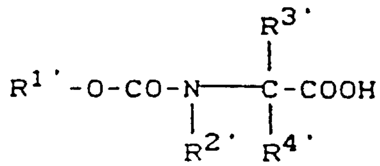

(II)

in which
$R^{1'}$, $R^{2'}$, $R^{3'}$ and $R^{4'}$ have the abovementioned meaning, or carboxy-activated derivatives thereof,
is reacted with an amine of the formula (III)

$HNR^{5'}R^{6'}$ (III)

in which
$R^{5'}$ and $R^{6'}$ have the abovementioned meaning,
if appropriate in the presence of a catalyst, if appropriate in the presence of an acid-binding agent and if appropriate in the presence of a diluent.

8. Agents for combating pests, characterized in that they contain at least one amino acid amide derivative of the formula (I) or (Ia) according to Claims 1 to 7.

9. Method of combating phytopathogenic fungi, characterized in that amino acid amide derivatives of the formula (I) or (Ia) according to Claims 1 to 7 are allowed to act on fungi and/or their environment.

10. Process for the preparation of agents for combating pests, characterized in that amino acid amide derivatives of the formula (I) or (Ia) according to Claims 1 to 7 are mixed with extenders and/or surface active agents.

**Claims for the following Contracting State : ES**

1. Use of substituted amino acid amide derivatives of the general formula (I)

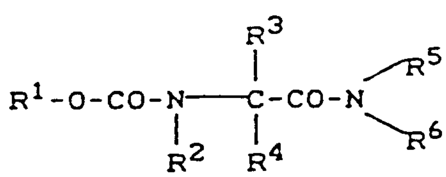

(I)

in which

| | |
|---|---|
| $R^1$ | represents alkyl, alkenyl, alkinyl, halogenoalkyl, halogenoalkenyl, halogenoalkinyl, cycloalkyl or cycloalkenyl, |
| $R^2$, $R^3$, $R^4$ and $R^5$ | are identical or different and represent hydrogen, cycloalkyl or alkyl, or |
| $R^3$ and $R^4$, | together with the carbon atom to which they are bonded, form a cycloalkyl ring, and |
| $R^6$ | represents hydrogen, alkyl, alkenyl, alkinyl, cyanoalkyl, unsubstituted or substituted phenylalkyl, unsubstituted or substituted phenyl or unsubstituted or substituted cycloalkyl, or represents unsubstituted or substituted heterocyclyl or heterocyclylalkyl, |

or
$R^5$ and $R^6$, together with the nitrogen atom to which they are bonded, represent a heterocyclyl radical, which can contain further hetero atoms,
for combating phytopathogenic fungi.

**2.** Process for the preparation of substituted amino acid amide derivatives of the general formula (Ia)

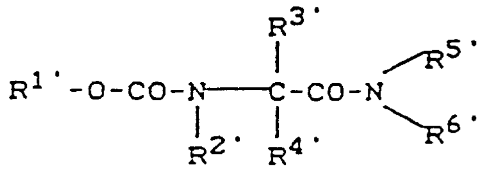

(Ia)

in which

$R^{1'}$ represents alkyl, alkenyl, alkinyl, halogenoalkyl, halogenoalkenyl, halogenoalkinyl, cycloalkyl or cycloalkenyl,

$R^{2'}$ and $R^{5'}$ are identical or different and represent hydrogen or alkyl,

$R^{3'}$ represents hydrogen and

$R^{4'}$ represents i-propyl, i-butyl, s-butyl, 3-pentyl or cycloalkyl or

$R^{3'}$ and $R^{4'}$, together with the carbon atom to which they are bonded, form a cycloalkyl ring, and

$R^{6'}$ represents hydrogen, alkyl, alkenyl, alkinyl, cyanoalkyl, unsubstituted or substituted phenylalkyl, unsubstituted or substituted phenyl or unsubstituted or substituted cycloalkyl, or represents unsubstituted or substituted heterocyclyl or heterocyclylalkyl,

or

$R^{5'}$ and $R^{6'}$, together with the nitrogen atom to which they are bonded, represent a heterocyclyl radical which can contain further hetero atoms, excluding compounds in which

a)

$R^{4'}$ represents i-butyl and

$R^{1'}$ represents methyl or t-butyl and

$R^{2'}$ represents hydrogen and

$R^{5'}$ represents hydrogen or methyl and

$R^{6'}$ represents hydrogen, methyl, ethyl, phenyl, benzyl, 2-phenethyl or pyridylmethyl or

$R^{5'}$ and $R^{6'}$, together with the nitrogen atom to which they are bonded, form a piperidine ring and

b)

$R^{4'}$ represents s-butyl and

$R^{1'}$ represents t-butyl and

$R^{2'}$ represents hydrogen and

$R^{5'}$ represents hydrogen and

$R^{6'}$ represents hydrogen or benzyl and

c)

$R^{4'}$ represents i-propyl and

$R^{1'}$ represents methyl, ethyl or t-butyl and

$R^{2'}$ represents hydrogen and

$R^{5'}$ represents hydrogen and

$R^{6'}$ represents hydrogen, methyl, phenyl or benzyl or

$R^{5'}$ and $R^{6'}$, together with the nitrogen atom to which they are bonded, form a pyrrolidine ring,

characterized in that

a substituted amino acid of the formula (II)

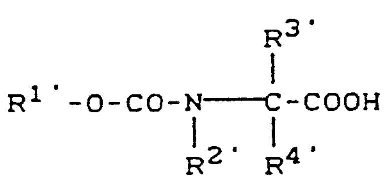

(II)

in which

$R^{1'}$, $R^{2'}$, $R^{3'}$ and $R^{4'}$ have the abovementioned meaning, or carboxy-activated derivatives thereof,

is reacted with an amine of the formula (III)

$HNR^{5'}R^{6'}$ (III)

in which

$R^{5'}$ and $R^{6'}$ have the abovementioned meaning,

if appropriate in the presence of a catalyst, if appropriate in the presence of an acid-binding agent and if appropriate in the presence of a diluent.

**3.** Process according to Claim 2, in which

$R^{1'}$ represents straight-chain or branched alkyl having 1 to 6 carbon atoms, straight-chain or branched alkenyl or alkinyl having 2 to 6 carbon atoms, straight-chain or branched halogenoalkyl having 1 to 6 carbon atoms and 1 to 9 identical or different halogen atoms, straight-chain or branched halogenoalkenyl or halogenoalkinyl having 2 to 6 carbon atoms and 1 to 9 identical or different halogen atoms or unsubstituted or substituted cycloalkyl or cycloalkenyl having 3 to 7 carbon atoms,

$R^{2'}$ and $R^{5'}$ are identical or different and represent hydrogen or alkyl having 1 to 6 carbon atoms,

$R^{3'}$ represents hydrogen and

$R^{4'}$ represents i-propyl, i-butyl, s-butyl or 3-pentyl, or

$R^{3'}$ and $R^{4'}$, together with the carbon atom to which they are bonded, form a cycloalkyl ring having 3 to 7 carbon atoms and

$R^{6'}$ represents hydrogen or straight-chain or branched alkyl having 1 to 6 carbon atoms, or represents alkenyl or alkinyl having in each case 2 to 6 carbon atoms, or represents cyanoalkyl having 1 to 4 carbon atoms in the alkyl part, phenylalkyl having 1 to 4 carbon atoms in the alkyl part, which is unsubstituted or substituted in the phenyl part by one to three identical or different substituents, or phenyl which is unsubstituted or substituted by one to three identical or different substituents, possible substituents in each case being: halogen; alkyl, alkoxy and alkylthio having in each case 1 to 4 carbon atoms; halogenoalkyl, halogenoalkoxy and halogenoalkylthio having in each case 1 to 4 carbon atoms and 1 to 9 identical or different halogen atoms; hydroxyl; cyano, nitro, amino, alkylamino and dialkylamino having in each case 1 to 4 carbon atoms and carboxyl; or represents cycloalkyl having 3 to 7 carbon atoms which is unsubstituted or substituted by identical or different substituents, possible substituents being alkyl and alkoxy having in each case 1 to 4 carbon atoms, or represents unsubstituted or substituted heterocyclyl or heterocyclylalkyl having 2 to 6 carbon atoms in the heterocyclyl part and one or two hetero atoms and if appropriate 1 to 4 carbon atoms in the alkyl part, possible substituents being the abovementioned substituents for phenyl,

or

$R^{5'}$ and $R^{6'}$, together with the nitrogen atom to which they are bonded, represent a 5- or 6-membered heterocyclic radical, which can optionally contain oxygen or nitrogen as further hetero atoms, excluding compounds in which

a)

$R^{4'}$ represents i-butyl and

$R^{1'}$ represents methyl or t-butyl and

$R^{2'}$ represents hydrogen and

$R^{5'}$ represents hydrogen or methyl and

$R^{6'}$ represents hydrogen, methyl, ethyl, phenyl, benzyl, 2-phenethyl or pyridylmethyl or

$R^{5'}$ and $R^{6'}$, together with the nitrogen atom to which they are bonded, form a piperidine ring and

b)

$R^{4'}$ represents s-butyl and

$R^{1'}$ represents t-butyl and

$R^{2'}$ represents hydrogen and

$R^{5'}$ represents hydrogen and

$R^{6'}$ represents hydrogen or benzyl and

c)

$R^{4'}$ represents i-propyl and
$R^{1'}$ represents methyl, ethyl or t-butyl and
$R^{2'}$ represents hydrogen and
$R^{5'}$ represents hydrogen and
$R^{6'}$ represents hydrogen, methyl, phenyl or benzyl or
$R^{5'}$ and $R^{6'}$, together with the nitrogen atom to which they are bonded, form a pyrrolidine ring.

**4.** Process according to Claim 2, in which

$R^{1'}$ represents straight-chain or branched alkyl having 1 to 4 carbon atoms, straight-chain or branched alkenyl or alkinyl having 2 to 4 carbon atoms, straight-chain or branched halogenoalkyl having 1 to 4 carbon atoms and 1 to 5 identical or different halogen atoms, straight-chain or branched halogenoalkenyl or halogenoalkinyl having 2 to 4 carbon atoms and 1 to 5 identical or different halogen atoms or unsubstituted or substituted cycloalkyl or cycloalkenyl having 3 to 6 carbon atoms,
$R^{2'}$ and $R^{5'}$ are identical or different and represent hydrogen, methyl or ethyl,
$R^{3'}$ represents hydrogen and
$R^{4'}$ represents i-propyl, i-butyl, s-butyl or 3-pentyl, or
$R^{3'}$ and $R^{4'}$, together with the carbon atom to which they are bonded, form a cycloalkyl ring having 3 to 6 carbon atoms, and
$R^{6'}$ represents hydrogen, methyl, ethyl, allyl or propargyl, or represents cyanoalkyl having 1 or 2 carbon atoms in the alkyl part; or furthermore represents phenylalkyl having 1 to 4 carbon atoms in the alkyl part, which is unsubstituted or substituted in the phenyl part by one or two identical or different substituents, or represents phenyl which is unsubstituted or substituted by one or two identical or different substituents, possible substituents in each case being: fluorine, chlorine and bromine, methyl, ethyl, n- and i-propyl, n-, i-, s- and t-butyl, methoxy, ethoxy, n- and i-propoxy, n-, i-, s-and t-butoxy, methylthio, ethylthio, n- and i-propylthio, n-, i-, s- and t-butylthio, trifluoromethyl, trifluoromethoxy, trifluoromethylthio, hydroxyl, nitro, cyano, amino, methylamino, ethylamino, dimethylamino, diethylamino and carboxyl; or represents cycloalkyl having 3 to 6 carbon atoms, in each case unsubstituted or substituted by one or two identical or different substituents, possible substituents being methyl, ethyl, n- and i-propyl, n-, i-, s- and t-butyl, methoxy, ethoxy, n- and i-propoxy and n-, i-, s- and t-butoxy, or represents heterocyclyl or heterocyclylalkyl having 4 or 5 carbon atoms in the heterocyclyl part and one or two nitrogen atoms and if appropriate 1 to 4 carbon atoms in the alkyl part and being unsubstituted or substituted by one or two identical or different substituents, possible substituents being the abovementioned substituents for phenyl; or
$R^{5'}$ and $R^{6'}$, together with the nitrogen atom to which they are bonded, represent a 5- or 6-membered heterocyclic radical, which can optionally contain oxygen or nitrogen as further hetero atoms, excluding compounds in which

a)

$R^{4'}$ represents i-butyl and
$R^{1'}$ represents methyl or t-butyl and
$R^{2'}$ represents hydrogen and
$R^{5'}$ represents hydrogen or methyl and
$R^{6'}$ represents hydrogen, methyl, ethyl, phenyl, benzyl, 2-phenethyl or pyridylmethyl or
$R^{5'}$ and $R^{6'}$, together with the nitrogen atom to which they are bonded, form a piperidine ring and

b)

$R^{4'}$ represents s-butyl and
$R^{1'}$ represents t-butyl and
$R^{2'}$ represents hydrogen and
$R^{5'}$ represents hydrogen and
$R^{6'}$ represents hydrogen or benzyl and

c)

$R^{4'}$ represents i-propyl and

$R^{1'}$ represents methyl, ethyl or t-butyl and
$R^{2'}$ represents hydrogen and
$R^{5'}$ represents hydrogen and
$R^{6'}$ represents hydrogen, methyl, phenyl or benzyl or
$R^{5'}$ and $R^{6'}$, together with the nitrogen atom to which they are bonded, form a pyrrolidine ring.

**5.** Process according to Claim 2, in which
$R^{1'}$ represents straight-chain or branched alkyl having 1 to 4 carbon atoms, straight-chain or branched alkenyl or alkinyl having 2 to 4 carbon atoms, straight-chain or branched halogenoalkyl having 1 to 4 carbon atoms and 1 to 5 identical or different halogen atoms, straight-chain or branched halogenoalkenyl or halogenoalkinyl having 2 to 4 carbon atoms and 1 to 5 identical or different halogen atoms or unsubstituted or substituted cycloalkyl or cycloalkenyl having 3 to 6 carbon atoms,
$R^{2'}$, $R^{3'}$ and $R^{5'}$ are identical or different and represent hydrogen and
$R^{4'}$ represents i-propyl, i-butyl, s-butyl or 3-pentyl, and
$R^{6'}$ represents phenylalkyl having 1 to 4 carbon atoms in the alkyl part, which is unsubstituted or substituted in the phenyl part by one or two identical or different substituents, possible substituents in each case being: fluorine, chlorine and bromine, methyl, ethyl, n- and i-propyl, n-, i-, s- and t-butyl, methoxy, ethoxy, n- and i-propoxy, n-, i-, s- and t-butoxy, methylthio, ethylthio, n- and i-propylthio, n-, i-, s- and t-butylthio, trifluoromethyl, trifluoromethoxy, trifluoromethylthio, cyano, dimethylamino, diethylamino and carboxyl; or represents heterocyclylalkyl having 4 or 5 carbon atoms in the heterocyclyl part and one or two nitrogen atoms and if appropriate 1 to 4 carbon atoms in the alkyl part and being unsubstituted or substituted by one or two substituents, possible substituents being the abovementioned substituents for phenyl, excluding compounds in which

a)
$R^{4'}$ represents i-butyl and
$R^{1'}$ represents methyl or t-butyl and
$R^{2'}$ represents hydrogen and
$R^{5'}$ represents hydrogen and
$R^{6'}$ represents benzyl, 2-phenethyl or pyridylmethyl

and

b)
$R^{4'}$ represents s-butyl and
$R^{1'}$ represents t-butyl and
$R^{2'}$ represents hydrogen and
$R^{5'}$ represents hydrogen and
$R^{6'}$ represents benzyl and

c)
$R^{4'}$ represents i-propyl and
$R^{1'}$ represents methyl, ethyl or t-butyl and
$R^{2'}$ represents hydrogen and
$R^{5'}$ represents hydrogen and
$R^{6'}$ represents benzyl.

**6.** Process according to Claim 2, in which
$R^{1'}$ represents methyl, ethyl, n- or i-propyl, n-, i-, s- or t-butyl, fluoromethyl, fluoroethyl, fluoropropyl, chloropropyl, fluorobutyl, chlorobutyl, difluoromethyl, difluoropropyl, dichloropropyl, difluorobutyl, dichlorobutyl, trifluoromethyl, trichloromethyl, trifluoroethyl, trichloroethyl, trifluoropropyl, trichloropropyl, trifluorobutyl, trichlorobutyl, allyl, butenyl, propargyl, butinyl, fluoro- or chloro-allyl, -butenyl, -propargyl, -butinyl, cyclopropyl, cyclopentyl, cyclohexyl, cyclopentenyl or cyclohexenyl and
$R^{2'}$, $R^{3'}$ and $R^{5'}$ represent hydrogen and
$R^{4'}$ represents i-propyl, i-butyl, s-butyl or 3-pentyl and
$R^{6'}$ represents benzyl, 1-phenethyl, 2-phenethyl or 1-phenylpropyl, 2-phenylpropyl,

3-phenylpropyl, 1-phenylisopropyl, 2-phenylisopropyl or 1-phenyl-n, -i- or -s-butyl, which is unsubstituted or substituted in the phenyl part by one or two identical or different substituents, possible substituents in each case being: fluorine, chlorine, bromine, methyl, ethyl, n- and i-propyl, n-, i-, s- and t-butyl, methoxy, ethoxy, n- and i-propoxy, n-, i-, s- and t-butoxy, methylthio, ethylthio, n- and i-propylthio, n-, i-, s- and t-butylthio, trifluoromethyl, trifluoromethoxy, trifluoromethylthio, cyano, dimethylamino and diethylamino; or represents pyridylmethyl or 1- or 2-pyridylethyl which is unsubstituted or substituted by one or two identical or different substituents, possible substituents being the abovementioned substituents for phenyl, excluding compounds in which

a)
$R^{4'}$ represents i-butyl and
$R^{1'}$ represents methyl or t-butyl and
$R^{2'}$ represents hydrogen and
$R^{5'}$ represents hydrogen and
$R^{6'}$ represents benzyl, 2-phenethyl or pyridylmethyl

and

b)
$R^{4'}$ represents s-butyl and
$R^{1'}$ represents t-butyl and
$R^{2'}$ represents hydrogen and
$R^{5'}$ represents hydrogen and
$R^{6'}$ represents benzyl and

c)
$R^{4'}$ represents i-propyl and
$R^{1'}$ represents methyl, ethyl or t-butyl and
$R^{2'}$ represents hydrogen and
$R^{5'}$ represents hydrogen and
$R^{6'}$ represents benzyl.

**7.** Agents for combating pests, characterized in that they contain at least one amino acid amide derivative of the formula (I) or (Ia) according to Claims 1 to 6.

**8.** Method of combating phytopathogenic fungi, characterized in that amino acid amide derivatives of the formula (I) or (Ia) according to Claims 1 to 7 are allowed to act on fungi and/or their environment.

**9.** Process for the preparation of agents for combating pests, characterized in that amino acid amide derivatives of the formula (I) or (Ia) according to Claims 1 to 7 are mixed with extenders and/or surface active agents.

**Revendications**

**Revendications pour les Etats contractants suivants : BE, CH, DE, FR, GB, GR, IT, LI, NL**

**1.** Utilisation de dérivés d'amides d'amino-acides substitués de formule générale (I)

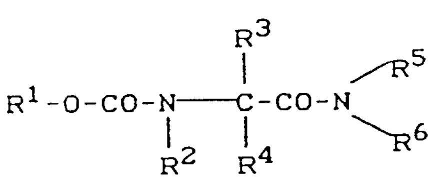

(I)

dans laquelle

$R^1$ est un groupe alkyle, alcényle, alcynyle, halogénalkyle, halogénalcényle, halogénalcynyle, cycloalkyle ou cycloalcényle,

$R^2$, $R^3$, $R^4$ et $R^5$ sont identiques ou différents et réprésentent de l'hydrogène, un groupe cycloalkyle ou alkyle, ou bien

$R^3$ et $R^4$ forment, conjointement avec l'atome de carbone auquel ils sont liés, un noyau cycloalkyle et

$R^6$ est de l'hydrogène, un groupe alkyle, alcényle, alcynyle, cycloalkyle, phénylalkyle non substitué ou substitué, phényle non substitué ou substitué, cycloalkyle non substitué ou substitué ou un groupe hétérocyclyle ou hétérocyclylalkyle non substitué ou substitué,

$R^5$ et $R^6$ forment, conjointement avec l'atome d'azote auquel ils sont liés, un reste hétérocyclyle qui peut contenir d'autres hétéroatomes,

pour combattre des champignons phytopathogènes.

**2.** Dérivés d'amides d'amino-acides substitués de formule générale (Ia)

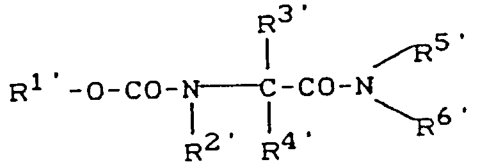

(Ia)

dans laquelle

$R^{1'}$ est un groupe alkyle, alcényle, alcynyle, halogénalkyle, halogénalcényle, halogénalcynyle, cycloalkyle ou cycloalcényle,

$R^{2'}$ et $R^{5'}$ sont identiques ou différents et représentent de l'hydrogène ou un groupe alkyle,

$R^{3'}$ est de l'hydrogène et

$R^{4'}$ est un groupe isopropyle, isobutyle, sec.-butyle, 3-pentyle ou cycloalkyle ou bien

$R^{3'}$ et $R^{4'}$ forment conjointement avec l'atome de carbone auquel ils sont liés un noyau cycloalkyle et

$R^{6'}$ représente de l'hydrogène, un groupe alkyle, alcényle, alcynyle, cyanalkyle, un groupe phénylalkyle non substitué ou substitué, un groupe phényle non substitué ou substitué, un groupe cycloalkyle non substitué ou substitué, ou un groupe hétérocyclyle ou hétérocyclylalkyle non substitué ou substitué,

ou bien

$R^{5'}$ et $R^{6'}$ forment, conjointement avec l'atome d'azote auquel ils sont liés, un reste hétérocyclyle qui peut contenir d'autres hétéroatomes, à l'exception de composés dans lesquels

a)

$R^{4'}$ est un groupe isobutyle

$R^{1'}$ est un groupe méthyle ou tertiobutyle,

$R^{2'}$ est un atome d'hydrogène,

$R^{5'}$ est un atome d'hydrogène ou un groupe méthyle

$R^{6'}$ est un atome d'hydrogène, un groupe méthyle, éthyle, phényle, benzyle, 2-phénéthyle ou pyridylméthyle, ou bien

$R^{5'}$ et $R^{6'}$ forment conjointement avec l'atome d'azote auquel ils sont liés un noyau pipéridine

b)

$R^{4'}$ est un groupe sec.-butyle

$R^{1'}$ est un groupe tertiobutyle

$R^{2'}$ est un atome d'hydrogène

$R^{5'}$ est un atome d'hydrogène et

$R^{6'}$ est un atome d'hydrogène ou un groupe benzyle

c)

$R^{4'}$ est un groupe isopropyle

$R^{1'}$ est un groupe méthyle, éthyle ou tertiobutyle

$R^{2'}$ est un atome d'hydrogène

$R^{5'}$ est un atome d'hydrogène et

$R^{6'}$ est un atome d'hydrogène, un groupe méthyle, phényle ou benzyle, ou bien

$R^{5'}$ et $R^{6'}$ forment conjointement avec l'atome d'azote auquel ils sont liés un noyau pyrrolidine.

3. Dérivés d'amides d'amino-acides substitués de formule (Ia) suivant la revendication 2, dans laquelle

$R^{1'}$ est un groupe alkyle linéaire ou ramifié ayant 1 à 6 atomes de carbone, un groupe alcényle ou alcynyle linéaire ou ramifié ayant 2 à 6 atomes de carbone, un groupe halogénalkyle linéaire ou ramifié ayant 1 à 6 atomes de carbone et 1 à 9 atomes d'halogènes identiques ou différents, un groupe halogénalcényle ou halogénalcynyle linéaire ou ramifié ayant 2 à 6 atomes de carbone et 1 à 9 atomes d'halogènes identiques ou différents et un groupe cycloalkyle ou cycloalcényle non substitué ou substitué, ayant 3 à 7 atomes de carbone, et

$R^{2'}$ et $R^{5'}$ sont identiques ou différents et représentent de l'hydrogène ou un groupe alkyle ayant 1 à 6 atomes de carbone, et

$R^{3'}$ est de l'hydrogène et

$R^{4'}$ est un groupe isopropyle, isobutyle, sec.-butyle ou 3-pentyle, ou bien

$R^{3'}$ et $R^{4'}$ forment conjointement avec l'atome de carbone auquel ils sont liés un noyau cycloalkyle de 3 à 7 atomes de carbone et

$R^{6'}$ est de l'hydrogène, un groupe alkyle linéaire ou ramifié ayant 1 à 6 atomes de carbone, un groupe alcényle ou alcynyle ayant chacun 2 à 6 atomes de carbone, un groupe cyanalkyle ayant 1 à 4 atomes de carbone dans la partie alkyle, un groupe phénylalkyle non substitué ou portant dans la partie phényle 1 à 3 substituants identiques ou différents, ayant 1 à 4 atomes de carbone dans la partie alkyle, ou un groupe phényle non substitué ou portant 1 à 3 substituants identiques ou différents, en considérant dans chaque cas comme substituants : un halogène ; des radicaux alkyle, alkoxy et alkylthio ayant chacun 1 à 4 atomes de carbone ; des radicaux halogénalkyle, halogénalkoxy et halogénalkylthio ayant chacun 1 à 4 atomes de carbone et portant 1 à 9 atomes d'halogènes identiques ou différents ; un radical hydroxy ; cyano, nitro, amino, alkylamino, dialkylamino avec dans chaque cas 1 à 4 atomes de carbone, et un radical carboxyle ; un groupe cycloalkyle de 3 à 7 atomes de carbone non substitué ou portant des substituants identiques ou différents, en considérant comme substituants des radicaux alkyle et alkoxy ayant chacun 1 à 4 atomes de carbone, ou un groupe hétérocyclyle ou hétérocyclylalkyle non substitué ou substitué ayant 2 à 6 atomes de carbone dans la partie hétérocyclyle et 1 ou 2 hétéroatomes, et le cas échéant 1 à 4 atomes de carbone dans la partie alkyle, en considérant comme substituants les substituants mentionnés ci-dessus pour le groupe phényle,

ou bien

$R^{5'}$ et $R^{6'}$ forment, conjointement avec l'atome d'azote auquel ils sont liés, un hétérocycle pentagonal ou hexagonal qui peut contenir le cas échéant de l'oxygène ou de l'azote comme autres hétéroatomes, à l'exception des composés dans lesquels

a)
$R^{4'}$ est un groupe isobutyle,
$R^{1'}$ est un groupe méthyle ou tertiobutyle
$R^{2'}$ est un atome d'hydrogène
$R^{5'}$ est un atome d'hydrogène ou un groupe méthyle
$R^{6'}$ est un atome d'hydrogène, un groupe méthyle, éthyle, phényle, benzyle, 2-phénéthyle ou pyridylméthyle, ou bien
$R^{5'}$ et $R^{6'}$ forment conjointement avec l'atome d'azote auquel ils sont liés, un noyau pipéridine,

b)
$R^{4'}$ est un groupe sec.-butyle
$R^{1'}$ est un groupe tertiobutyle
$R^{2'}$ est de l'hydrogène
$R^{5'}$ est de l'hydrogène, et
$R^{6'}$ est de l'hydrogène ou un groupe benzyle, et

c)
$R^{4'}$ est un groupe isopropyle
$R^{1'}$ est un groupe méthyle, éthyle ou tertiobutyle
$R^{2'}$ est de l'hydrogène
$R^{5'}$ est de l'hydrogène
$R^{6'}$ est de l'hydrogène, un groupe méthyle, phényle ou benzyle, ou bien
$R^{5'}$ et $R^{6'}$ forment conjointement avec l'atome d'azote auquel ils sont liés un noyau pyrrolidine.

**4.** Dérivés d'amides d'amino-acides substitués de formule (Ia) suivant la revendication 2, dans laquelle

$R^{1'}$ est un groupe alkyle linéaire ou ramifié ayant 1 à 4 atomes de carbone, un groupe alcényle ou alcynyle linéaire ou ramifié ayant 2 à 4 atomes de carbone, un groupe halogénalkyle linéaire ou ramifié ayant 1 à 4 atomes de carbone et 1 à 5 atomes d'halogènes identiques ou différents, un groupe halogénalcényle ou halogénalcynyle linéaire ou ramifié ayant 2 à 4 atomes de carbone et 1 à 5 atomes d'halogènes identiques ou différents ou un groupe cycloalkyle ou cycloalcényle non substitué ou substitué ayant 3 à 6 atomes de carbone,

$R^{2'}$ et $R^{5'}$ sont identiques ou différents et représentent de l'hydrogène, un groupe méthyle ou éthyle,

$R^{3'}$ est de l'hydrogène

$R^{4'}$ est un groupe isopropyle, isobutyle, sec.-butyle ou 3-pentyle, ou bien

$R^{3'}$ et $R^{4'}$ forment conjointement avec l'atome de carbone auquel ils sont liés un noyau cycloalkyle de 3 à 6 atomes de carbone et

$R^{6'}$ est de l'hydrogène, un groupe méthyle, éthyle, allyle, propargyle, un groupe cyanalkyle ayant 1 ou 2 atomes de carbone dans la partie alkyle ; en outre, un groupe phénylalkyle ayant 1 à 4 atomes de carbone dans la partie alkyle, non substitué ou portant dans la partie phényle 1 ou 2 substituants identiques ou différents, un groupe phényle non substitué ou portant 1 ou 2 substituants identiques ou différents, en considérant dans chaque cas comme substituants : le fluor, le chlore et le brome, un radical méthyle, éthyle, n-propyle, isopropyle, n-butyle, isobutyle, sec.-butyle, tertiobutyle, méthoxy, éthoxy, n-propyloxy, isopropyloxy, n-butyloxy, isobutyloxy, sec.-butyloxy, tertiobutyloxy, méthylthio, éthylthio, n-propylthio, isopropylthio, n-butylthio, isobutylthio, sec.-butylthio, tertiobutylthio, trifluorométhyle, trifluorométhoxy, trifluorométhylthio, hydroxy, nitro, cyano, amino, méthylamino, éthylamino, diméthylamino, diéthylamino et carboxy ; un groupe cycloalkyle ayant 3 à 6 atomes de carbone, chacun étant non substitué ou portant 1 ou 2 substituants identiques ou différents, en considérant comme substituants les radicaux méthyle, éthyle, n-propyle, isopropyle, n-butyle, isobutyle, sec.-butyle, tertiobutyle, méthoxy, éthoxy, n-propyloxy, isopropyloxy, n-butyloxy, isobutyloxy, sec.-butyloxy et tertiobutyloxy, ou un groupe hétérocyclyle ou hétérocyclylalkyle non substitué ou portant 1 ou 2 substituants identiques ou différents, avec 4 ou 5 atomes de carbone dans la partie hétérocyclyle et 1 ou 2 atomes d'azote et le cas échéant 1 à 4 atomes de carbone dans la partie alkyle, en considérant comme substituants les substituants indiqués ci-dessus pour le groupe phényle ; ou bien

$R^{5'}$ et $R^{6'}$ forment, conjointement avec l'atome d'azote auquel ils sont liés, un hétérocycle pentagonal ou hexagonal qui peut contenir le cas échéant de l'oxygène ou de l'azote comme autres hétéroatomes, à l'exception des composés dans lesquels

a)

$R^{4'}$ est un groupe isobutyle

$R^{1'}$ est un groupe méthyle ou tertiobutyle

$R^{2'}$ est un atome d'hydrogène

$R^{5'}$ est un atome d'hydrogène ou un groupe méthyle, et

$R^{6'}$ est un atome d'hydrogène, un groupe méthyle, éthyle, phényle, benzyle, 2-phénéthyle ou pyridylméthyle, ou bien

$R^{5'}$ et $R^{6'}$ forment un noyau pipéridine conjointement avec l'atome d'azote auquel ils sont liés,

b)

$R^{4'}$ représente un groupe sec.-butyle

$R^{1'}$ est un groupe tertiobutyle

$R^{2'}$ est un atome d'hydrogène

$R^{5'}$ est un atome d'hydrogène et

$R^{6'}$ est un atome d'hydrogène ou un groupe benzyle, et

c)

$R^{4'}$ est un groupe isopropyle

$R^{1'}$ est un groupe méthyle, éthyle ou tertiobutyle

$R^{2'}$ est un atome d'hydrogène

$R^{5'}$ est un atome d'hydrogène et

$R^{6'}$ est un atome d'hydrogène ou un groupe méthyle, phényle ou benzyle, ou bien

$R^{5'}$ et $R^{6'}$ forment un noyau pyrolidine conjointement avec l'atome d'azote auquel ils sont liés.

**5.** Dérivés d'amides d'amino-acides substitués de formule (Ia) suivant la revendication 2, dans laquelle

$R^{1'}$ est un groupe alkyle linéaire ou ramifié ayant 1 à 4 atomes de carbone, un groupe alcényle ou alcynyle linéaire ou ramifié ayant 2 à 4 atomes de carbone, un groupe halogénalkyle linéaire ou ramifié ayant 1 à 4 atomes de carbone et 1 à 5 atomes d'halogènes identiques ou différents, un groupe halogénalcényle ou halogénalcynyle linéaire ou ramifié ayant 2 à 4 atomes de carbone et 1 à 5 atomes d'halogènes identiques ou différents ou un groupe cycloalkyle ou cycloalcényle substitué ou non substitué ayant 3 à 6 atomes de carbone,

$R^{2'}$, $R^{3'}$ et $R^{5'}$ sont identiques ou différents et représentent de l'hydrogène

$R^{4'}$ est un groupe isopropyle, isobutyle, sec.-butyle ou 3-pentyle

$R^{6'}$ est un groupe phénylalkyle ayant 1 à 4 atomes de carbone dans la partie alkyle, non substitué ou portant dans la partie phényle 1 ou 2 substituants identiques ou différents, en considérant dans chaque cas comme substituants : le fluor, le chlore et le brome, un radical méthyle, éthyle, n-propyle, isopropyle, n-butyle, isobutyle, sec.-butyle, tertiobutyle, méthoxy, éthoxy, n-propoxy, isopropoxy, n-butyloxy, isobutyloxy, sec.-butoxy, tertiobutyloxy, méthylthio, éthylthio, n-propylthio, isopropylthio, n-butylthio, isobutylthio, sec.-butylthio, tertiobutylthio, trifluorométhyle, trifluorométhoxy, trifluorométhylthio, cyano, diméthylamino, diéthylamino, et carboxy ; ou un groupe hétérocyclylalkyle non substitué ou portant 1 ou 2 substituants, ayant 4 ou 5 atomes de carbone dans la partie hétérocyclyle et 1 ou 2 atomes d'azote et le cas échéant 1 à 4 atomes de carbone dans la partie alkyle, en considérant comme substituants les substituants du groupe phényle indiqués ci-dessus, à l'exception des composés dans lesquels

a)
- $R^{4'}$ est un groupe isobutyle
- $R^{1'}$ est un groupe méthyle ou tertiobutyle
- $R^{2'}$ est de l'hydrogène
- $R^{5'}$ est de l'hydrogène et
- $R^{6'}$ est un groupe benzyle, 2-phénéthyle ou pyridylméthyle

b)
- $R^{4'}$ est un groupe sec.-butyle
- $R^{1'}$ est un groupe tertiobutyle
- $R^{2'}$ est de l'hydrogène
- $R^{5'}$ est de l'hydrogène, et
- $R^{6'}$ est un groupe benzyle, et

c)
- $R^{4'}$ est un groupe isopropyle
- $R^{1'}$ est un groupe méthyle, éthyle ou tertiobutyle
- $R^{2'}$ est de l'hydrogène
- $R^{5'}$ est de l'hydrogène et
- $R^{6'}$ est un groupe benzyle.

**6.** Dérivés d'amides d'amino-acides substitués de formule (Ia) suivant la revendication 2, dans laquelle

$R^{1'}$ est un groupe méthyle, éthyle, n-propyle, isopropyle, n-butyle, isobutyle, sec.-butyle, tertiobutyle, fluorométhyle, fluoréthyle, fluoropropyle, chloropropyle, fluorobutyle, chlorobutyle, difluorométhyle, difluoropropyle, dichloropropyle, difluorobutyle, dichlorobutyle, trifluorométhyle, trichlorométhyle, trifluoréthyle, trichloréthyle, trifluoropropyle, trichloropropyle, trifluorobutyle, trichlorobutyle, allyle, butényle, propargyle, butynyle, fluorallyle, chlorallyle, fluoro- ou chlorobutényle, fluoro- ou chloropropargyle, fluoro- ou chlorobutynyle, cyclopropyle, cyclopentyle, cyclohexyle, cyclopentényle ou cyclohexényle,

$R^{2'}$, $R^{3'}$ et $R^{5'}$ représentent de l'hydrogène

$R^{4'}$ est un groupe isopropyle, isobutyle, sec.-butyle ou 3-pentyle

$R^{6'}$ est un groupe benzyle, 1-phénéthyle, 2-phénéthyle ou 1-phénylpropyle, 2-phénylpropyle, 3-phénylpropyle, 1-phénylisopropyle, 2-phénylisopropyle ou 1-phényl-n-, -iso- ou sec.-butyle non substitué ou portant dans la partie phényle 1 ou 2

substituants identiques ou différents, en considérant dans chaque cas comme substituants : le fluor, le chlore, le brome, un radical méthyle, éthyle, n-propyle, isopropyle, n-butyle, isobutyle, sec.-butyle, tertiobutyle, méthoxy, éthoxy, n-propyloxy, isopropoxy, n-butyloxy, isobutyloxy, sec.-butyloxy, tertiobutyloxy, méthylthio, éthylthio, n-propylthio, isopropylthio, n-butylthio, isobutylthio, sec.-butylthio, tertiobutylthio, trifluorométhyle, trifluorométhoxy, trifluorométhylthio, cyano, diméthylamino et diéthylamino ; un groupe pyridylméthyle ou 1- ou 2-pyridyléthyle non substitué ou portant 1 ou 2 substituants identiques ou différents, en considérant comme substituants les substituants mentionnés ci-dessus pour le groupe phényle, excepté les composés dans lesquels

a)

$R^{4'}$ est un groupe isobutyle
$R^{1'}$ est un groupe méthyle ou tertiobutyle
$R^{2'}$ est de l'hydrogène
$R^{5'}$ est de l'hydrogène et
$R^{6'}$ est un groupe benzyle, 2-phénéthyle ou pyridylméthyle

b)

$R^{4'}$ est un groupe sec.-butyle
$R^{1'}$ est un groupe tertiobutyle
$R^{2'}$ est de l'hydrogène
$R^{5'}$ est de l'hydrogène, et
$R^{6'}$ est un groupe benzyle, et

c)

$R^{4'}$ est un groupe isopropyle
$R^{1'}$ est un groupe méthyle, éthyle ou tertiobutyle
$R^{2'}$ est de l'hydrogène
$R^{5'}$ est de l'hydrogène et
$R^{6'}$ est un groupe benzyle.

**7.** Procédé de production de dérivés d'amides d'amino-acides substitués de formule générale (Ia)

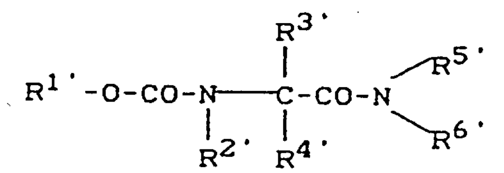

(Ia)

dans laquelle

$R^{1'}$ est un groupe alkyle, alcényle, alcynyle, halogénalkyle, halogénalcényle, halogénalcynyle, cycloalkyle ou cycloalcényle,
$R^{2'}$ et $R^{5'}$ sont identiques ou différents et représentent de l'hydrogène ou un groupe alkyle,
$R^{3'}$ est de l'hydrogène et
$R^{4'}$ est un groupe isopropyle, isobutyle, sec.-butyle, 3-pentyle ou cycloalkyle ou bien
$R^{3'}$ et $R^{4'}$ forment conjointement avec l'atome de carbone auquel ils sont liés un noyau cycloalkyle et
$R^{6'}$ représente de l'hydrogène, un groupe alkyle, alcényle, alcynyle, cyanalkyle, un groupe phénylalkyle non substitué ou substitué, un groupe phényle non substitué ou substitué, un groupe cycloalkyle non substitué ou substitué, ou un groupe hétérocyclyle ou hétérocyclylalkyle non substitué ou substitué,

ou bien

$R^{5'}$ et $R^{6'}$ forment, conjointement avec l'atome d'azote auquel ils sont liés, un reste hétérocyclyle qui peut contenir d'autres hétéroatomes, à l'exception de composés dans lesquels

a)

$R^{4'}$ est un groupe isobutyle
$R^{1'}$ est un groupe méthyle ou tertiobutyle,
$R^{2'}$ est un atome d'hydrogène,

$R^{5'}$ est un atome d'hydrogène ou un groupe méthyle
$R^{6'}$ est un atome d'hydrogène, un groupe méthyle, éthyle, phényle, benzyle, 2-phénéthyle ou pyridylméthyle, ou bien
$R^{5'}$ et $R^{6'}$ forment conjointement avec l'atome d'azote auquel ils sont liés un noyau pipéridine

b)
$R^{4'}$ est un groupe sec.-butyle
$R^{1'}$ est un groupe tertiobutyle
$R^{2'}$ est un atome d'hydrogène
$R^{5'}$ est un atome d'hydrogène et
$R^{6'}$ est un atome d'hydrogène ou un groupe benzyle

c)
$R^{4'}$ est un groupe isopropyle
$R^{1'}$ est un groupe méthyle, éthyle ou tertiobutyle
$R^{2'}$ est un atome d'hydrogène
$R^{5'}$ est un atome d'hydrogène et
$R^{6'}$ est un atome d'hydrogène, un groupe méthyle, phényle ou benzyle, ou bien
$R^{5'}$ et $R^{6'}$ forment conjointement avec l'atome d'azote auquel ils sont liés un noyau pyrrolidine,

caractérisé en ce qu'on fait réagir un amino-acide substitué de formule (II)

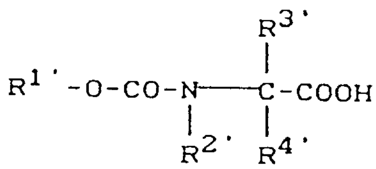

(II)

dans laquelle
$R^{1'}$, $R^{2'}$, $R^{3'}$ et $R^{4'}$ ont la définition indiquée ci-dessus, ou ses dérivés à groupe carboxy activé,
le cas échéant en présence d'un catalyseur, en présence éventuelle d'un accepteur d'acide et, le cas échéant en présence d'un diluant,
avec une amine de formule (III)

$HNR^{5'}R^{6'}$ (III)

dans laquelle
$R^{5'}$ et $R^{6'}$ ont la définition indiquée ci-dessus.

**8.** Compositions pesticides, caractérisées par une teneur en au moins un dérivé d'amide d'amino-acides de formule (I) ou de formule (Ia) suivant les revendications 1 à 7.

**9.** Procédé pour combattre des champignons phytopathogènes, caractérisé en ce qu'on fait agir des dérivés d'amides d'amino-acides de formule (I) ou (Ia) suivant les revendications 1 à 7 sur des champignons et/ou sur leur milieu.

**10.** Procédé de préparation de compositions pesticides, caractérisé en ce qu'on mélange des dérivés d'amides d'amino-acides de formule (I) ou (Ia) suivant les revendications 1 à 7 avec des diluants et/ou des agents tensio-actifs.

**Revendications pour l'Etat contractant suivant : ES**

**1.** Utilisation de dérivés d'amides d'amino-acides substitués de formule générale (I)

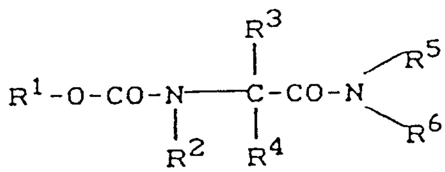

(I)

dans laquelle

- $R^1$ est un groupe alkyle, alcényle, alcynyle, halogénalkyle, halogénalcényle, halogénalcynyle, cycloalkyle ou cycloalcényle,
- $R^2$, $R^3$, $R^4$ et $R^5$ sont identiques ou différents et réprésentent de l'hydrogène, un groupe cycloalkyle ou alkyle, ou bien
- $R^3$ et $R^4$ forment, conjointement avec l'atome de carbone auquel ils sont liés, un noyau cycloalkyle et
- $R^6$ est de l'hydrogène, un groupe alkyle, alcényle, alcynyle, cycloalkyle, phénylalkyle non substitué ou substitué, phényle non substitué ou substitué, cycloalkyle non substitué ou substitué ou un groupe hétérocyclyle ou hétérocyclylalkyle non substitué ou substitué, ou
- $R^5$ et $R^6$ forment, conjointement avec l'atome d'azote auquel ils sont liés, un reste hétérocyclyle qui peut contenir d'autres hétéroatomes,

pour combattre des champignons phytopathogènes.

**2.** Procédé de production de dérivés d'amides d'amino-acides substitués de formule générale (Ia)

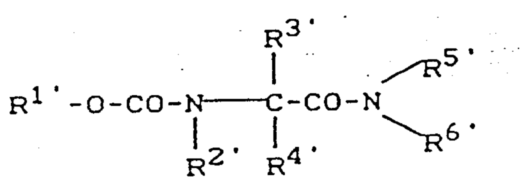

(Ia)

dans laquelle

- $R^{1'}$ est un groupe alkyle, alcényle, alcynyle, halogénalkyle, halogénalcényle, halogénalcynyle, cycloalkyle ou cycloalcényle,
- $R^{2'}$ et $R^{5'}$ sont identiques ou différents et représentent de l'hydrogène ou un groupe alkyle,
- $R^{3'}$ est de l'hydrogène et
- $R^{4'}$ est un groupe isopropyle, isobutyle, sec.-butyle, 3-pentyle ou cycloalkyle ou bien
- $R^{3'}$ et $R^{4'}$ forment conjointement avec l'atome de carbone auquel ils sont liés un noyau cycloalkyle et
- $R^{6'}$ représente de l'hydrogène, un groupe alkyle, alcényle, alcynyle, cyanalkyle, un groupe phénylalkyle non substitué ou substitué, un groupe phényle non substitué ou substitué, un groupe cycloalkyle non substitué ou substitué, ou un groupe hétérocyclyle ou hétérocyclylalkyle non substitué ou substitué,

ou bien

- $R^{5'}$ et $R^{6'}$ forment, conjointement avec l'atome d'azote auquel ils sont liés, un reste hétérocyclyle qui peut contenir d'autres hétéroatomes, à l'exception de composés dans lesquels

a)

- $R^{4'}$ est un groupe isobutyle
- $R^{1'}$ est un groupe méthyle ou tertiobutyle,
- $R^{2'}$ est un atome d'hydrogène,
- $R^{5'}$ est un atome d'hydrogène ou un groupe méthyle

$R^{6'}$ est un atome d'hydrogène, un groupe méthyle, éthyle, phényle, benzyle, 2-phénéthyle ou pyridylméthyle, ou bien

$R^{5'}$ et $R^{6'}$ forment conjointement avec l'atome d'azote auquel ils sont liés un noyau pipéridine

b)

$R^{4'}$ est un groupe sec.-butyle

$R^{1'}$ est un groupe tertiobutyle

$R^{2'}$ est un atome d'hydrogène

$R^{5'}$ est un atome d'hydrogène et

$R^{6'}$ est un atome d'hydrogène ou un groupe benzyle

c)

$R^{4'}$ est un groupe isopropyle

$R^{1'}$ est un groupe méthyle, éthyle ou tertiobutyle

$R^{2'}$ est un atome d'hydrogène

$R^{5'}$ est un atome d'hydrogène et

$R^{6'}$ est un atome d'hydrogène, un groupe méthyle, phényle ou benzyle, ou bien

$R^{5'}$ et $R^{6'}$ forment conjointement avec l'atome d'azote auquel ils sont liés un noyau pyrrolidine,

caractérisé en ce qu'on fait réagir un amino-acide substitué de formule (II)

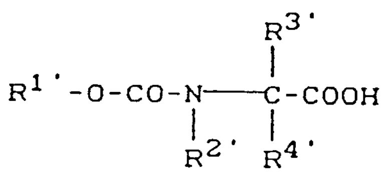

(II)

dans laquelle

$R^{1'}$, $R^{2'}$, $R^{3'}$ et $R^{4'}$ ont la définition indiquée ci-dessus, ou ses dérivés à groupe carboxy activé,

le cas échéant en présence d'un catalyseur, en présence éventuelle d'un accepteur d'acide et, le cas échéant en présence d'un diluant,

avec une amine de formule (III)

$HNR^{5'}R^{6'}$ (III)

dans laquelle

$R^{5'}$ et $R^{6'}$ ont la définition indiquée ci-dessus.

**3.** Procédé suivant la revendication 2, dans lequel

$R^{1'}$ est un groupe alkyle linéaire ou ramifié ayant 1 à 6 atomes de carbone, un groupe alcényle ou alcynyle linéaire ou ramifié ayant 2 à 6 atomes de carbone, un groupe halogénalkyle linéaire ou ramifié ayant 1 à 6 atomes de carbone et 1 à 9 atomes d'halogènes identiques ou différents, un groupe halogénalcényle ou halogénalcynyle linéaire ou ramifié ayant 2 à 6 atomes de carbone et 1 à 9 atomes d'halogènes identiques ou différents et un groupe cycloalkyle ou cycloalcényle non substitué ou substitué, ayant 3 à 7 atomes de carbone, et

$R^{2'}$ et $R^{5'}$ sont identiques ou différents et représentent de l'hydrogène ou un groupe alkyle ayant 1 à 6 atomes de carbone, et

$R^{3'}$ est de l'hydrogène et

$R^{4'}$ est un groupe isopropyle, isobutyle, sec.-butyle ou 3-pentyle, ou bien

$R^{3'}$ et $R^{4'}$ forment conjointement avec l'atome de carbone auquel ils sont liés un noyau cycloalkyle de 3 à 7 atomes de carbone et

$R^{6'}$ est de l'hydrogène, un groupe alkyle linéaire ou ramifié ayant 1 à 6 atomes de carbone, un groupe alcényle ou alcynyle ayant chacun 2 à 6 atomes de carbone, un groupe cyanalkyle ayant 1 à 4 atomes de carbone dans la partie alkyle, un groupe phénylalkyle non substitué ou portant dans la partie phényle 1 à 3 substituants identiques ou différents, ayant 1 à 4 atomes de carbone dans la partie alkyle, ou un groupe phényle non substitué ou portant 1 à 3 substituants identiques ou différents, en considérant dans chaque cas comme substituants : un halogène ; des radicaux alkyle,

alkoxy et alkylthio ayant chacun 1 à 4 atomes de carbone ; des radicaux halogénalkyle, halogénalkoxy et halogénalkylthio ayant chacun 1 à 4 atomes de carbone et portant 1 à 9 atomes d'halogènes identiques ou différents ; un radical hydroxy ; cyano, nitro, amino, alkylamino, dialkylamino avec dans chaque cas 1 à 4 atomes de carbone, et un radical carboxyle ; un groupe cycloalkyle de 3 à 7 atomes de carbone non substitué ou portant des substituants identiques ou différents, en considérant comme substituants des radicaux alkyle et alkoxy ayant chacun 1 à 4 atomes de carbone, ou un groupe hétérocyclyle ou hétérocyclylalkyle non substitué ou substitué ayant 2 à 6 atomes de carbone dans la partie hétérocyclyle et 1 ou 2 hétéroatomes, et le cas échéant 1 à 4 atomes de carbone dans la partie alkyle, en considérant comme substituants les substituants mentionnés ci-dessus pour le groupe phényle,

ou bien

$R^{5'}$ et $R^{6'}$ forment, conjointement avec l'atome d'azote auquel ils sont liés, un hétérocycle pentagonal ou hexagonal qui peut contenir le cas échéant de l'oxygène ou de l'azote comme autres hétéroatomes, à l'exception des composés dans lesquels

a)
- $R^{4'}$ est un groupe isobutyle,
- $R^{1'}$ est un groupe méthyle ou tertiobutyle
- $R^{2'}$ est un atome d'hydrogène
- $R^{5'}$ est un atome d'hydrogène ou un groupe méthyle
- $R^{6'}$ est un atome d'hydrogène, un groupe méthyle, éthyle, phényle, benzyle, 2-phénéthyle ou pyridylméthyle, ou bien
- $R^{5'}$ et $R^{6'}$ forment conjointement avec l'atome d'azote auquel ils sont liés, un noyau pipéridine,

b)
- $R^{4'}$ est un groupe sec.-butyle
- $R^{1'}$ est un groupe tertiobutyle
- $R^{2'}$ est de l'hydrogène
- $R^{5'}$ est de l'hydrogène, et
- $R^{6'}$ est de l'hydrogène ou un groupe benzyle, et

c)
- $R^{4'}$ est un groupe isopropyle
- $R^{1'}$ est un groupe méthyle, éthyle ou tertiobutyle
- $R^{2'}$ est de l'hydrogène
- $R^{5'}$ est de l'hydrogène
- $R^{6'}$ est de l'hydrogène, un groupe méthyle, phényle ou benzyle, ou bien
- $R^{5'}$ et $R^{6'}$ forment conjointement avec l'atome d'azote auquel ils sont liés un noyau pyrrolidine.

**4.** Procédé suivant la revendication 2, dans lequel

$R^{1'}$ est un groupe alkyle linéaire ou ramifié ayant 1 à 4 atomes de carbone, un groupe alcényle ou alcynyle linéaire ou ramifié ayant 2 à 4 atomes de carbone, un groupe halogénalkyle linéaire ou ramifié ayant 1 à 4 atomes de carbone et 1 à 5 atomes d'halogènes identiques ou différents, un groupe halogénalcényle ou halogénalcynyle linéaire ou ramifié ayant 2 à 4 atomes de carbone et 1 à 5 atomes d'halogènes identiques ou différents ou un groupe cycloalkyle ou cycloalcényle non substitué ou substitué ayant 3 à 6 atomes de carbone,

$R^{2'}$ et $R^{5'}$ sont identiques ou différents et représentent de l'hydrogène, un groupe méthyle ou éthyle,

$R^{3'}$ est de l'hydrogène

$R^{4'}$ est un groupe isopropyle, isobutyle, sec.-butyle ou 3-pentyle, ou bien

$R^{3'}$ et $R^{4'}$ forment conjointement avec l'atome de carbone auquel ils sont liés un noyau cycloalkyle de 3 à 6 atomes de carbone et

$R^{6'}$ est de l'hydrogène, un groupe méthyle, éthyle, allyle, propargyle, un groupe cyanalkyle ayant 1 ou 2 atomes de carbone dans la partie alkyle ; en outre, un groupe phénylalkyle ayant 1 à 4 atomes de carbone dans la partie alkyle, non substitué ou portant dans la partie phényle 1 ou 2 substituants identiques ou différents, un groupe phényle non substitué ou portant 1 ou 2 substituants identiques ou différents, en considérant dans chaque cas comme substituants : le fluor, le chlore et le brome, un radical méthyle, éthyle, n-propyle, isopropyle, n-butyle, isobutyle, sec.-butyle, tertiobutyle, méthoxy, éthoxy, n-propoxy, isopropoxy, n-butyloxy, isobutyloxy, sec.-butyloxy, tertiobutyloxy, méthylthio, éthylthio, n-propylthio, isopropylthio, n-butylthio, isobutylthio, sec.-butylthio, tertiobutylthio, trifluorométhyle, trifluorométhoxy, trifluorométhylthio, hydroxy, nitro, cyano, amino, méthylamino, éthylamino, diméthylamino, diéthylamino et carboxy ; un groupe cycloalkyle linéaire ou ramifié de 3 à 6 atomes de carbone, chacun étant non substitué ou portant 1 ou 2 substituants identiques ou différents, en considérant comme substituants les radicaux méthyle, éthyle, n-propyle, isopropyle, n-butyle, isobutyle, sec.-butyle, tertiobutyle, méthoxy, éthoxy, n-propyloxy, isopropyloxy, n-butyloxy, isobutyloxy, sec.-butyloxy et tertiobutyloxy, ou un groupe hétérocyclyle ou hétérocyclylalkyle non substitué ou portant 1 ou 2 substituants identiques ou différents, avec 4 ou 5 atomes de carbone dans la partie hétérocyclyle et 1 ou 2 atomes d'azote et le cas échéant 1 à 4 atomes de carbone dans la partie alkyle, en considérant comme substituants les substituants indiqués ci-dessus pour le groupe phényle ; oou bien

$R^{5'}$ et $R^{6'}$ forment, conjointement avec l'atome d'azote auquel ils sont liés, un hétérocycle pentagonal ou hexagonal qui peut contenir le cas échéant de l'oxygène ou de l'azote comme autres hétéroatomes, à l'exception des composés dans lesquels

a)
- $R^{4'}$ est un groupe isobutyle
- $R^{1'}$ est un groupe méthyle ou tertiobutyle
- $R^{2'}$ est un atome d'hydrogène
- $R^{5'}$ est un atome d'hydrogène ou un groupe méthyle, et
- $R^{6'}$ est un atome d'hydrogène, un groupe méthyle, éthyle, phényle, benzyle, 2-phénéthyle ou pyridylméthyle, ou bien
- $R^{5'}$ et $R^{6'}$ forment un noyau pipéridine conjointement avec l'atome d'azote auquel ils sont liés,

b)
- $R^{4'}$ représente un groupe sec.-butyle
- $R^{1'}$ est un groupe tertiobutyle
- $R^{2'}$ est un atome d'hydrogène
- $R^{5'}$ est un atome d'hydrogène et
- $R^{6'}$ est un atome d'hydrogène ou un groupe benzyle, et

c)
- $R^{4'}$ est un groupe isopropyle
- $R^{1'}$ est un groupe méthyle, éthyle ou tertiobutyle
- $R^{2'}$ est un atome d'hydrogène
- $R^{5'}$ est un atome d'hydrogène et
- $R^{6'}$ est un atome d'hydrogène ou un groupe méthyle, phényle ou benzyle, ou bien
- $R^{5'}$ et $R^{6'}$ forment un noyau pyrolidine conjointement avec l'atome d'azote auquel ils sont liés.

**5.** Procédé suivant la revendication 2, dans lequel

$R^{1'}$ est un groupe alkyle linéaire ou ramifié ayant 1 à 4 atomes de carbone, un groupe alcényle ou alcynyle linéaire ou ramifié ayant 2 à 4 atomes de carbone, un groupe halogénalkyle linéaire ou ramifié ayant 1 à 4 atomes de carbone et 1 à 5 atomes d'halogènes identiques ou différents, un groupe halogénalcényle ou halogénalcynyle linéaire ou ramifié ayant 2 à 4 atomes de carbone et 1 à 5 atomes d'halogènes identiques ou différents ou un groupe cycloalkyle ou cycloalcényle substitué ou non substitué ayant 3 à 6 atomes de carbone,

$R^{2'}$, $R^{3'}$ et $R^{5'}$ sont identiques ou différents et représentent de l'hydrogène

$R^{4'}$ est un groupe isopropyle, isobutyle, sec.-butyle ou 3-pentyle

$R^{6'}$ est un groupe phénylalkyle ayant 1 à 4 atomes de carbone dans la partie alkyle, non substitué ou portant dans la partie phényle 1 ou 2 substituants identiques ou différents, en considérant dans chaque cas comme substituants : le fluor, le chlore et le brome, un radical méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, n-propylthio, isopropylthio, n-butylthio, isobutylthio, sec.-butylthio, tertiobutylthio, trifluorométhyle, trifluorométhoxy, trifluorométhylthio, cyano, diméthylamino, diéthylamino, et carboxy ; ou un groupe hétérocyclylalkyle non substitué ou portant 1 ou 2 substituants, ayant 4 ou

5 atomes de carbone dans la partie hétérocyclyle et 1 ou 2 atomes d'azote et le cas échéant 1 à 4 atomes de carbone dans la partie alkyle, en considérant comme substituants les substituants du groupe phényle indiqués ci-dessus, à l'exception des composés dans lesquels

a)
$R^{4'}$ est un groupe isobutyle
$R^{1'}$ est un groupe méthyle ou tertiobutyle
$R^{2'}$ est de l'hydrogène
$R^{5'}$ est de l'hydrogène et
$R^{6'}$ est un groupe benzyle, 2-phényléthyle ou pyridylméthyle

b)
$R^{4'}$ est un groupe sec.-butyle
$R^{1'}$ est un groupe tertiobutyle
$R^{2'}$ est de l'hydrogène
$R^{5'}$ est de l'hydrogène, et
$R^{6'}$ est un groupe benzyle, et

c)
$R^{4'}$ est un groupe isopropyle
$R^{1'}$ est un groupe méthyle, éthyle ou tertiobutyle
$R^{2'}$ est de l'hydrogène
$R^{5'}$ est de l'hydrogène et
$R^{6'}$ est un groupe benzyle.

**6.** Procédé suivant la revendication 2, dans lequel

$R^{1'}$ est un groupe méthyle, éthyle, n-propyle, isopropyle, n-butyle, isobutyle, sec.-butyle, tertiobutyle, fluorométhyle, fluoréthyle, fluoropropyle, chloropropyle, fluorobutyle, chlorobutyle, difluorométhyle, difluoropropyle, dichloropropyle, difluorobutyle, dichlorobutyle, trifluorométhyle, trichlorométhyle, trifluoréthyle, trichloréthyle, trifluoropropyle, trichloropropyle, trifluorobutyle, trichlorobutyle, allyle, butényle, propargyle, butynyle, fluorallyle, chlorallyle, fluoro- ou chlorobutényle, fluoro- ou chloropropargyle, fluoro- ou chlorobutynyle, cyclopropyle, cyclopentyle, cyclohexyle, cyclopentényle ou cyclohexényle,

$R^{2'}$, $R^{3'}$ et $R^{5'}$ représentent de l'hydrogène

$R^{4'}$ est un groupe isopropyle, isobutyle, sec.-butyle ou 3-pentyle

$R^{6'}$ est un groupe benzyle, 1-phénéthyle, 2-phénéthyle ou 1-phénylpropyle, 2-phénylpropyle, 3-phénylpropyle, 1-phénylisopropyle, 2-phénylisopropyle ou 1-phényl-n-, -iso- ou sec.-butyle non substitué ou portant dans la partie phényle 1 ou 2 substituants identiques ou différents, en considérant dans chaque cas comme substituants : le fluor, le chlore, le brome, un radical méthyle, éthyle, n-propyle, isopropyle, n-butyle, isobutyle, sec.-butyle, tertiobutyle, méthoxy, éthoxy, n-propyloxy, isopropoxy, n-butyloxy, isobutyloxy, sec.-butyloxy, tertiobutyloxy, méthylthio, éthylthio, n-propylthio, isopropylthio, n-butylthio, isobutylthio, sec.-butylthio, tertiobutylthio, trifluorométhyle, trifluorométhoxy, trifluorométhylthio, cyano, diméthylamino et diéthylamino ; un groupe pyridylméthyle ou 1- ou 2-pyridyléthyle non substitué ou portant 1 ou 2 substituants identiques ou différents, en considérant comme substituants les substituants mentionnés ci-dessus pour le groupe phényle, excepté les composés dans lesquels

a)
$R^{4'}$ est un groupe isobutyle
$R^{1'}$ est un groupe méthyle ou tertiobutyle
$R^{2'}$ est de l'hydrogène
$R^{5'}$ est de l'hydrogène et
$R^{6'}$ est un groupe benzyle, 2-phénéthyle ou pyridylméthyle

b)
$R^{4'}$ est un groupe sec.-butyle
$R^{1'}$ est un groupe tertiobutyle
$R^{2'}$ est de l'hydrogène
$R^{5'}$ est de l'hydrogène, et

$R^{6'}$ est un groupe benzyle, et

c)

$R^{4'}$ est un groupe isopropyle

$R^{1'}$ est un groupe méthyle, éthyle ou tertiobutyle

$R^{2'}$ est de l'hydrogène

$R^{5'}$ est de l'hydrogène et

$R^{6'}$ est un groupe benzyle.

**7.** Compositions pesticides, caractérisées par une teneur en au moins un dérivé d'amide d'amido-acide de formule (I) ou (Ia) suivant les revendications 1 à 6.

**8.** Procédé pour combattre des champignons phytopathogènes, caractérisé en ce qu'on fait agir des dérivés d'amides d'amino-acides de formule (I) ou (Ia) suivant les revendications 1 à 6 sur des champignons et/ou sur leur milieu.

**9.** Procédé de préparation de compositions pesticides, caractérisé en ce qu'on mélange des dérivés d'amides d'amino-acides de formule (I) ou (Ia) suivant les revendications 1 à 6 avec des diluants et/ou des agents tensio-actifs.